(19) Europäisches Patentamt
European Patent Office
Office européen des brevets

(11) **EP 1 553 768 A2**

(12) **EUROPEAN PATENT APPLICATION**

(43) Date of publication:
**13.07.2005 Bulletin 2005/28**

(51) Int Cl.⁷: **H04N 5/85**

(21) Application number: **05100037.0**

(22) Date of filing: **05.01.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **08.01.2004 JP 2004003109**

(71) Applicant: **Kabushiki Kaisha Toshiba
Tokyo 105-8001 (JP)**

(72) Inventors:
• **Kikuchi, Shinichi Toshiba Corp. Intell. Prop. Div.
Tokyo 105-801 (JP)**

• **Nakashika, Masahiro Toshiba Corp.
Intell. Prop. Di
Tokyo 105-8001 (JP)**
• **Tsumagari, Yasufumi Toshiba Corp.
Intell. Prop. Di
Tokyo 105-8001 (JP)**

(74) Representative: **Henkel, Feiler & Hänzel
Möhlstrasse 37
81675 München (DE)**

(54) **Information recording medium, information recording method, information playback method, information recording apparatus, and information playback apparatus**

(57) An information recording medium (100) for recording a digital stream signal, comprises a management region (130) for storing control information and a table (MNF_ID_TBL) of identifiers representing manufactures of recording devices, and a data region (131) for storing objects (SOBU) storing portions of the digital stream signal and number information for specifying an identifier in the table representing a manufacturer of a recording device which records the objects in the data region (131).

FIG. 2

**Description**

The present invention relates to an information recording medium (or a data structure); an information recording/playback method; and an information recording/playback apparatus suitable for recording/playing back a digital stream signal for use in digital TV broadcasting or the like.

In recent years, digital broadcasting of preparing a program of high resolution AV information as main broadcast contents has been prevailingly more popular. In currently implemented BS digital broadcasting (and terrestrial digital TV broadcasting whose implementation has been started), an MPEG-2 transport stream (hereinafter, referred to as MPEG-TS) is employed. In the field of digital broadcasting using a moving picture, the MPEG-TS is believed to be used as standard in the future. With start of such digital TV broadcasting, there is a growing market need for a streamer which can record contents of digital TV broadcasting as they are.

An example of a streamer utilizing an optical disk such as a DVD-RAM includes a "recording and playback apparatus" as disclosed in Japanese Patent Application KOKAI Publication No. 6-225239 (Page 2, FIG. 1).

In the meantime, it is considered for recording device to have a unique function which is not defined in DVD specifications and is in accordance with manufacturers or device type, and discriminates from other competitors. In that case, it is considered necessary to record information unique to manufacturers in a recording medium. However, the above patent document fails to disclose any support for such a case.

It is an object of the present invention to provide an information recording medium (or a data structure), an information recording/playback method, and an information recording/playback apparatus capable of efficiently recording information relating to recording device manufacturers in an information recording medium and capable of easily playing back such information relating to recording device manufacturers.

According to an embodiment of the present invention, an information recording medium for recording a digital stream signal, comprises:

a management region for storing control information and a table of identifiers representing manufactures of recording devices; and
a data region for storing objects storing portions of the digital stream signal and number information for specifying an identifier in the table representing a manufacturer of a recording device which records the objects in the data region.

According to another embodiment of the present invention, a method for recording a digital stream signal in an information recording medium comprising a management region and a data region, the method comprises:

storing, in the management region, control information and a table of identifiers representing manufactures of recording devices in the management region;
storing, in the data region, objects storing portions of the digital stream signal and number information for specifying an identifier in the table representing a manufacturer of a recording device which records the objects;
selecting number information specifying a specified identifier in the table; and
recording the selected number information in the data region.

According to another embodiment of the present invention, a method for reproducing information from an information recording medium for recording a digital stream signal, the recording medium comprising:

a management region for storing control information and
a table of identifiers representing manufactures of recording devices; and a data region for storing objects storing portions of the digital stream signal and number information for specifying an identifier in the table representing a manufacturer of a recording device which records the objects in the data region, the method comprises:

specifying a device manufacturer having undergone recording operation to the information recording medium based on the number information read from the data region; and
executing a function unique to a device manufacture in accordance with the specified device manufacturer.

According to another embodiment of the present invention, an apparatus for recording a digital stream signal in an information recording medium comprising a management region and a data region, the apparatus comprises:

means for storing, in the management region, control information and a table of identifiers representing manufactures of recording devices in the management region;
means for storing, in the data region, objects storing portions of the digital stream signal and number information for specifying an identifier in the table representing a manufacturer of a recording device which records the objects; means for selecting number information specifying a specified identifier in the table; and means for recording the selected number information in the data region.

According to another embodiment of the present invention, an apparatus for reproducing information from an information recording medium for recording a digital stream signal, the recording medium comprising:

a management region for storing control information and a table of identifiers representing manufactures of recording devices; and a data region for storing objects storing portions of the digital stream signal and number information for specifying an identifier in the table representing a manufacturer of a recording device which records the objects in the data region, the method comprises:

means for specifying a device manufacturer having undergone recording operation to the information recording medium based on the number information read from the data region; and means for executing a function unique to a device manufacture in accordance with the specified device manufacturer.

This summary of the invention does not necessarily describe all necessary features so that the invention may also be a sub-combination of these described features.

The invention can be more fully understood from the following detailed description when taken in conjunction with the accompanying drawings, in which:

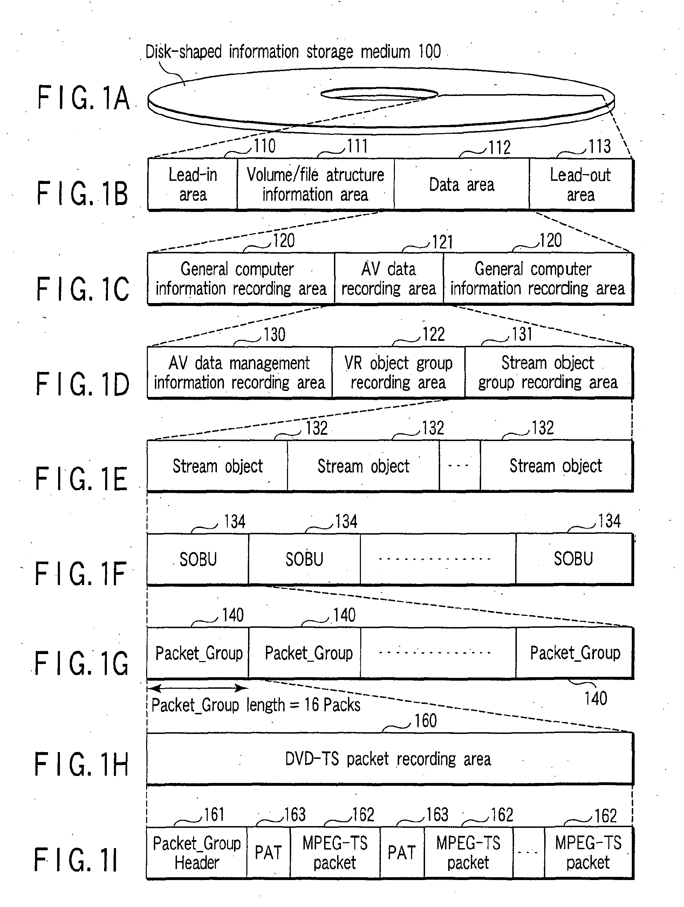

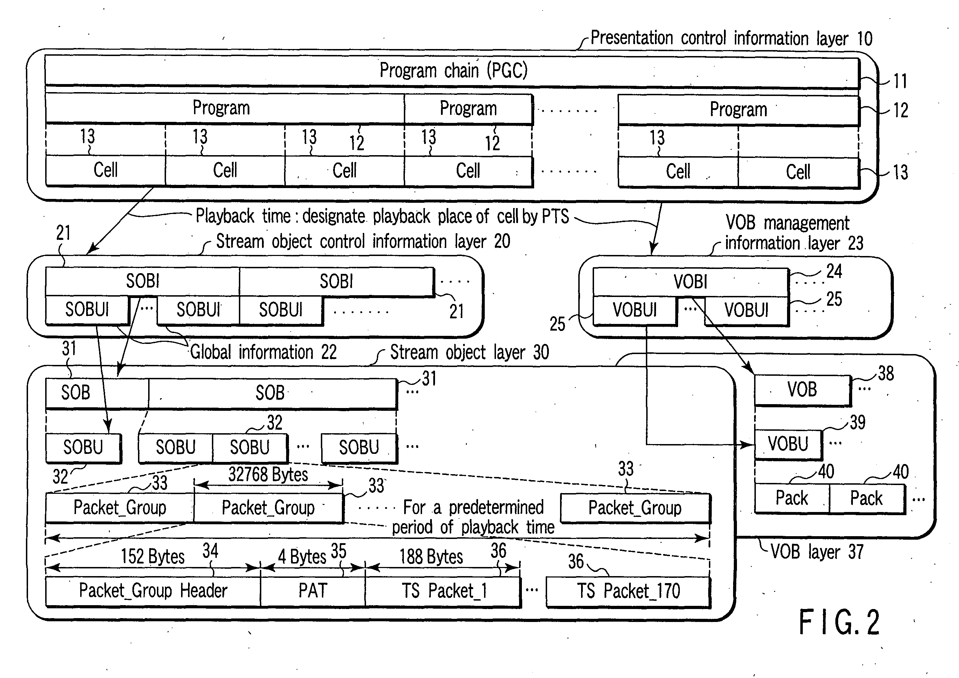

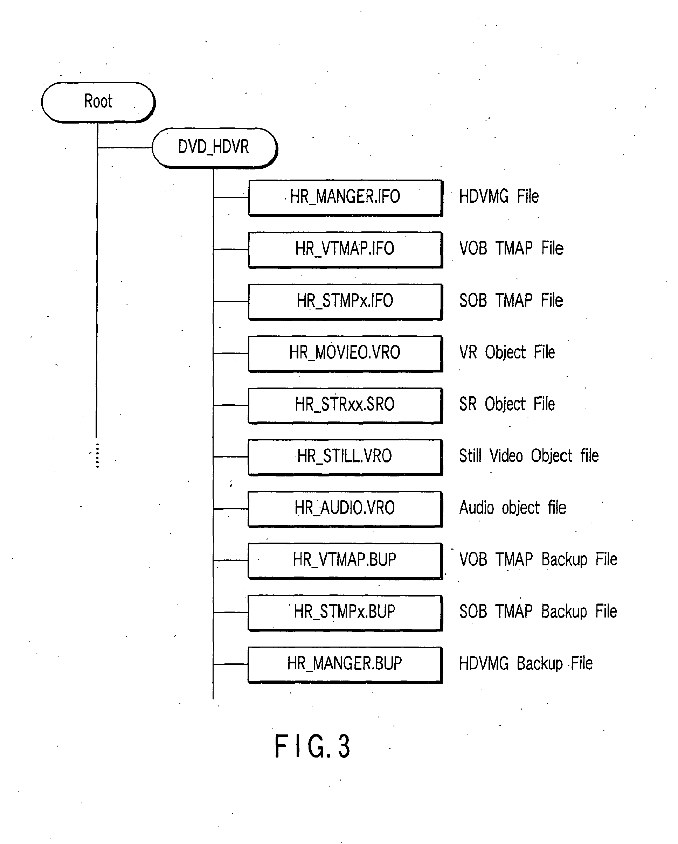

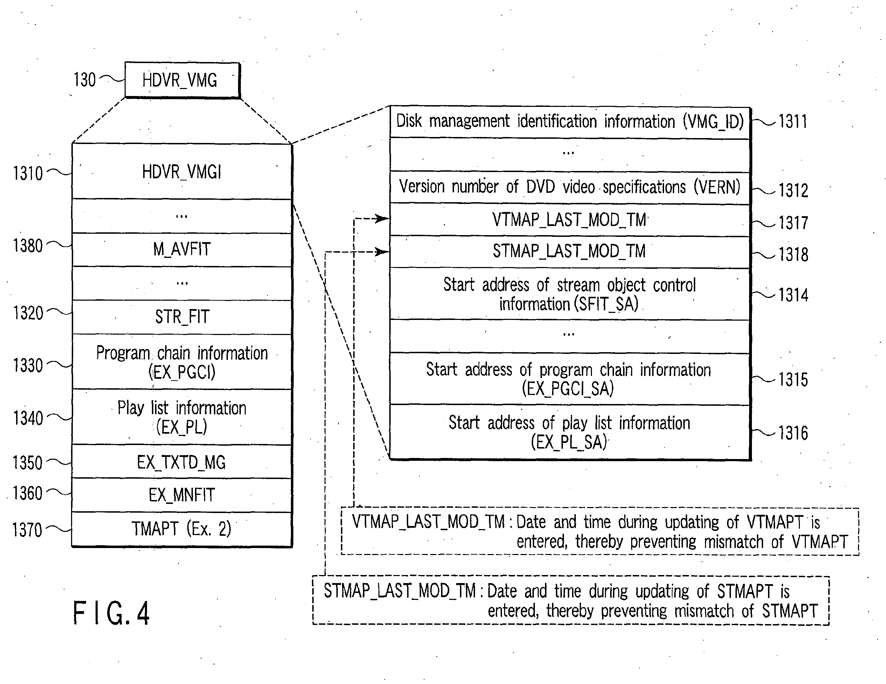

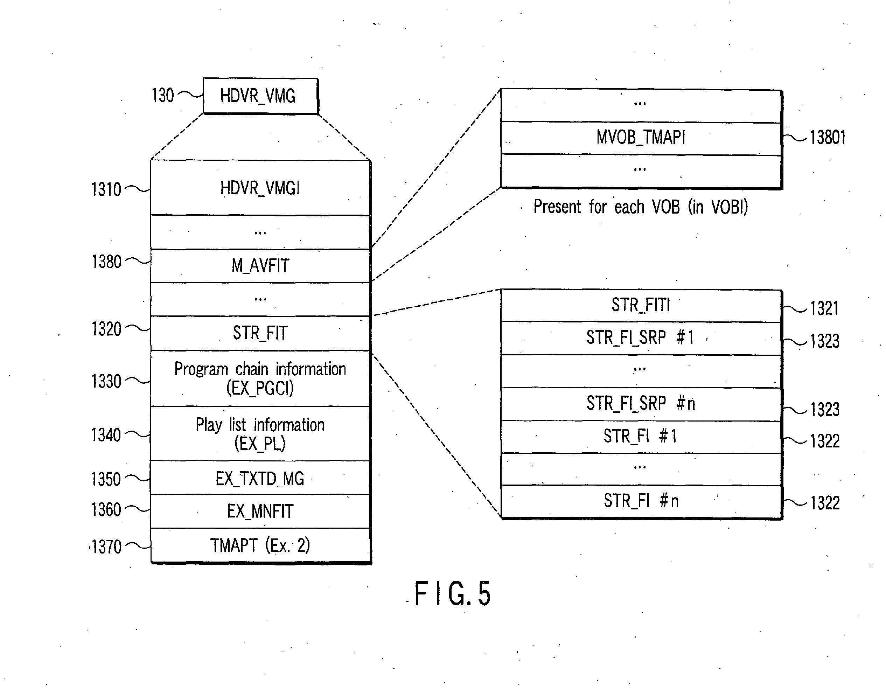

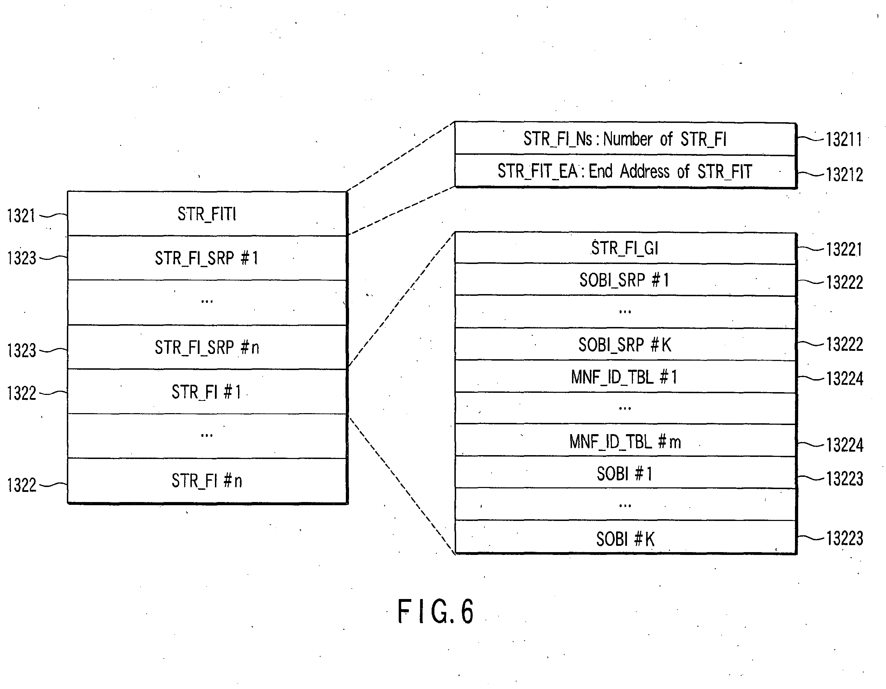

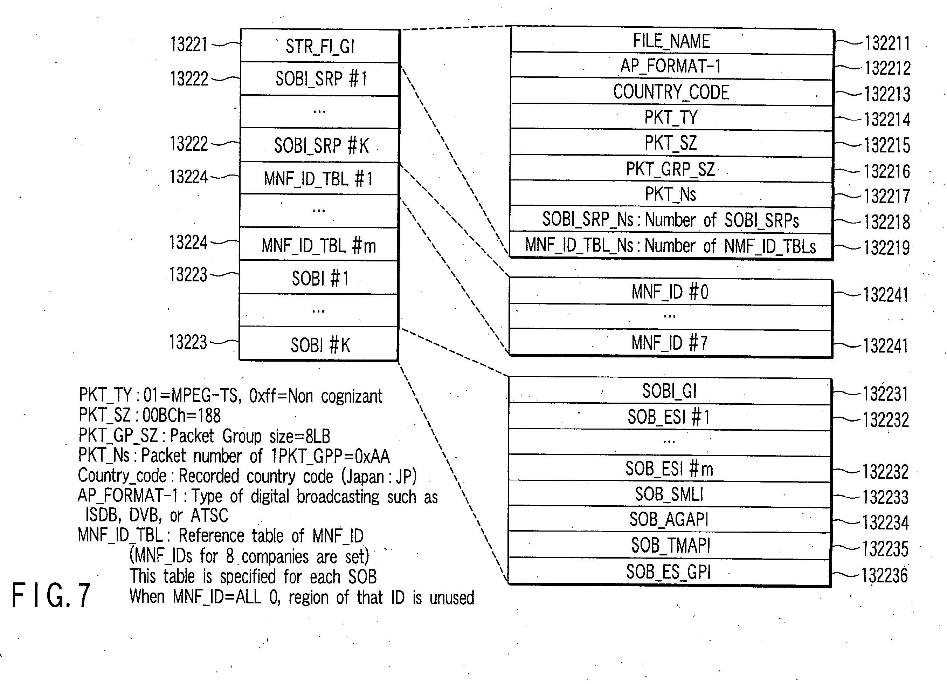

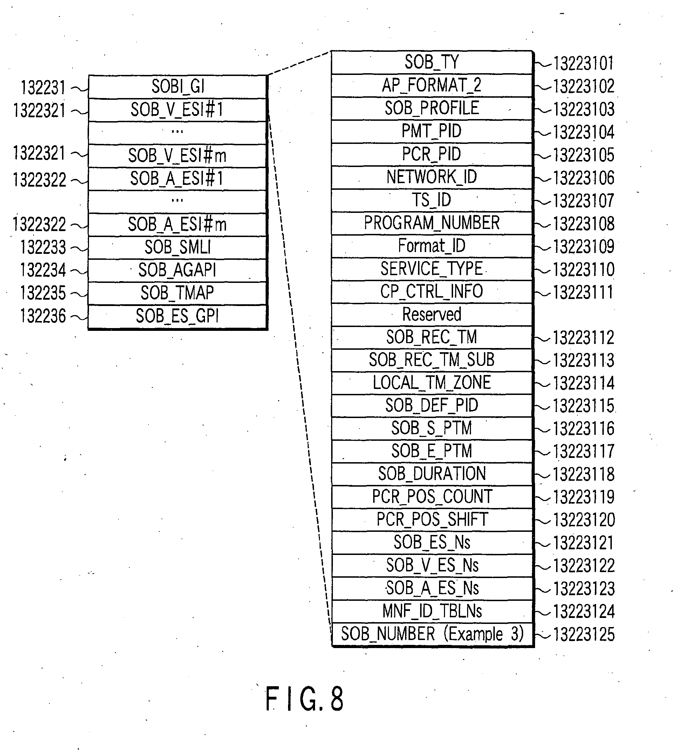

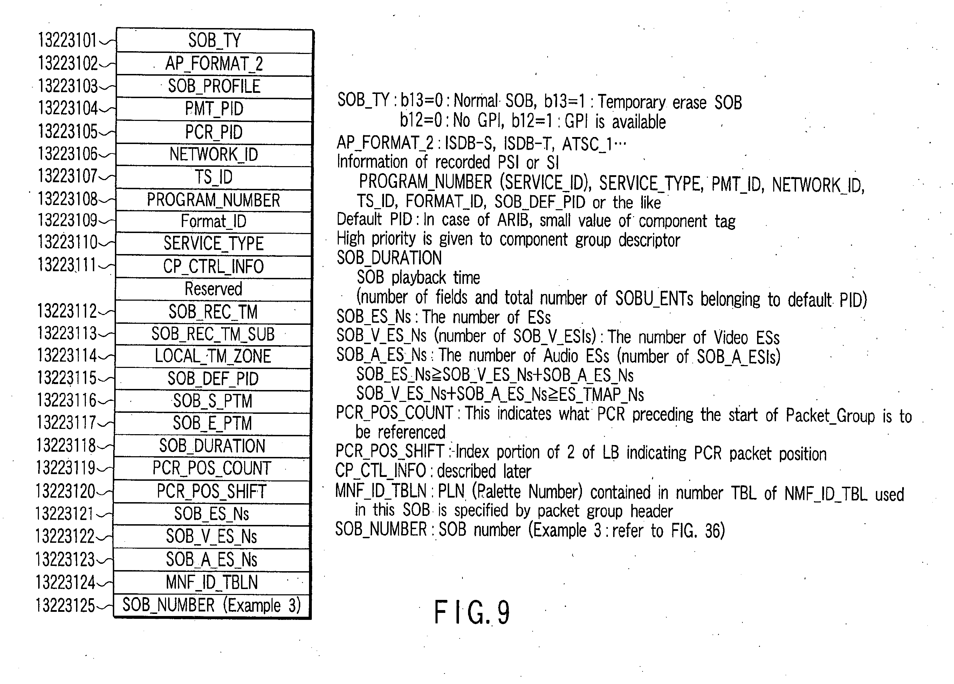

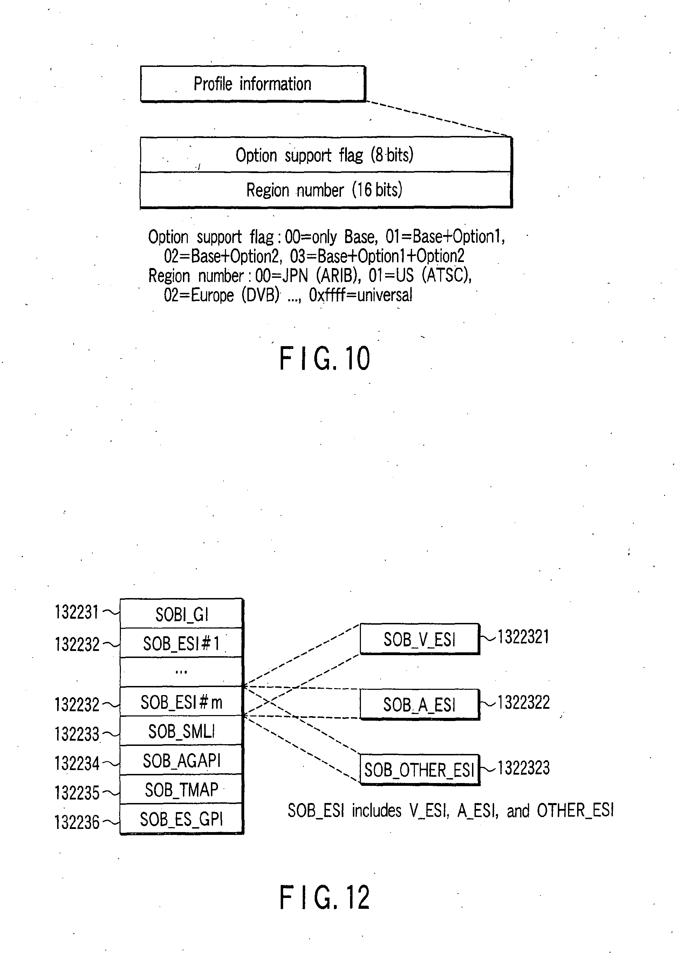

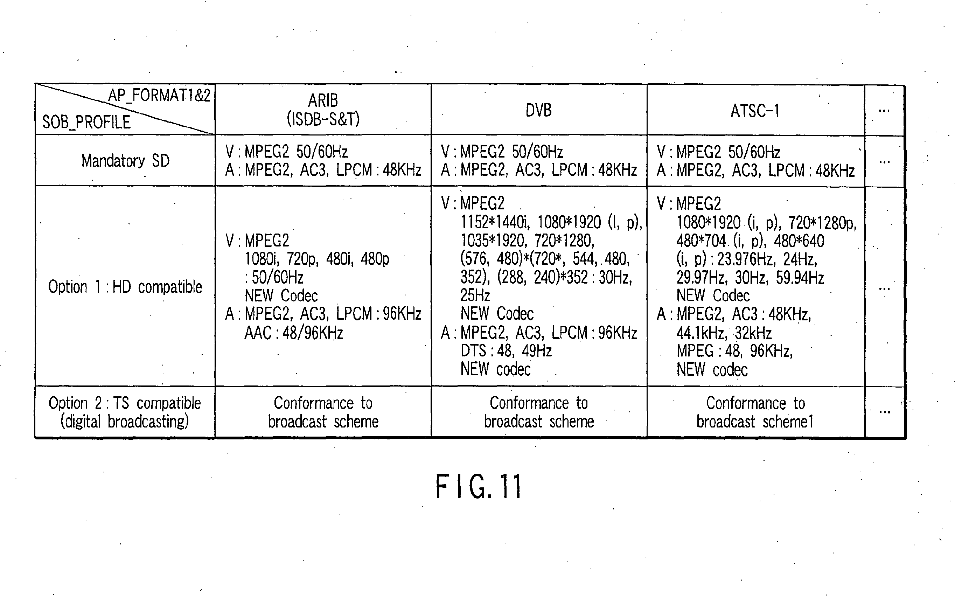

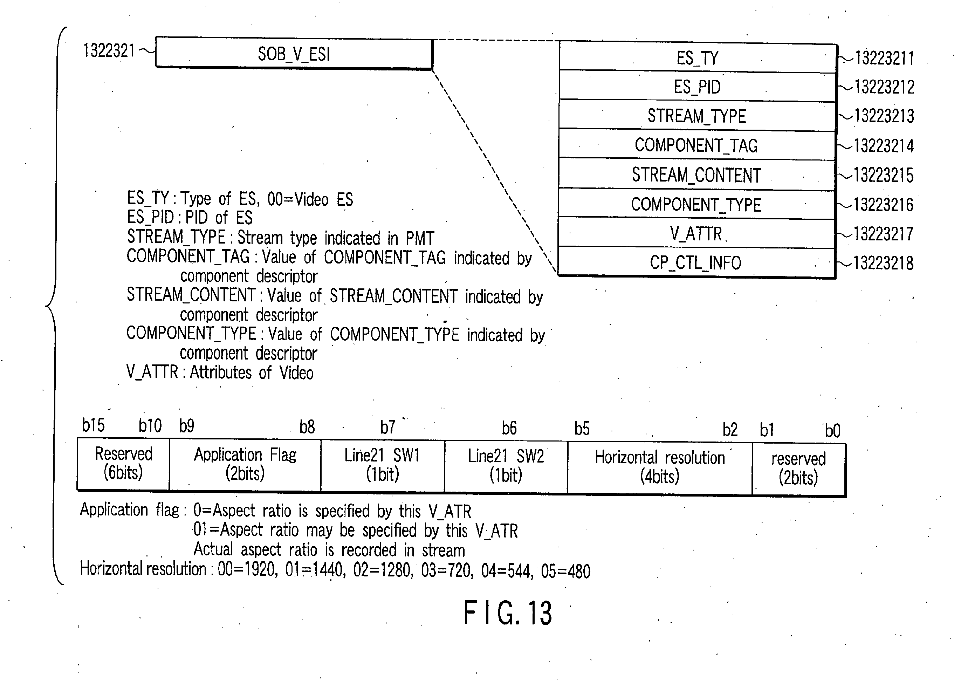

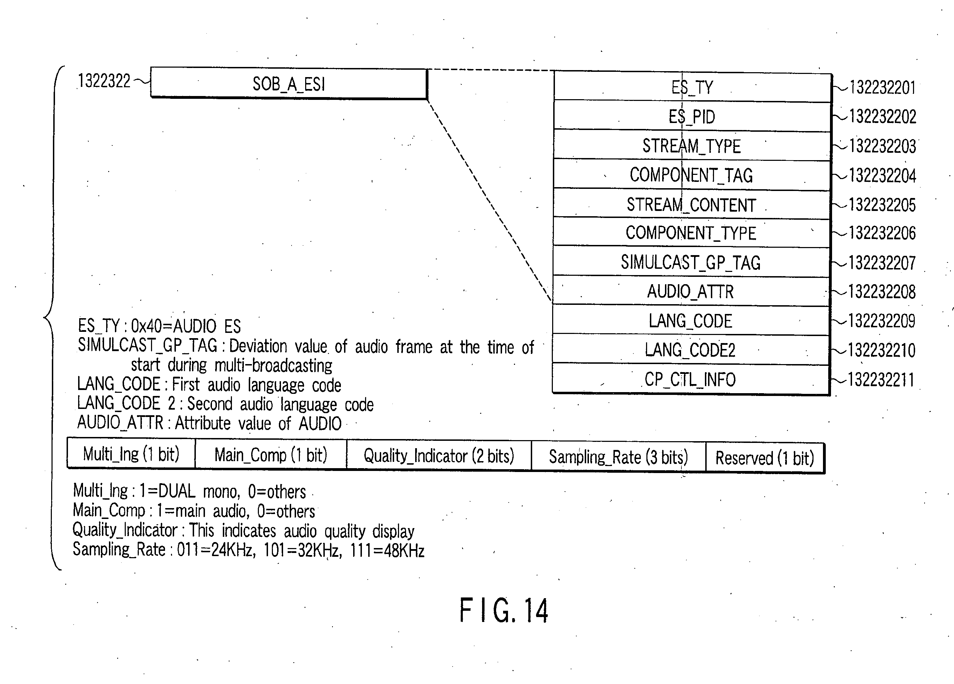

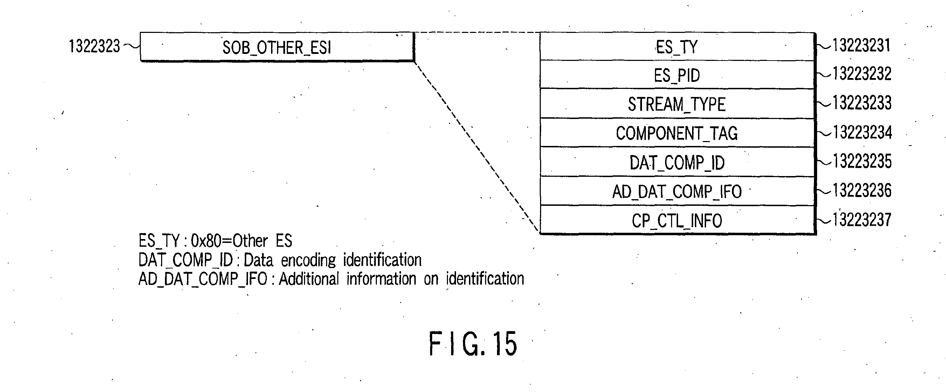

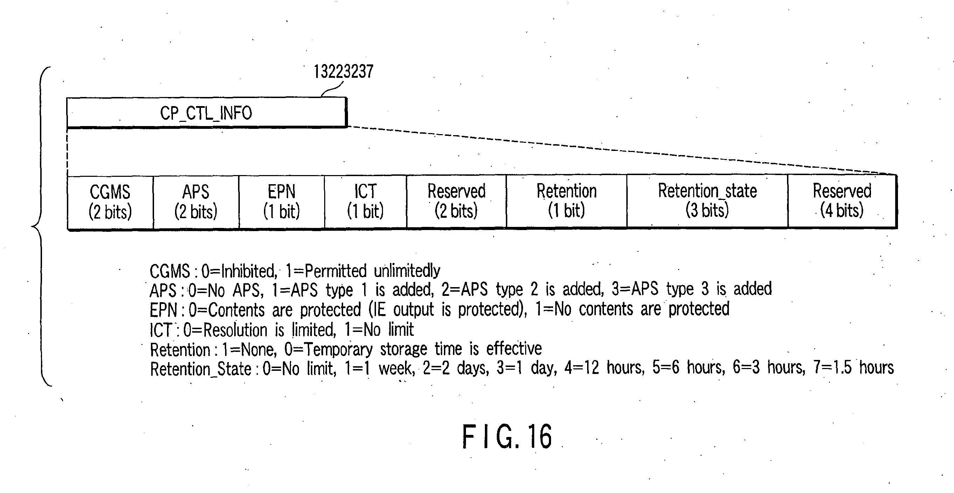

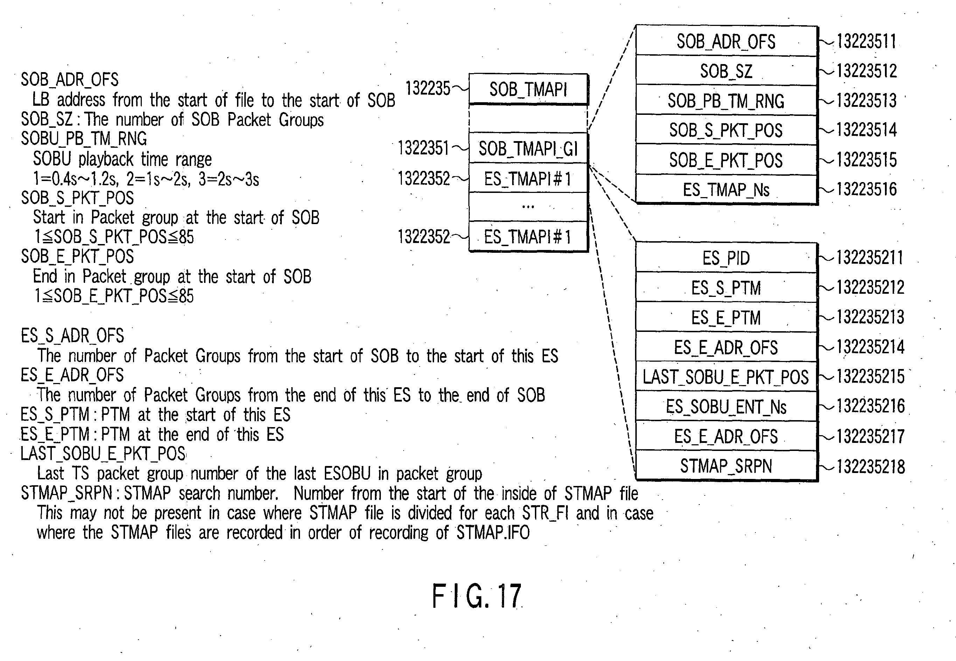

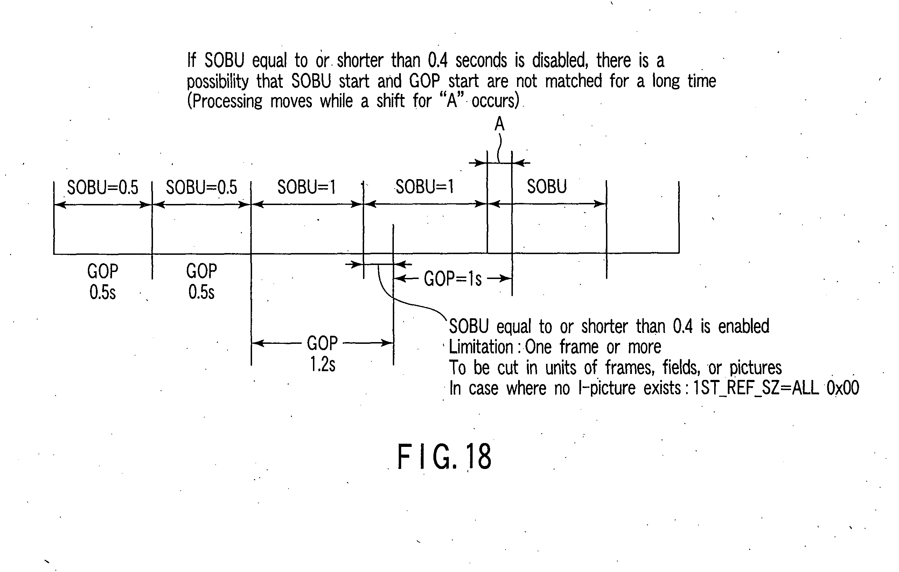

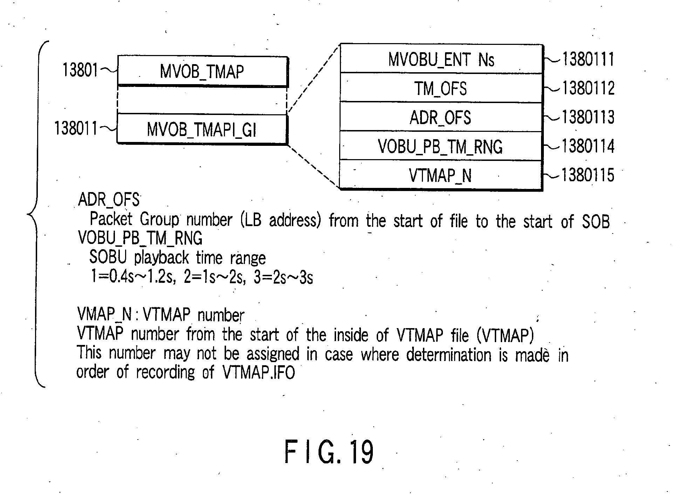

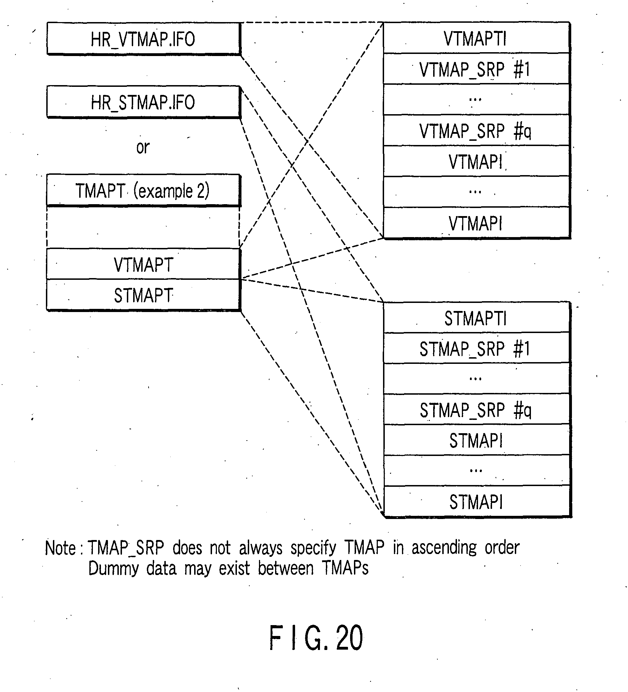

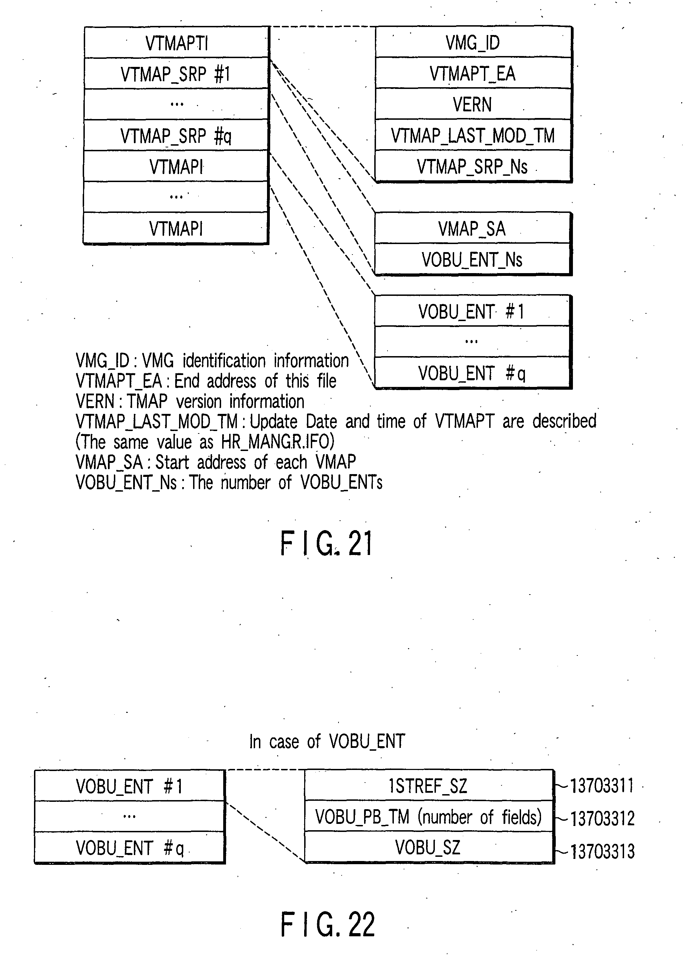

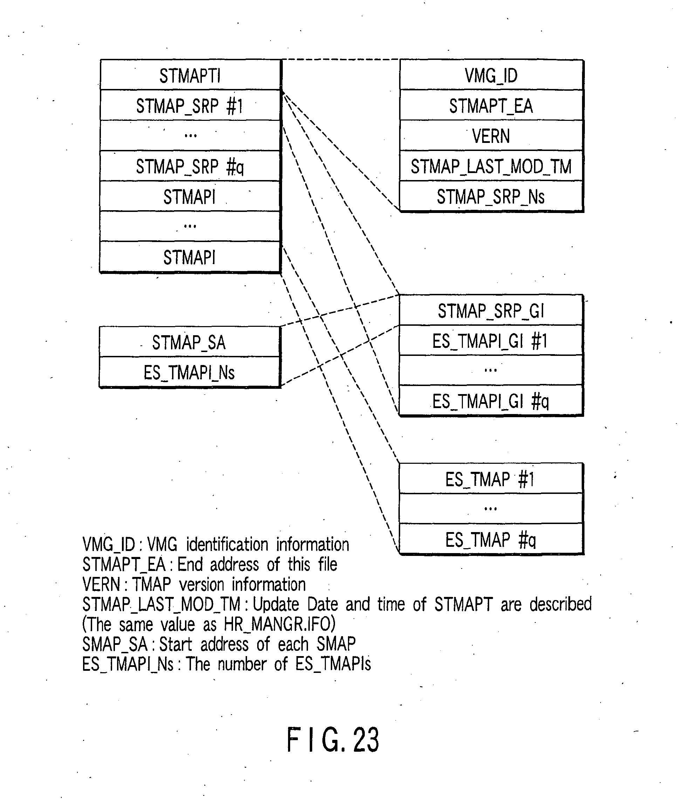

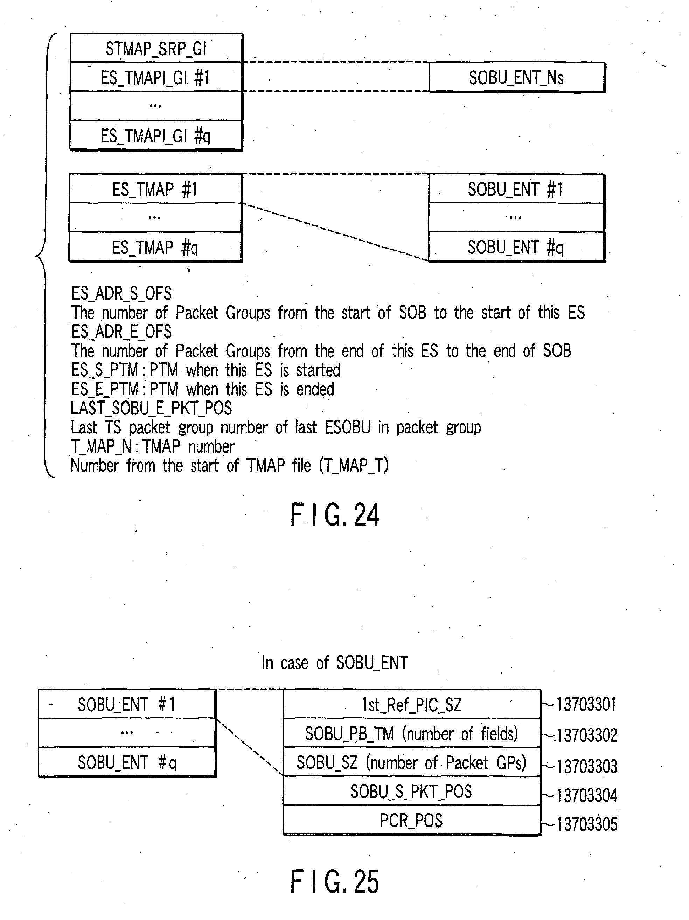

FIGS. 1A to 1I are views each illustrating a data structure according to an embodiment of the present invention; FIG. 2 is a view illustrating a relationship among a presentation control information layer, a stream object control information layer and a stream object layer in the data structure according to the embodiment of the present invention; FIG. 3 is a view illustrating a file structure according to the embodiment of the present invention; FIG. 4 is a view illustrating an example of a part (HDVR_VMGI) of one item (HDVR_VMG) of control information to be recorded in an AV data control information recording region 130, (profile information (Example 1) is included in this HDVR_VMGI); FIG. 5 is a view illustrating an example of other parts (M_AVFIT and STR_FIT) of one item (HDVR_VMG) of control information in the data structure according to the embodiment of the present invention; FIG. 6 is a view illustrating an example of STR_FITI and STR_FI of FIG. 5; FIG. 7 is a view illustrating an example of STR_FI_GI and SOBI of FIG. 6; FIG. 8 is a view illustrating another example of SOBI and an example of SIBI_GI included in this SOBI (profile information is indicated at the end of this SOBI_GI) ; FIG. 9 is a view illustrating a variety of information included in SOBI_GI of FIG. 7; FIG. 10 is a view illustrating a specific example of the profile information of FIG. 8; FIG. 11 is a view illustrating a relationship between an SOBU profile and an AP format; FIG. 12 is a view illustrating an example of SOB_ESI included in SOBI of FIG. 7; FIG. 13 is a view illustrating an example of SOB_V_ESI included in each SOB_ESI (SOB_ESI in this case) and an example of a video attribute V_ATTR included in this SOB_V_ESI; FIG. 14 is a view illustrating an example of SOB_A_ESI included in each SOB_ESI (SOB_ESI in this case) and an example of an audio attribute AUDIO_ATTR included in this SOB_A_ESI; FIG. 15 is a view illustrating an example of SOB_OTHER_ESI included in each SOB_ESI (SOB_ESI in this case); FIG. 16 is a view illustrating another example of copy control information (copyright protection information) CP_CTL_INFO included in SOB_OTHER_ESI of FIG. 15; FIG. 17 is a view illustrating an example of SOB_TMAP included in SOBI of FIG. 7; FIG. 18 is a view illustrating a lower limit of an SOBU recording time; FIG. 19 is a view illustrating an example of MVOB_TMAP_GI included in MVOB_TMAP of FIG. 5; FIG. 20 is a view illustrating an example of a time map file HR_TMAP.IFO (Example 1) included in a DVD_HDVR directory of FIG. 3 or a time map table TMAPT (Example 2) allocated at the end of HDVR_VMG of FIG. 5; FIG. 21 is a view illustrating an example of a variety of information included in VTMAPT of FIG. 20; FIG. 22 is a view illustrating an example of the contents of each SOBU VOBU_ENT (in the case of VOBU_ENT) of FIG. 21; FIG. 23 is a view illustrating an example of a variety of information included in STMAPT of FIG. 20; FIG. 24 is a view illustrating an example of what kind of information is stored in ES_TMAPI_GI and ES_TMAP included in STMAP_SRP and STMAP of FIG. 23;

FIG. 25 is a view illustrating an example of the contents of each SOBU/VOBU_ENT (in the case of SOBU_ENTRY) of FIG. 24;

FIG. 26 is a view illustrating an example of what contents the SOBU shown in FIG. 2 has in the presence or absence of video data and audio data;

FIG. 27 is a view showing an image of SOB in the case of recording a multi-view broadcast;

FIG. 28 is a view showing a structural relationship between a data structure and actual SOBU;

FIG. 29 is a view illustrating an example of SOB_ES_GPI included in SOBI of FIG. 7 (GPI structural example 1);

FIG. 30 is a view illustrating an example of SOB_ES_GPI_GI, GPI_SRP, and GPI included in SOB_ES_GPI of FIG. 29 (GPI structure of Example 1);

FIG. 31 is a view illustrating an example of GPI_GI included in GPI of FIG. 30 (GPI structure of Example 1);

FIG. 32 is a view illustrating an outline of a stream when plural types of broadcast programs are broadcasted;

FIG. 33 is a view illustrating an example (Example 1) of a group configuration of streams when plural types of broadcast programs are broadcasted;

FIG. 34 is a view illustrating an example of PGC information (ORG_EX_PGC information and EX_play list information/UD_EX_PGCT information) included in HDVR_VMG of FIG. 5;

FIG. 35 is a view illustrating an example of EX_PGC information of FIG. 34;

FIG. 36 is a view illustrating an example of how to assign an SOBU number;

FIG. 37 is a view illustrating an example of a data unit (SOBU) for a stream object shown in FIG. 1F or FIG. 2;

FIG. 38 is a view illustrating an example of DCI_CCI included in a packet group header shown in FIG. 37;

FIG. 39 is a view illustrating Examples (Example 1 and Example 2) of each item of copy control information CCI included in DCI_CCI of FIG. 38;

FIG. 40 is a view illustrating an example of MNI 153 included in the packet group header shown in FIG. 37;

FIG. 41 is a block diagram illustrating an example of an apparatus for recording and playing back AV information (such as digital TV broadcast program) in an information recording medium (such as an optical disk or a hard disk) by utilizing the data structure according to the embodiment of the present invention;

FIG. 42 is a flow chart illustrating an example of a whole operation of the apparatus of FIG. 41;

FIG. 43 is a flow chart illustrating an example of an edition process (ST28) shown in FIG. 42 (process flow of editing operation);

FIG. 44 is a flow chart illustrating an example of initial setting (ST10) shown in FIG. 42 (initial setting process flow example 1);

FIG. 45 is a view showing an operation chart for specifying a combination between a basic format (Base) and one or more options (Option 1, Option 2) based on a relationship between a disk used and a recording operation/ playback operation (a relationship chart between an option and a recording/playback operation);

FIG. 46 is a view illustrating an example of delimitation between the base format (Base) and one or more options;

FIG. 47 is a view illustrating another example of delimitation between the base format (Base) and one or more options;

FIG. 48 is a view illustrating a still another example of delimitation between the base format (Base) and one or more options;

FIG. 49 is a flow chart illustrating an example (Example 1) of a recording operation of the apparatus of FIG. 41;

FIG. 50 is a flow chart illustrating an example (Example 2) of a recording operation of the apparatus of FIG. 41;

FIG. 51 is a flow chart illustrating an example of a buffer fetch process (ST130) shown in FIG. 50 (buffer fetch process flow) ;

FIG. 52 is a flow chart illustrating an example of a CPI setting process (ST1330) shown in FIG. 51 (CPI preparation process flow);

FIG. 53 is a view showing a data structure of a packet group in a process (packet group aligning process) of step T13308 of FIG. 52;

FIG. 54 is a flow chart illustrating an example of an MNFI setting process (ST1331) shown in FIG. 51 (MNFI preparation process flow);

FIG. 55 is a flow chart illustrating an example of preprocessing for starting recording into a disk-shaped information storage medium (for example, an optical disk using blue laser) shown in FIG. 1 (recording preprocessing flow);

FIG. 56 is a flow chart illustrating an example of a stream information (ESI) preparation process (ST120) shown in FIG. 50 (ESI setting process flow);

FIG. 57 is a flow chart illustrating an example of a stream file information (STR_FI) preparation process in a recording end process (ST150) shown in FIG. 50 (stream file information preparation process flow with GPI setting process and TMAP setting process);

FIG. 58 is a flow chart illustrating an example (Example 1) of a GPI setting process ST1530 of FIG. 57;

FIG. 59 is a flow chart illustrating a TMAP setting process ST1540 of FIG. 57;

FIG. 60 is a flow chart illustrating an example (Example 1) of an MNF_ID_TBL setting process (ST1535) of FIG. 57;

FIG. 61 is a flow chart illustrating a VOB/SOB structure setting process ST15400 of FIG. 59;

FIG. 62 is a flow chart illustrating a CP_CTL_INFO preparation process ST1220 of FIG. 56;

FIG. 63 is a flow chart (program setting process flow) illustrating an example of a program chain (PGC) preparation process (including a program setting process) in the recording end process (ST150) shown in FIG. 50;

FIG. 64 is a flow chart (whole playback operation flow) illustrating an example of a playback operation of the apparatus of FIG. 41;

FIG. 65 is a flow chart illustrating a decoder setting process ST217 of FIG. 64;

FIG. 66 is a flow chart illustrating an example of a process (ST220) during cell playback of FIG. 64;

FIG. 67 is a flow chart illustrating a buffer data decoder transfer process ST2220 of FIG. 66;

FIG. 68 is a flow chart illustrating an example (Example 1) of a GP switch setting process ST2240 of FIG. 66; and

FIG. 69 is a flow chart illustrating an example of a recording operation setting process ST6070 of FIG. 55.

An embodiment of the present invention will now be described with reference to the accompanying drawings. Expression "and/or" is used in the present specification, intending to indicate a case of "A and B" or case of "A or B". A disk-shaped information recording medium 100 (refer to FIG. 1A) includes a recordable optical disk such as a DVD-RAM using blue laser, a present DVD-RAM, a present DVD-RW, or a present DVD-R; and a recordable magnetic disk such as hard disk.

A further description will be given below by way of example of an optical disk such as a DVD-RAM using a laser of 405 nm to 650 nm, for example.

First Embodiment

FIGS. 1A to 1I are diagrams showing constitutions of an information recording medium, an information recording method, and an information reproducing method according to a first embodiment of the present invention.

FIGS. 1A to 1I are explanatory views of a data structure according to one embodiment of the present invention. Examples of a disc-shaped information storage medium 100 (FIG. 1A) include recordable optical disks such as DVD-RAM, DVD-RW, and DVD-R, and recordable magnetic disks such as a hard disk and the like.

The optical disk 100 has a lead-in area 110, a volume/file structure information area 111, a data area 112, and a lead-out area 113 from an inner peripheral side toward an outer peripheral side thereof (FIG. 1B). A file system is stored in the volume/file structure information area 111. The file system comprises information indicating a file and a place where the file is recorded. Recorded contents are stored in the data area 112 (FIG. 1C).

The data area 112 is divided into a general computer information recording area 120 and an AV data recording area 121. The AV data recording area 121 comprises an AV data management information recording area 130 in which a file (VMG/ESMG file) for controlling AV data is recorded, a VR object group recording area 122 in which an object data (VOBS) file (VRO file) of video recording specifications is recorded, and a stream object group recording area 131 in which a stream object set (SOBS) corresponding to the digital broadcasting is recorded (FIG. 1D). That is, in the embodiment, stream objects of the digital broadcasting are recorded as the stream object set SOBS which is a file separate from a VR object (FIG. 1E).

The SOBS comprises one or more stream objects (SOBs) 132. Each stream object (SOB) 132 comprises a set of one or more stream object units (SOBUs) 134 which are data units constituting access units to the disk 100. Each stream object unit (SOBU) 134 comprises a set of one or more packet groups (Packet_Groups) 140 comprising a set of a plurality of TS packets (FIG. 1G).

In the embodiment, each packet group 140 comprises a set of sixteen packs (or logical blocks: LBs). When one pack (or LB) has a size of 2 Kbytes, a size of each packet group 140 is 32 Kbytes, and the size of the group is the same as that of ECC block defined by the DVD specifications.

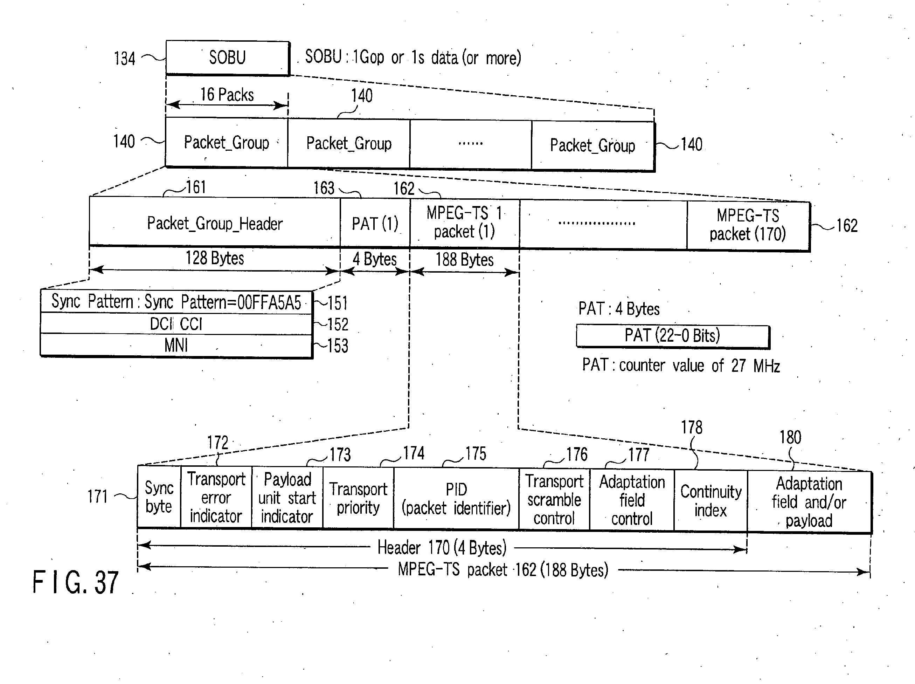

Each packet group 140 constitutes a packet recording area (DVD-TS packet recording area) 160 in stream recording (SR) provided by the present embodiment (FIG. 1H). The DVD-TS packet recording area 160 can comprise a packet group header 161, a plurality of (e.g., 170) MPEG-TS packets 162, and a plurality of (e.g., 170 pieces of) packet arrival time information (PAT) 163 (FIG. 1I). The contents of the packet group 140 will be described later with reference to FIG. 37.

FIG. 2 is an explanatory view of a relation among a presentation control information layer 10, a stream object control information layer 20, and a stream object layer 30 in a data structure according to one embodiment of the present invention. The management information (VMG file) recorded on the AV data management information recording area 130 of FIG. 1D has the presentation control information layer 10 to manage playback procedures of both recording contents based on video recording specifications and stream recording contents based on the present embodiment.

That is, one or more cells 13 which are playback units of a stream-recorded object constitute a program 12 together, one or more cells 13 which are playback units of a video-recorded object constitute a program 12 together, and arrangement (playback procedure) of these programs 12 is managed by management information (PGCI) of a program chain (PGC) 11.

Here, even when the playback is started halfway from the cell 13 on a stream recording, or halfway from the cell 13 on a video recording, a user can designate a playback place by a playback time (PTS).

That is, when the playback is started halfway from the cell 13 on the stream recording at the playback time (PTS), the stream object (SOB) 132 in the stream object layer 30 is designated via stream object information (SOBI) 21 in the stream object control information layer 20. The stream object unit (SOBU) 134 in the stream object layer 30 is designated via stream object unit entry information (SOBUI) 22 in the stream object control information layer 20. When the stream object (SOB) 31 and the stream object unit (SOBU) 32 are designated, the playback start place is specified. The stream object unit information (SOBUI) 22 may be referred to as global information 22.

The stream object unit (SOBU) 32 comprises one or more packet groups 33. The stream object unit (SOBU) 32 corresponds to, for example, one or two GOPs, or is a unit from a head of an i-th I picture to a head of an (i+n)-th I picture (n is an integer). Additionally, when a breakpoint of the GOP is not found, the stream object unit (SOBU) 32 is divided by a unit corresponding to a data amount for one second (playback time) at maximum. Accordingly, overflow of each information field is prevented.

Each packet group 33 comprises 16 LBs (32,768 bytes), the head thereof has a packet group header 34, and thereafter a plurality of (in this example, 170) transport stream packets (TS_Packets) 36, and a plurality of pieces of (in this example, 170) packet arrival time information (PATs) 34. The recording contents of the stream recording are stored in these TS_Packets 36.

On the other hand, when the playback is started halfway from the cell 13 on the video recording at the playback time (PTS), a video object (VOB) 38 in a video object layer 37 is designated via video object information (VOBI) 24 in a video object (VOB) management information layer 23. A video object unit (VOBU) 39 in the video object layer 37 is designated via video object unit information (VOBUI) 25 in the video object management information layer 23. When the video object (VOB) 38 and the video object unit (VOBU) 39 are designated, the playback start place is specified. The video object unit (VOBU) 39 comprises a plurality of packs 40, and the recording contents of the video recording are stored in these packs.

When playback is started from the middle of the cell 13 at the stream recording side, a playback start place can be specified at a time interval based on the number of fields by SOBU_PB_TM. Further, when playback is started from the middle of the cell 13 at the video recording side, a playback start place can be specified according to VOBU_PB_TM (described later in detail with reference to FIG. 22) in time map information (TMAPI) defined in a video recording standard.

A description of what is shown in FIG. 2 is as follows. That is, a structure of SOBS (Stream Object Set) comprises one or more SOBs (Stream OBjects). This SOB corresponds to one program, for example. The SOB comprises one or more SOBUs (Stream Object Unit). This SOBU corresponds to object data for a predetermined time interval (refer to VOBU/SOBU_PB_TM_RNG (changing according to a value of VOBU/SOBU Playback Time Range) in FIG. 19/FIG. 17 described later) or one or more items of GOP data.

However, when a transfer rate is low, one GOP may not be sent within one second (In a DVD-VR in which an analog video input is MPEG-encoded in an apparatus, a structure of a data unit can be freely set because of its internal encoding. However, in the case of digital broadcasting, data is encoded in the broadcast station side, and thus, it may be unknown as to what type of data is transmitted).

In addition, there may be a case in which a transmission rate is high, and an intra (I) picture is frequently sent. In that case, SOBU is frequently delimited, and concurrently, SOBU control information increases, so that there is a danger that whole control information becomes large in amount. Therefore, it is proper that SOBU according to the embodiment of the present invention be delimited at a predetermined time interval (the lowest limit must be other than SOBU at the end of SOB, and SOBU is delimited in units of pictures) or one or more GOPs.

One SOBU 32 comprises one or more packet groups 33, and each packet group comprises 16 packs (one pack = one LB: 2,048 bytes in size) (32,640 bytes). In addition, the packet group 33 comprises a packet group header 34 and TS packets.36 (170 packets). An arrival time of each TS packet 36 can be recognized from PAT (Packet Arrival Time: 4 bytes) allocated in pair in front of each TS packet 36.

Now, control information will be described with reference to FIGS. 3 to 41. FIG. 3 is a view illustrating a file structure according to the embodiment of the present invention. As described in FIG. 1B, the data' contained in the disk 100 comprises a volume/file structure information area 111 storing a file system and a data area 112 for actually recording a data file. The file system stored in the volume/file structure information area 111 comprises information indicating what file is recorded and where such file is recorded, as shown in FIG. 3. The data area 112 is divided into a general computer information recording area 120 and an AV data recording area 121. The AV data recording area 121 comprises an AV data management information area 130 in which an HDVMG file (and a backup file thereof) for controlling the recorded AV data is recorded; a VR object group recording area 122 for recording an object data (VOBS) file (VRO file) in accordance with video recording specifications; and a stream object group recording area 131 for recording an object (SOBS) compatible with digital broadcasting.

Here, information of respective formats are stored in different directories such that DVD-Video (ROM Video) information are stored in a VIDEO-TS directory and DVD-RTR (recording/playback DVD) information are stored in a DVD-RTR directory, for example. Information of the present DVD specifications compatible with a digital broadcasting are also recorded in a directory called DVD_HDVR, for example.

Namely, as shown in FIG. 3, the directory called DVD_HDVR records: VMG files for controlling data (HR_MANAGER.IFO and its backup HR_MANGER.BUP); a VRO file (HR_MOVIEO.VRO) which is an object file for analog AV information recording such as analog broadcasting and analog line input; SRO files (HR_STRxx.SRO; x = 0, 1, 2, ...) which is an object of digital broadcasting; a still object file (HR_STILL.VRO); and an audio object file (HR_AUDIO.VRO). Here, an object of the SRO file is defined as SOBS.

In FIG. 3, a time map file (HR_VTMAP.IFO) shown as Example 1 and its backup file (HR_VTMAP.BUP) are further recorded as independent files. These files (HR_VTMAP.IFO and HR_VTMAP.BUP) can store information contained in the time map table TMAPT (Namely, TMAPT can be controlled as a file independent of another item of control information).

As shown in FIG. 3, SR (stream recording) control data is recorded in an HDVMG file common to VR (video recording), and is controlled to be common to VR. As shown in FIG. 2, the SR control data and VR control data are linked with each other in units of cells, and a playback place is specified in units of playback time intervals.

In the DVD_HDVR directory, although not shown, HR_THNL.DAT can be provided as a file for thumbnail (reduced image) which can be utilized for a chapter menu or the like. Further, although not shown, additional text file: HR_TEXT.DAT other than item text (IT_TXT) or HR_EXEP. DAT for storing information added to an entry point (EP) can be provided in the DVD_HDVR directory.

As shown in FIG. 3, a method for adding TMAPT shown as Example 2 to the end of HDVR_VMG, as shown in FIG. 4, instead of defining TMAPT as another file, can also be used.

FIG. 4 is a view illustrating an example of how a part (HDVR_VMGI), one item of the control information (HDVR_VMG) recorded in the AV data control information recording region 130, is configured. Stream recording in the present embodiment is abbreviated as SR (Stream Recording), and video recording is abbreviated as VR (Video Recording). Then, SR data control information (STR_FIT: Stream File Information Table) is stored in the HDVR_VMG 130 (in HR_MANGER.IFO of FIG. 3), and is stored in the same manner as VR data.

The HDVR_VMG 130 comprises: video manager information (HDVR_VMGI) 1310; a stream file information table (STR_FIT) 1320; (original) program chain information (PGCI) 1330; play list information (PL) 1340; a text data manager (TXTD_MG) 1350; a manufacturer information table (MNFIT) 1360; a_time map table TMAPT '(Example 2) 1370; and a movie AV file information table (M_AVFIT) 1380. The STR_FIT (Stream File Information Table) 1320 is added to control information conforming to conventional DVD-VR specifications.

Here, it is important that a position of the TMAPT 1370 is present at the end of the HDVR_VMG 130. That is, since the position of the TMAPT 1370 is present at the end of the HDVR_VMG 130, there is no need for rewriting the HDVR_VMGI 1310 to EX_MNFIT 1360 every time even if TMAP is frequently rewritten and its data size increases or decreases.

In other words, a DVD recorder has time map information (TMAPI) as VOB control information. This information is intended to enable playback, specific playback and the like in units of object data (VOB/SOB) divided into data units (VOBU/SOBU), and one item of information is required for every 0.5 seconds at a maximum. Thus, when a disk space increases or a compression scheme with high compression efficiency is employed in the future, an amount of time map information TMAPI increases, and processing becomes complicated in the case where an edition process or the like is performed. If this TMAPI is present in a control information file (HR_MANGER. IFO of FIG. 3), there is a need for moving or rewriting control data contained in irrelevant another region, and efficiency is worsened.

Therefore, in the embodiment of the present invention, TMAPI is recorded in another region (such as HR_TMAP.IFO of FIG. 3 or TMAPT allocated at the end of HDVR_VMG of FIG. 4) in order to improve such a situation.

In FIG. 4, the HDVR_VMGI 1310 comprises: disk management identification information (VMG_ID) 1311; a version umber of DVD video specifications (VERN) 1312; VTMAP_LAST_MOD_TM 1317 for describing an update date and time of VTMAPT; STMAP_LAST_MOD_TM 1318 for describing an update date and time of STMAPT; a start address of stream object control information (SFIT_SA) 1314; a start address of program chain information (EX_PGCI_SA) 1315; and a start address of play list information (EX_PL_SA) 1316. SR stream control information is stored in the STR_FIT 1320.

In an example of FIG. 4, each TMAP is defined as a file other than VMG, and each file can be individually modified by a personal computer or the like. In order to prevent such modification, VTMAPT update date and time information which is TMAP (Time Map) for self recording/playback of VR (video recording) and STMAPT update date and time information which is TMAP (Time Map) for digital broadcast recording of stream recording (SR) are described, and this value and the update date and time information described in each TMAP file are compared with each other. If the values are equal to each other, processing can be performed, assuming that a coincidence is obtained.

FIG. 5 is a view illustrating an example of how other parts (M_AVFIT 1380 and STR_FIT 1320), one item (HDVR_VMG 130) of the control information, is configured in the data structure according to the embodiment of the present invention. VR data control information and SR stream control information are stored in the HDVR_VMG 130, whereby stream data is managed in the same manner as VR data. That is, VR data control information is stored in the M_AVFIT (Movie AV File Information Table) 1380, and the M_AVFIT 1380 includes MVOB_TMAPI (Movie Video Object Time Map Information) 13801 in VOBI (Video Object Information) for each VOB. In addition, stream control information is stored in the STR_FIT (Stream File Information Table) 1320, and the STR_FIT 1320 comprises STR_FITI (STR_FIT Information) 1321, one or more STR_FI_SRPs #1 to #n 1323, and one or more items of STR_FI (Stream File Information) #1 to #n 1322 indicated by its SRP. In addition, each STR_FI 1322 includes SOB_TMAPI having a function compatible with MVOB_TMAPI 13801 in its data hierarchy (described later with reference to FIG. 7).

FIG. 6 is a view illustrating an example of how the STR_FITI 1321 and STR_FI 1322 of FIG. 5 are configured. That is, the STR_FITI 1321 comprises: the total number of STR_FIs 1322 (STR_FI_Ns 13211); and an end address (STR_FIT_EA 13212) of this table (STR_FIT 1320).

Further, the STR_FI 1322 comprises: STR_FI_GI (STR_FI General Information) 13221; one or more SOBI_SRP (Stream Object Information Search Pointer) 13222; SOBI (SOB Information) #1 to #n 13223 equal to this SOBI_SRP #1 to #n 13222 in number and indicated by that value; and one or more MNF_ID_TBL (MNF_ID: Recorder manufacturer ID: up to eight manufacturers can be recorded) 13224.

FIG. 7 is a view illustrating an example of how the STR_FI_GI 13221 and SOBI 13223 of FIG. 6 are configured. The SRT_FI_GI 13221 comprises: a file name/file number 13221 of an object managed by this STR_FI; a digital broadcast type (AP_FORMAT_1) 132212 which is a source of contents to be recorded; COUNTRY_CODE 132213; PKT_TY (1 = MPEG-TS) 132214: PKT_SZ 132215; PKT_GRP_SZ 132216 (fixed to 16 logical blocks); PKT_Ns 132217 (0xAA: fixed to 170 TS packets); the number of SOBI_SRPs 13222 (SOBI_SRP_Ns) 132218 contained in this STP_FI; and the number of MNF_ID_TBLs 13224 (MNF_ID_TBL_Ns) 132219. For SOB, a table used in the above NF_ID_TBL 13224 is selected (or registered), and one TBL is utilized by selecting it.

If the PKT_TY 132214 is 01 in its contents, it is indicated that a stream included in a packet is MPEG-TS stream. If it is 0xff, it is indicated that a stream in a packet cannot be recognized or analyzed (Non-cognizant). If the PKT_SZ 132215 is 00Bch, it is indicated that a packet size is 188 bytes.

The PKT_GRP_SZ 132216 indicates what is the size of packet group (such as 8 logical blocks or 16 logical blocks). According to the illustration of FIG. 1G, PKT_GRP_SZ = 16 logical blocks is fixed. The PKT_Ns 132217 indicates the number of packets in one packet group (for example, 0xAA: fixed to 170 TS packets). The COUNTRY_CODE 132213 indicates a code of country to which a device having undergone recording (such as recorder) is to be sold or distributed (for example, JPN = Japan). If the AP_FORMAT_1 132212 is 1 in its contents, it is indicated to be ARIB (ISDB). If it is 2, it is indicated to be ATSC, and if it is 3, it is indicated to be DVB.

Further, SOBI #1 to #k 13223 indicated by SOBI_SRP #1 to #k 13222 of corresponding number comprises: SOBI_GI (SOBI General Information) 132231; one or more SOB_ESI #1 to #m (SOB Elementary Stream Information) 132232; SOB_SMLI (SOB Seamless Information) 132233; SOB_AGAPI (SOB Audio GAP Information) 132234; SOB_TMAPI (SOB Time Map Information) 132235; and SOB_ES_GPI (SOB Elementary Stream Group Information) 132236. Here, the SOB_ESI 132232 further comprises: SOB_V_ESI (SOB Video ESI) #1 to #m 1322321 and SOB_A_ESI (SOB Audio ESI) #1 to #m 1322322 (refer to FIG. 8).

FIG. 8 is a view illustrating an example of how the SOBI_GI 132231 included in SOBI of FIG. 7 is configured. As shown in FIG. 8, the SOBI_GI 132231 comprises: AP_FORMAT_2 13223102 (when ARIB is specified by AP_FORMAT_1, 1 = ISDB-S (BS/CS broadcasting), 2 = ISDB-T (terrestrial digital broadcasting)); PROGRAM_NUMBER (SERVICE_ID) 13223108 further based on PSI or SI value; PMT_PID (PMT packet ID) 13223104; PCR_PID (PCR packet ID) 13223105; NETWORK_ID (network ID) 13223106; TS_ID (transport stream ID) 13223107; FORMAT_ID (format ID) 13223109; SOB_ES_Ns (the number of ESs selected for recording) 13223121 based on data to be recorded; SOB_V_ES_Ns (the number of ESs having prepared TMAP from among recorded video ES) 13223122; SOB_A_ES_Ns (the number of ESs having prepared TMAP from among recorded audio ES) 13223123; PCR_POS_COUNT 13223119 (indicating which PCR preceding the start of Packet_Group is referred to); PCR_POS_SHIFT (index portion of 2 of LB indicating a position of PCR packet) 13223120; CP_CTRL_IFO (copy control information) 13223111; SOB_PROFILE (profile information) 13223103 (support state of decode function, composed of region number and optional support flag: described later with reference to FIG. 10); and MNF_ID_TBLN 13223124. The MNF_ID_TBLN 13223124 corresponds to a TBL number set in an STR_FI hierarchy, and the TBL number which SOB uses is set.

The SOBI_GI further includes: SOB_TY (SOB type) 13223101; SOB_DEF_PID (SOB default PID) 13223115; SOB_REC_TM (SOB recording time) 13223112; SOB_REC_TM_SUB (SOB recording subsidiary time) 13223113; LOCAL_TM_ZONE (local time zone) 13223114; SOB_DURATION (SOB duration) 13223118; SOB_S_PTM (SOB start time) 13223116; and SOB_E_PTM (SOB end time) 13223117.

FIG. 9 is a view illustrating a variety of information included in SOBI_G1 132231 of FIG. 8. In SOB_TY 13223101, when bit B13 is 0, it is indicated to be normal SOB. When bit b13 is 1, it is indicated to be SOB in a temporarily erased state. When bit B12 is 0, it is indicated that GPI is not present. When bit b12 is 1, it is indicated that GPI is present.

In addition, SOB_ES_Ns 13223121; SOB_V_ES_Ns 13223122; SOB_A_ES_Ns 13223123; and ES_TMAP_Ns 13223516 (refer to FIG. 17) have a relationship indicated by the following formula:

$$SPB\_ES\_Ns \geqq SOB\_V\_ES\_Ns + SOB\_A\_ES\_Ns,$$

$$SOB\_V\_ES\_Ns + SOB\_A\_ES\_Ns \geqq ES\_TMAP\_Ns$$

In addition, it is indicated as to what PCR preceding the start of packet group is referred to by PCR_POS_COUNT. An index portion of 2 of LB indicating a position of a PCR packet is indicated by PCR_POS_SHIFT. A copy control is made for the purpose of copyright protection or the like by CP_CTRL_INFO.

When Default PID (SOB_DEF_PID) is ARIB, it indicates a small vale of a component tag (Note that a high priority is given to a value of a component group descriptor). SOB_DURATION indicates SOB playback time and corresponds to a total playback time of SOBU_ENT which belongs to ES indicated by default PID.

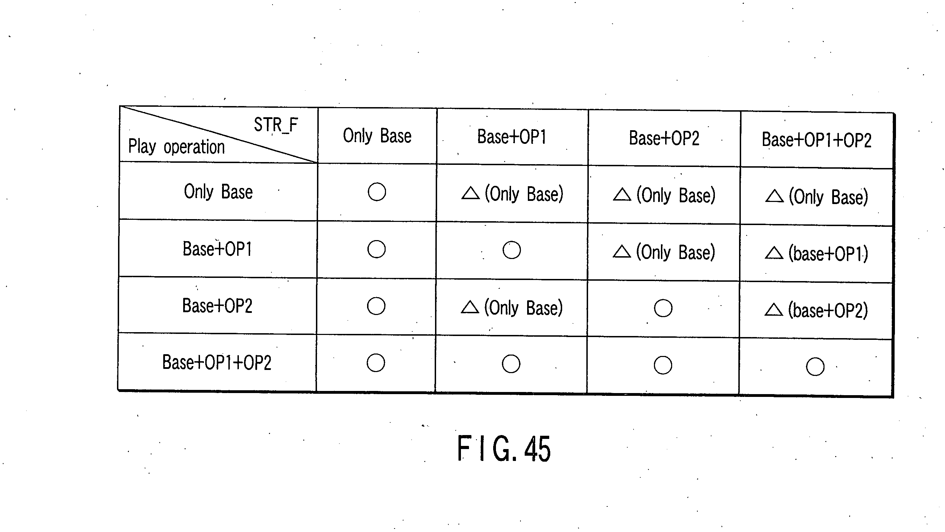

FIG. 8 and FIG. 9 show a case in which profile information is not provided on a disk by disk basis, and profile information is provided on a SOB by SOB basis in each disk. Profile information 13223103 included in FIG. 8 will be described in detail with reference to FIG. 10 and will be described later with reference to FIG. 45. This information can be used to indicate support states of a variety of decode functions. FIG. 10 is a view illustrating a specific example of profile information of FIG. 8 and FIG. 9. This profile information comprises an 8-bit option support flag and a 16-bit region number. With respect to this region number, 00 indicates Japan (ARIB); 01 indicates the US (ATSC); 02 indicates Europe (DVB); and 0xffff indicates a universal code common to all countries in the world. In this manner, region data corresponding to the region number can be played back as the recorded contents.

In general, in DVD, all the registered compression formats must be played back in principle, thereby obtaining DVD recorder compatibility between manufacturers. However, in the next generation DVD (HD-DVD), plural types of video formats are registered. Thus, if all the formats must be played back, all devices become very expensive DVD recorders.

In order to improve this problem associated with expensiveness, a format compatible function is divided into a base and a plurality of options, and options to be supported are used depending on purposes or prices. In this case, the above information is included in contents so that one's own support state and stream state can be compared with each other when data on options which are not supported is generated. In the embodiment of the present invention, in such a state, the related option state (option support flag of FIG. 10) is inserted into VMGI, thereby making it possible to provide a DVD recorder compatible with a variation of plural potions (described later'in detail with reference to FIG. 45).

In the meantime, digital broadcast schemes are different depending on country. For example, in Japan, ARIB (Association of Radio Industries and Businesses) is used; in Europe, DVB (Digital Video Broadcasting) is used; and in US, ATSC (Advanced Television Systems Committee) is used.

[1] In ARIB, the video is MPEG-2; the resolution is 1080 (i), 720 (p), 480 (i), 480 (p); the frame rate is 29.97 Hz, 55.94 Hz, the audio is AAC (MPEG-2 Advanced Audio Coding); and the sampling frequency is 48 kHz, 44.1 kHz, 32 kHz, 24 kHz, 22.05 kHz, 16 kHz.

[2] In DVD, the video is MPEG-2; the resolution is 1152 × 1440 (i), 1080 × 1920 (i, p), 1035 × 1920, 720 × 1280, (576, 480) × (720, 544, 480, 352), (288, 240) × 352; the frame frequency is 30 Hz, 25 Hz; the audio is MPEG-1 audio, MPEG-2 Audio; and the sampling frequency is 32 kHz, 44.1 kHz, 48 kHz.

[3] In ATSC, the video is MPEG-2, the resolution is 1080 × 1920 (i, p), 720 × 1280 (p), 480 × 704 (i, p), 480 × 640 (i, p); the frame frequency is 23.976 Hz, 24 Hz, 29.97 Hz, 30 Hz, 59.94 Hz, 60 Hz, the audio is MPEG-1 Audio Layer 1 & 2 (DirecTV), AC3 Layer 1 & 2 (Primstar); and the sampling frequency is 48 kHz, 44.1 kHz, 32 kHz.

FIG. 11 shows that the above settings are provided in a correlation table.

From this fact, decoders to be mounted on recorders are different depending on a region to be used. Thus, VMGI stores information indicating what is supported by a recorder used for a disk recorded by a recorder (region code of FIG. 9) so as to indicate what is written in a disk by the supported recorder.

In addition, streams to be decoded at the apparatus side include a number of variations which are different depending on regions. If all of these variations are supported, a configuration of a recording and playback apparatus (such as DVD recorder) becomes very huge (or complicated). As a result, apparatus cost becomes high. Therefore, in the present embodiment, contrivance is made to streams while compatibility with a number of variations is maintained, thereby making it possible to relatively lighten (simplify) device configuration.

FIG. 12 is a view illustrating an example of how SOB_ESI included in SOBI of FIG. 7 is configured. In this example, SOB_ESI is divided into three types (SOB_V_ESI 1322321, SOB_A_ESI 1322322, and SOB_OTHER_ESI 1322323).

FIG. 13 is a view illustrating an example of how SOB_V_ESI included in each SOB_ESI of FIG. 12 is configured and an example of how a video attribute V_ATTR included in this SOB_V_ESI is configured.

The SOB_V_ESI 1322321 comprises: ES_TY 13223211 indicating a format of ES; ES_PID 13223212 indicating PID of ES; STREAM_TYPE (STREAM type indicated in PMT) 13223212; COMPONENT_TAG (value of COMPONENT_TAG indicated by component descriptor) 13223214; COMPONENT_TYPE (value of COMPONENT_TYPE indicated by component descriptor) 13223216; V_ATTR 13223217 indicating a video attribute; and CP_CTL_INFO (copy control information/copyright control information) 1322318.

V_ATTR (16 bits) 13223217 comprises an application flag for specifying a video aspect ratio and data or the like indicating a horizontal resolution.

In general, playback is performed in accordance with this attribute. However, when a change occurs in the middle of SOB, a high priority is given to a value of DCI in a packet header.

FIG. 14 is a view illustrating an example of how SOB_A_ESI included in each SOB_ESI of FIG. 12 is configured and an example of how an audio attribute AUDIO_ATTR included in this SOB_A_ESI is configured.

The SOB_A_ESI 1322322 comprises: ES_TY 132232201 indicating a type of ES; ES_PID 132232202 indicating PID of ES; STREAM_TYPE (STREAM type indicated in PMT) 132232203; COMPONENT_TAG (value of COMPONENT_TAG indicated by component descriptor) 132232204; STREAM_CONTENT (value of STREAM_CONTENT indicated by component descriptor) 132232205; COMPONENT_TYPE (value of COMPONENT_TYPE indicated by component descriptor) 132232206; SIMULCAST_GP_TAG (difference value of audio frame at the time of multicast and at the start of broadcast) 132232207; AUDIO_ATTR (audio attribute value) 132232208; LANG_CODE (first audio language code) 132232209; LANG_CODE 2 (second audio language code) 132232210; and CP_CTL_INFO (copy control information/copyright control information) 132232211.

The AUDIO_ATTR 132232208 comprises: Multi_Ing (1 = DUAL mono, 0 = others); Main_Comp (1 = main audio, 0 = others); Quality_Indicator (indicating sound quality display); and Sampling_Rate (011 = 24 kHz, 101 = 32 kHz, 111 = 48 kHz). This value is set by a value of an audio component descriptor.

FIG. 15 is a view illustrating an example of how SOB_OTHER_ESI included in each SOB ESI of FIG. 12 is configured.

The SOB_OTHER_ESI 1322323 comprises: ES_TY 13223231; ES_PID 13223232; STREAM_TYPE 13223233; COMPONENT_TAG 13223234; CP_CTL_INFO 13223237; and DAT_COMP_ID (data contents encoding identifier) 13223235 and AD_DAT_COMP_IFO (Additional data Component Information) 13223236.

FIG. 16 is a view illustrating another example of how copy control information (copyright protection information) CP_CTL_INFO 13223237 included in SOB_OTHER_ESI of FIG. 15 is configured. CP_CTKL_INFO 13223237 is present in SOBI_GI and SOB_V_ESI; SOB_A_ESI, and CPI of Packet Group Header. CPI of SOBI_GI makes whole copy control; CPI of ESI makes copy control of each ES; and copy control of each Packet Group is made by CPI of Packet Group Header. However, a higher priority is given to CPI of ESI than a CPI value of SOBI_GI, and further, the highest priority is given to CPI of Packet Group Header. These CPI values are set by a digital copy control descriptor and a content utilization descriptor or the like.

The contents are: CCI or CGMS (0 = copying disabled; 1 = copying is enabled unlimitedly); APS (0 = No APS, 1 = APS type 1 is added, 2 = APS type 2 is added, 3 = APS type 3 is added); EPN (0 = contents are protected (Internet output is protected), 1 = No contents protection) ; ICT (0 = Resolution is limited, 1 = No limit); Retention (1 = None, 0 = Temporary storage time is valid); and Retention_State (0 = No limit, 1 = 1 week, 2 = 2 days, 3 = 1 day, 4 = 12 hours, 5 = 6 hours, 6 = 3 hours, 7 = 1.5 hours). Among them, with respect to retention, when copying is disabled when Retention = 0, temporary storage is enabled by a time interval indicated by Retention_State. After that time interval has elapsed, it is required to erase such temporary stored data.

FIG. 17 is a view illustrating an example of how SOB_TMAPI 132235 included in SOBI of FIG. 9 is configured. The SOB_TMAPI 132235 comprises SOB_TMAPI_GI 1322351 and one or more ES_TMAPI #1 to #n 1322352. The SOB_TMAPI_GI 1322351 comprises: an LB address (logical address) SOB_ADR_OFS 13223511 from the start of a file to the start of SOB; SOBU_PB_TM_RNG (SUBU playback time range: 1 = 0.4 seconds to 1.2 seconds, 2 = 1 second to 2 seconds, 3 = 2 seconds to 3 seconds) 13223513; SOB_S_PKT_POS (starting in Packet group at the start of SOB: $1 \leq$ SOB_S_PKT_POS $\leq$ 85) 13223514; SOB_E_PKT_POS (End in Packet group at the start of SOB: $1 \leq$ SOB_E_PKT_POS $\leq$ 85) 13223515; ES_TMAP_Ns (the number of ES_TMAPs) 13223516 and the like.

Further, each ES_TMAPI 1322352 comprises: ES_PID (PID of target ES of this TMAP) 132235211; ES_S_ADR_OFS (Packet group number from the start of SOB file to the start of this ES (or LB address) 132235214; ES_E_ADR_OFS (Packet group number from the start of SOB file to the end of this ES (or LB address) 132235217; ES_S_PTM (start PTM) 132235212; ES_E_PTM (end PTM) 132235213; ES_SOBU_ENT_Ns (the number of SOBU_ENTs) 132235216; LAST_SOBU_E_PKT_POS (position in Packet Group of the last SOBU) 132235215;

STMAP_SPRN (TMAP numbers in STMAPT which belongs to this ES. However, in the case where STMAPT is recorded in another file for each STR_FI and in the case where each STMAPT is recorded in order, this number may not be required) 132235218; and the like. The STMAPT is recorded in another region (in another file or at the end of IFO), and comprises STMAPT 1; one or more STMAPI_SRPs; and STMAPIs whose number is equal thereto. The STMAPTI comprises: STMAPT end address information; version information on this TMAP; STMAP_SRP_Ns (the number of TAMP_SRPIs = the number of TMAPTs); and STMAP update date and time information (equal to a value of VMGI). The STMAP_SRP comprises STMAPI address information which is an element of each STMAPT, and each STMAPI comprises a required number of STMAPTI_GIs and SOBU_ENTs. The STMAPI_GI comprises SOBU_ENT_Ns (the number of ENTRYs). However, dummy data may be present between SOBU_ENTs.

SOBU/VOBU_PB_TM_RNG is properly set, thereby making it possible to prevent TMAPI information from being extremely increased even if a recording time increases. However, in that case, a possibility that double speed playback or the like cannot be performed smoothly increases because a time interval of each ENTRY increases.

In addition, with respect to the lower limit of the SOBU recording time, as shown in FIG. 18, when 0.4 seconds or less is not enabled, a time interval of A in the figure is shifted. Thus, there is a possibility that the start of SOBU cannot be aligned with that of GOP; an I (intra) picture cannot be played back; a video image cannot be played back by specific playback or the like; or a display video image is skipped. Therefore, for the sake of a case in which SOBU is cut out by time-out, if SOBU does not have I-picture, SOBU of 0.4 seconds (minimum time interval) is enabled. However, in this case, SOBU is limited to 1 frame or more and to units of frames, fields, or pictures. Further, when no I-picture is present in SOBU, 1ST_REF_SZ is set to 00 at the time of recording.

FIG. 19 is a view illustrating an example of how MVOB_TMAP_GI 138011 included in MVOB_TMAP 13801 of FIG. 5 is configured. MVOB_TMAP_GI 138011 comprises: the number of VR movie VOBU entries MVOBU_ENT_Ns 1380111; a time offset TM_OFS 1380112; an address offset ADR_OFS 1380113; a VOBU playback time range VOBU_PB_TM_RNG (value 1 indicates 0.4 seconds to 1.2 seconds; value 2 indicates 1 second to 2 seconds, and 3 indicates 2 seconds to 3 seconds) 1380114; and a VTMAP number VTMAP_N 1380115.

FIG. 20 is a view illustrating an example of how a time map file HR_TMAP.IFO (Example 1) included in the DVD_HDVR directory of FIG. 3 or a time map table TMAPT (Example 2) allocated at the end of HDVR_VMG of FIG. 5 is configured.

The TMAPT is recorded in another region (in another file (FIG. 3 or the like) or at the end of IFO (FIG. 4 or the like)).

FIG. 21 is a view illustrating an example of how a variety of information included in VTMAPT of FIG. 20 are configured. As shown in FIG. 21, the VTMAPT comprises: VTMAPTI; VTMAP_SRPT; and VTMAP #1 to #n. The VTMAPTI comprises: VMG_ID (value equal to VMG_ID present at the start of VMGI); VTMAPT_EA (VTMAPT end address); VERN (TMAP version information); IFO_LAST_MOD_TM (TMAPT update date and time information. A value equal to HR_MANGR. IFO); and VTMAP_SRP Ns (the total number of search information). The VTMAP_SRPT comprises one or more VTMAP_SRPs (search information on each VTMAP). Further, the VTMAP_SRP comprises: VTMAP_SA (VTMAP start address) and VOBU_ENT_Ns (the total' number of VOEU_ENTs), and the VTMAP comprises one or more VOBU_ENTs.

FIG. 22 is a view illustrating an example of how the contents of VOBU_ENT of FIG. 21 are configured. The VOBU_ENT has the same structure as normal VR (1STREF_SZ 13703311; VOBU_PB_TM 13703312; VOBU_SZ 13703313). In the next generation optical disk, the number of bits in each field increases with an increase of recording capacity.

FIG. 23 is a view illustrating an example of how a variety of information included in STMAPT of FIG. 20 are configured. In FIG. 23, the STMAPTI comprises: VMG_ID; STMAPT_EA (STMAP end address); VERN; STMAP_LAST_MOD_TM; and STMAP_SRP_Ns. The STMAP_SRP comprises: STMAP_SA (STMAP start address); ES_TMAPI_Ns (total number of ES_TMAPs); and ES_TMAPI_GI #1 to #q. The STMAP comprises one or more ES_TMAPs.

FIG. 24 is a view illustrating an example of what kind of information is stored in ES_TMAPI_GI and ES_TMAP included in STMAP_SRP and STMAP of FIG. 23. The ES_TMAP_GI comprises SOBU_ENT_Ns (the total number of SOBU_ENTs) and each ES_TMAP comprises one or more SOBU_ENTs #1 to #q. Though the TMAP_SRP does not always specify TMAP in ascending order, there is no problem since individual TMAP is pointed by SRP. Dummy data may be input between TMAP fields (for example, there is no problem even if invalid data is present between TMAP #1 and TMAP #3).

FIG. 25 is a view illustrating an example of how the contents of each SOBU_ENT of FIG. 24 are configured. SOBU_ENT comprises: 1st_Ref_PIC_SZ 13703301; SOBU_PB_TM (the number of fields) 13703302; SOBU_SZ (Packet GP number) 13703303; SOBU_S_PKT_POS 13703304; and PCR_POS 13703305.

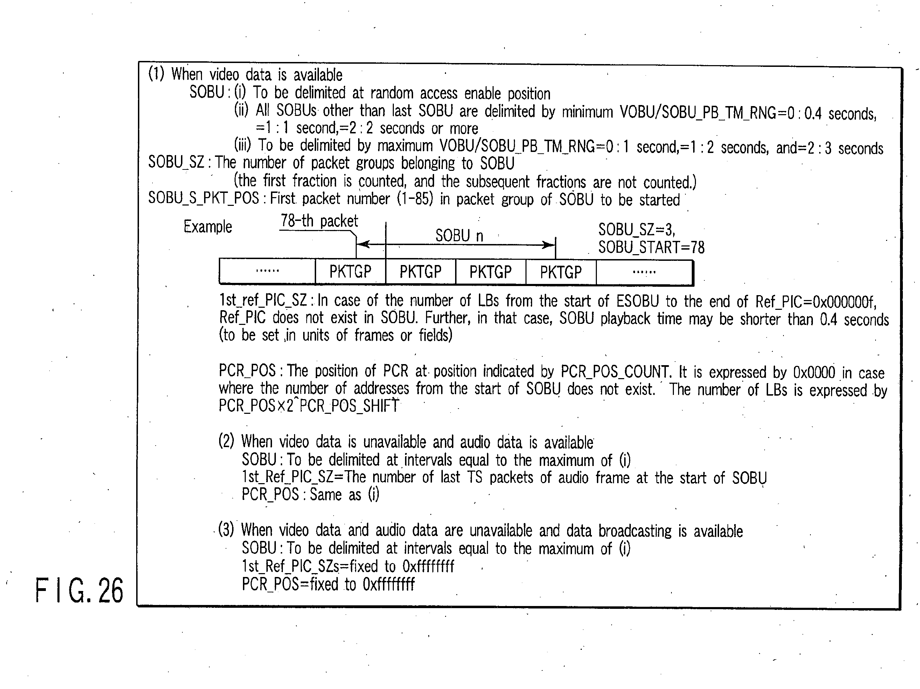

FIG. 26 is a view illustrating of what contents SOBU has in the presence or absence of video data and audio data. As shown in FIG. 26, three cases are assumed, i.e., (1) a case in which video data is available; (2) a case in which audio data is available and video data is' not available; and (3) a case in which only another item of information is available. Types (1), (2), and (3) are assumed. That is, in accordance with these types, SOBU entry information (SOBU_ENT) has three types as described above. The following description will be given with reference to FIG. 25.

(1) When video data is available, the SOBU entry information comprises: final address information from the start of SOBU of the first reference picture (such as I-picture) in entry (LB unit) 1st_Ref_PIC_SZ 13703301; SOBU playback time (the number of fields) SOBU_PB_TM 13703302; SOBU_SZ (size expressed by the number of packet groups and the number of packet groups belonging to SOBU) 13703303; SOBU_S_PKT_POS (the number of packets from the start of packet group in which the start of SOBU is included) 13703304; and PCR_POS 13703305.

In the PCR_POS 13703305, a position of PCR which is a position indicated by PCR_POS_COUNT is indicated by the number of addresses from the start of SOBU. When PCR does not exist, PCR_POS is 0xffff. In addition, the number of LBs of the PCR_POS 13703305 can be expressed by PCR_POS × 2PCR_POS_SHIFT. Here, PCR denotes a position of PCR several minutes before indicated by a PCR interval, the position preceding a position at which a reference picture is available.

In this manner, in the case of a time search, SOBU of a target time is obtained by accumulation of the SOBU_PB_TM 13703302, and a playback start PTM can be calculated by the number of fields from the start of that SOBU. Here, when SOBU targeted for time search is defined as K, and that target address is defined as $\underline{A}$, $\underline{A}$ can be obtained by the following equation (1).

$$A = \text{SOB\_ADR\_OFS} + \text{ES\_ADR\_OFS (of target ES)}$$
$$+ \sum_{N=1}^{k-1} \text{SOBU\_SZ (N)} \times 16 + 1 \qquad (1)$$

Further, the first packet is obtained as a packet whose value is SOBU_S_PKT_POS 13703304, and an access is provided to this address.

(2) When audio data is available and video data is not available, the SOBU entry information comprises: final address information from the start of SOBU of the first audio frame in entry (same as above); an SOBU playback time (the number of fields); SOBU size (same as above); and PCR_POS 13703305.

(3) When only another item of information is available, no entry information is configured, and thus, all data of the SOBU entry information is padded with FF.

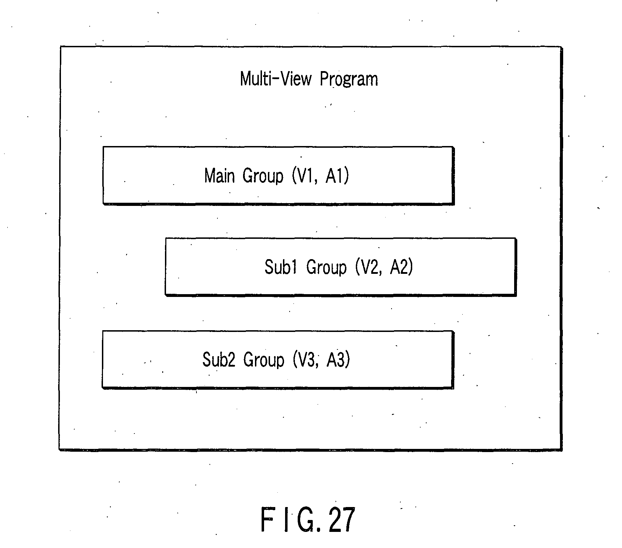

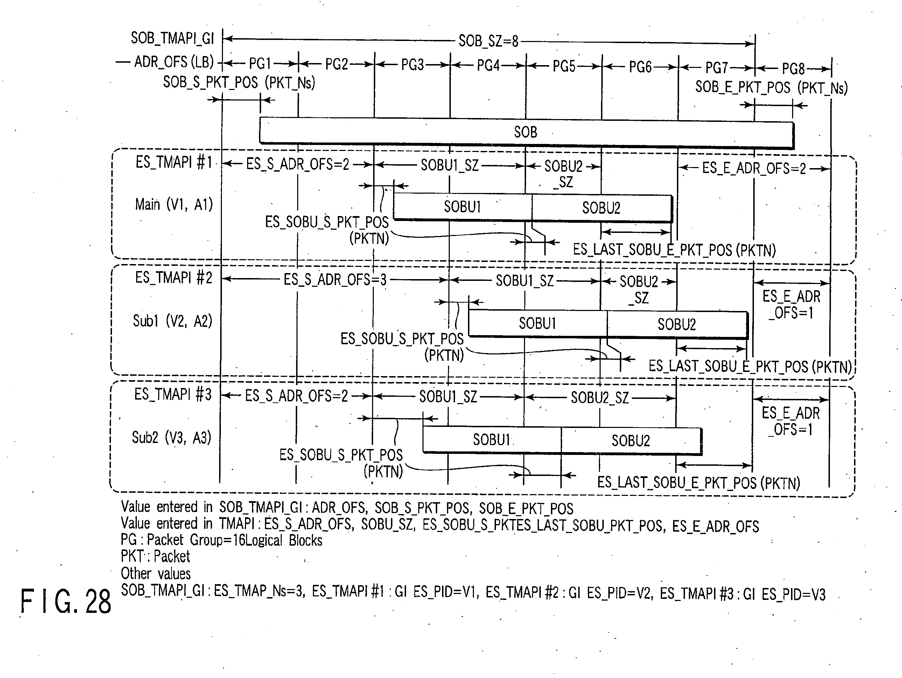

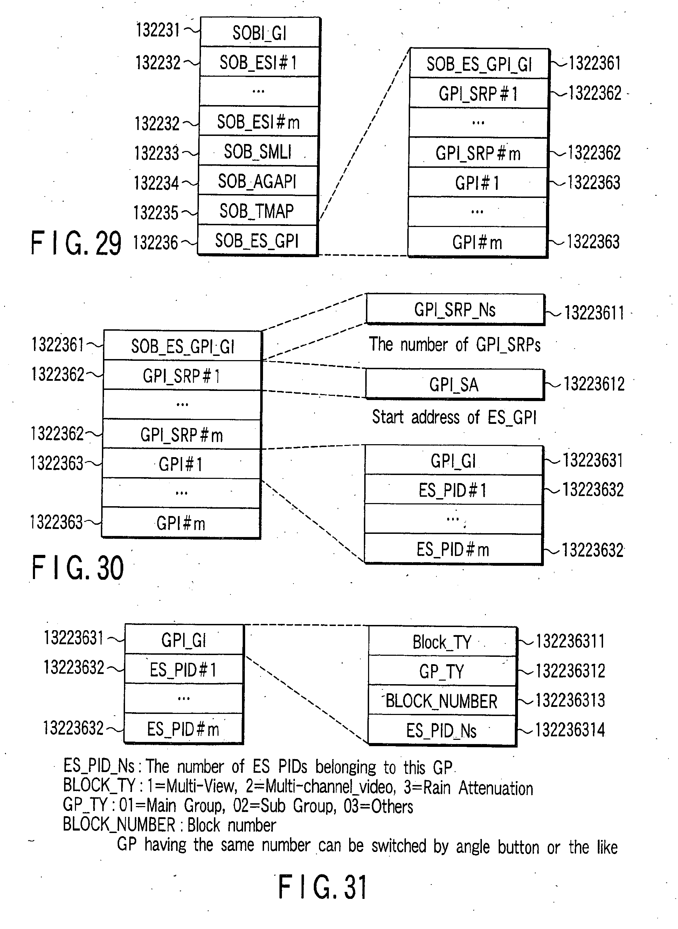

Now, a structural relationship between a data structure and actual SOBU is shown in FIGS. 27 and 28 (In the following description, Packet Group is abbreviated as PG). FIG. 27 is a view showing an image of SOB in the case where a multi-view broadcast is recorded, and FIG. 28 shows a detail of FIG. 27. In FIG. 28, at the upper stage SOB_TMAPI_GI (refer to FIG. 17), ADR_OFS and SOB_SZ, SOB_E_PKT_POS are entered as values relating to values of the whole SOBU. The other stages indicate the contents of TMAPI for each ES. In ES_TMAPI (refer to FIG. 17), ES_ADR_S_OFS (address (PG) from the start of SOB to the first SOBU of this ES); ES_ADR_E_OFS (address (PG) from the last SOBU of this ES to the end of SOB); ES_LAST_SOBU_E_PKT_POS (the number of packets up to the last Packet in Packet Group of the last SOBU); SOBU_ENTN (the total number of SOBU_ENTs); and default PID of this ES are entered as the value of the whole ES_TMAP. In SOBU_ENT (refer to FIG. 29) in each STMAPI, ES_SOBU_S_PKT_POS and SOBU_SZ are entered as a value belonging to SOBU.

Further, when SOB_SZ is present, ES_ADR_E_OFS can be obtained through calculation by the following equation (2), and thus, either of them may be present.

$$\text{ES\_ADR\_E\_OFS} = \text{SOB\_SZ}$$
$$- (\text{ES\_ADR\_S\_OFS} + \sum_{N=1}^{k-1} \text{SOBU\_SZ (N)} + 1) \quad (2)$$

SOB_SZ > ES_ADR_S_OFS, SOB_SZ > SOBU_SZ or the like is also established.

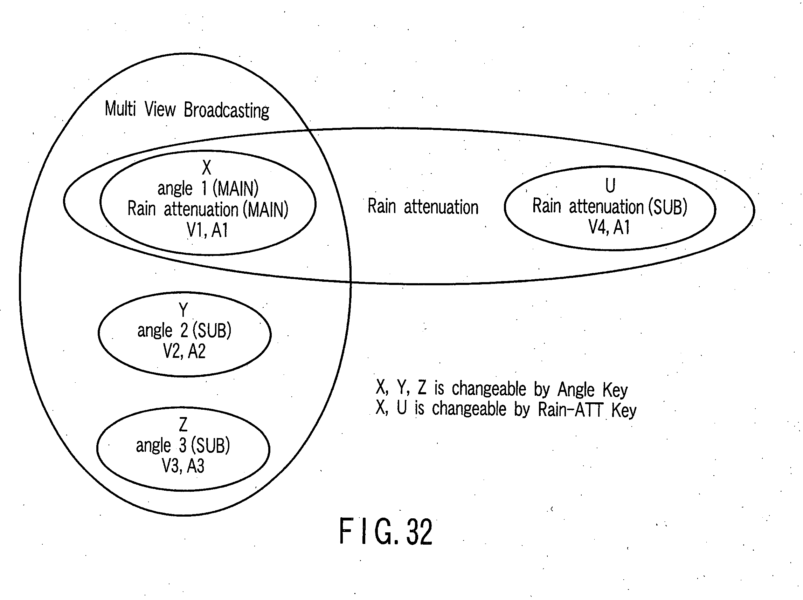

FIG. 29 is a view illustrating an example of how SOB_ES_GPI 132236 included in SOBI of FIG. 7 is configured (GPU structural example 1). FIG. 30 is a view illustrating an example of how SOB_ES_GPI_GI 1322361, GPI_SRP 1322362, and GPI 1322363 included in SOB_ES_GPI 132236 of FIG. 29 are configured (GPI structure of Example 1). FIG. 31 is a view illustrating an example of how CPI_GI 13223631 included in GPI 1322363 of FIG. 30 is configured (GPI structure of Example 1).

In SOB, a multi-view broadcast or a rainfall compatible broadcast, and SOB_ES_GPI (SOB_ES Group information) 1322361 compatible with simultaneous recording are provided, and two types of structures are assumed. First is a structure shown in FIGS. 29 to 31 in which plural types of information is provided to GPI, and control is made according to such type of information.

First, SQB_ES_GPI 132236 comprises: SOB_ES_GPI_GI 1322361; GPI_SRP #1 to #m 1322362; and GPI #1 to #m 1322363 (FIG. 29). GPI_SRP_Ns (the number of ES_GPI_SRPs) 13223611 is entered into the SOB_ES_GPI_GI 1322361 (FIG. 30). The GPI_SRP 1322362 comprises: GPI_SA (GPI start address) 13223621 and GPI_SZ (GPI size) (Alternatively, this can be substituted by PID_Ns indicating the number of PIDs, see FIG. 30). Each GPI 1322363 comprises: GPI_GI 13223631 and one or more ES_PIDs #1 to #n 13223632 (FIG. 30). The GPI_GI 13223631 comprises: ES_PID_Ns (the number of ESs of this group) 132236314; Block_TY (4 bits: 1 = multi-view broadcast, 2 = rainfall compatible broadcast, 3 = multi-channel recording) 132236311; GP_TY (4 bits: 0 = Main GP, 1 = SUB) 132236312; and BLOCK_NUMBER (Block number: this can be switched by the same block number) 132236313 (FIG. 31).

Here, it is found as to which type of group this group belong to by the Block_TY 132236311; as to whether or not switching is provided by an angle button; and as to whether or not switching is performed by a rainfall compatible button (if any) (When another program is recorded at the same time, this button cannot be freely switched). In addition, it is found as to which GP is switched with by the block number (BLOCK_NUMBER) 132236313. This is effective, for example, when two types of multi-view broadcasts are recorded. When one ES belongs to a plurality of GPs, for example, when an angle is switched by pressing a multi-angle key, and further, an image is distorted due to a rainfall and when a rainfall GP is switched by the rainfall button, the same ES is recorded in plurality for each GP, thereby making it possible to undergo troubleshooting.

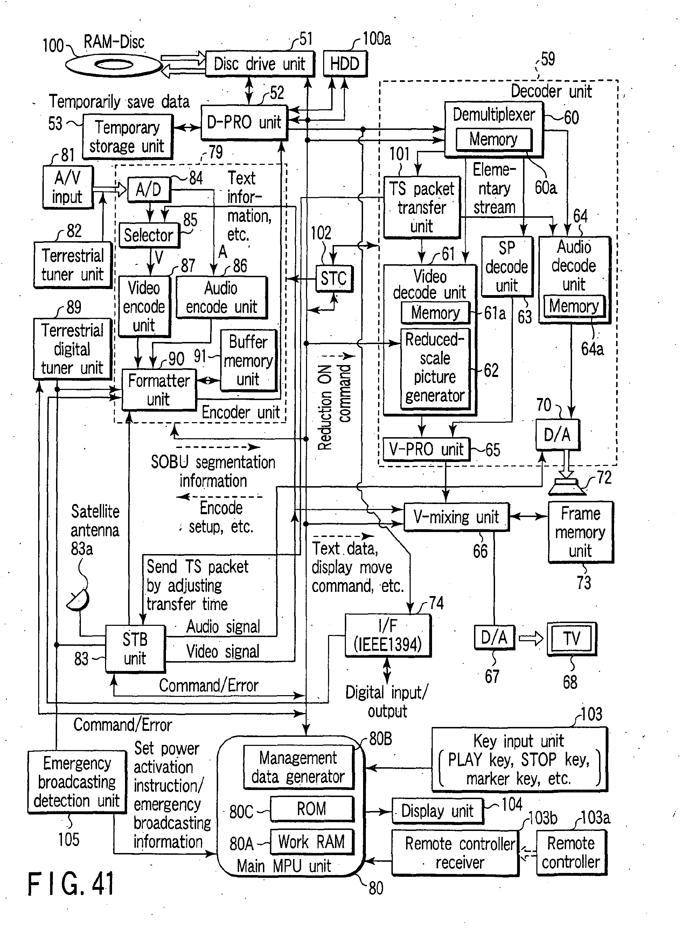

Now, a description will be given by way of specific example. FIG. 32 is a view illustrating an outline of how streams are configured when plural types of broadcasts are prepared. One feature of digital broadcasting includes, for example, multi-view broadcasting. In the multi-view broadcasting, a plurality of video images are prepared at the same time (by time-sharing), and among them, a user can selectively play back only a required video image. In this manner, a plurality of contents are selected according to the user's preference or the like. For example, as shown in FIG. 32, when X, Y, and Z streams in multi-angle broadcasting and a U stream in rainfall compatible broadcasting are received as one TS by a recorder (such as an apparatus of FIG. 41 described later), there is a need for selectively playing back a stream which is required during playback and there is a need for freely switching streams by a key such as a remote controller. In the embodiment of the present invention, the user selection of the plurality of contents (between streams) can be made by adding grouping information (GPI of FIG. 30).

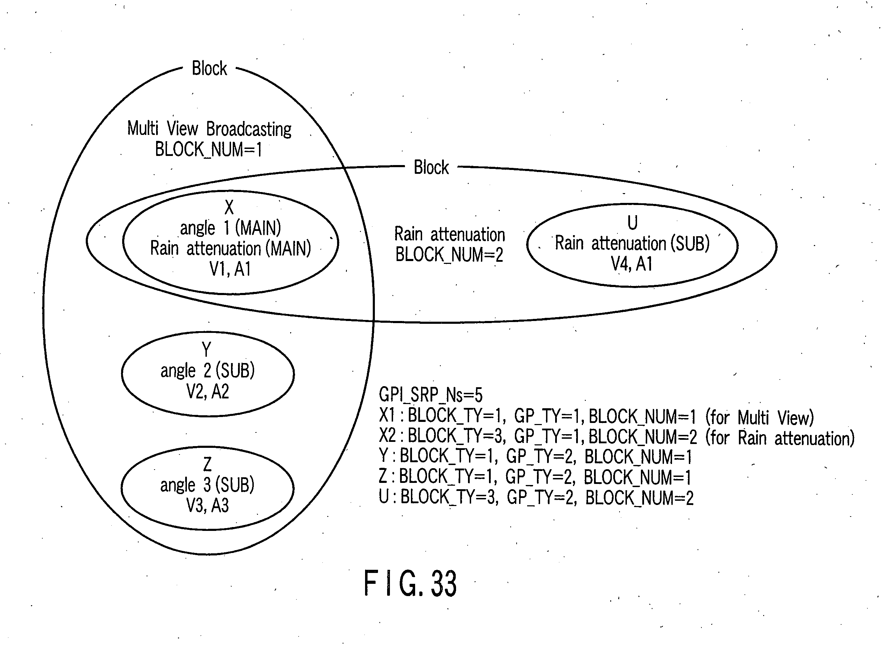

Now, let us consider an example of the above-described stream as shown in FIG. 32. That is, in the case of recording a broadcast such that X (MAIN), Y, and Z streams are provided in a multi-view broadcast and U is provided as a rainfall compatible stream, the method according to the embodiment of the present invention is used. Like FIG. 33 which is a view illustrating an example (Example 1) of a group configuration of streams when plural types of broadcasts are prepared, GPI_SRP_Ns is 5 because there are 5 groups consisting of X1 (for multi-views), X2 (rainfall compatible), Y, Z, and U; GPI of X1 is Block_TY = 1, GP_TY = 1, BLOCK_NUMBER = 1 because it relates to a multi-angle and a main group; GPI of X2 is Block_TY = 2, GP_TY = 1, BLOCK_NUMBER = 2 because it is compatible with a rainfall, and relates to a main group; GPI of Y is Block_TY = 1, GP_TY = 2, BLOCK_NUMBER = 1 because it relates to a subsidiary group in multi-view broadcasting; GPI of Z is Block_TY = 1, GP_TY = 2, BLOCK_NUMBER = 1 because it relates to a subsidiary group in multi-view broadcasting; and GPI of U is Block_TY = 1, GP_TY = 2, BLOCK_NUMBER = 2 because it relates to a subsidiary group compatible with a rainfall.

Therefore, during playback, it is determined whether or not Block_TY of the target type is included by referring to GPI in playback according to type of the pressed remote controller key (angle key in the case of multi-view broadcasting). When the determination result is affirmative, GP of the same Block_TY is searched by the same BLOCK_NUMBER and the same Block_TY assigned to that GP_TY. When the GP has been found out, switching to that GP is performed.

When a broadcast has only one type of GP_TY, a method for carrying out troubleshooting by entering only GP_TY and switching the same GP_TY without providing BLOCK_NUMBER is also used as a modified example of the method according to the embodiment of the invention.

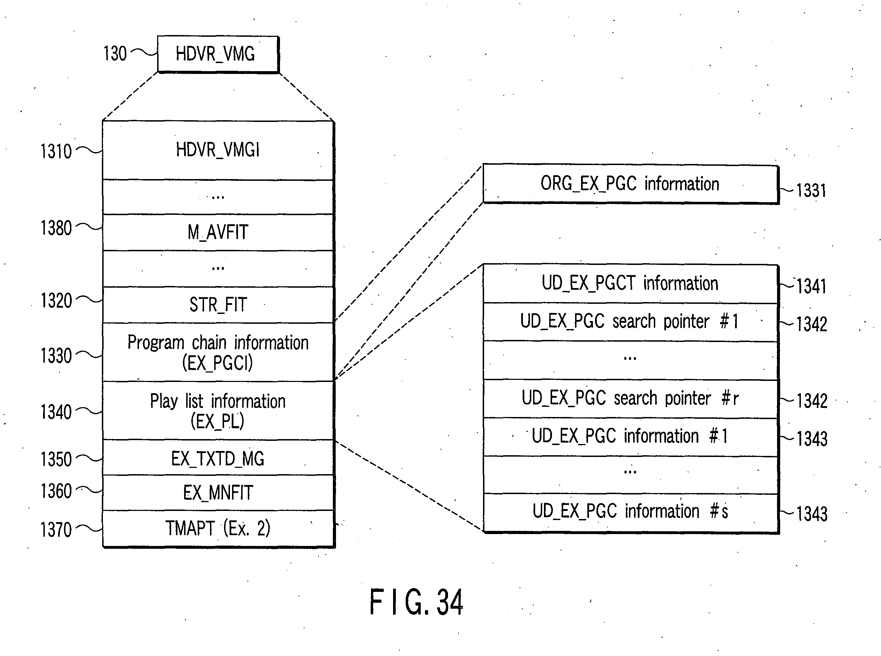

FIG. 34 is a view illustrating an example of how PGC information (ORG_EX_PGC information and EX_play list information/UD_EX_PGCT information) included in HDVR_VMG of FIG. 7 are configured. Original PGC information ORG_EX_PGCI 1331 is stored in the EX program chain information (PGCI) 1330. The EX play list information (or user defined information table information) 1340 comprises: user defined PGC table information UD_EX_PGCTI 1341; one or more UD_EX_PGC_SRP #1 to #r 1342; and one or more user defined PGC information UD_EX_PGCI #1 to #s 1343.

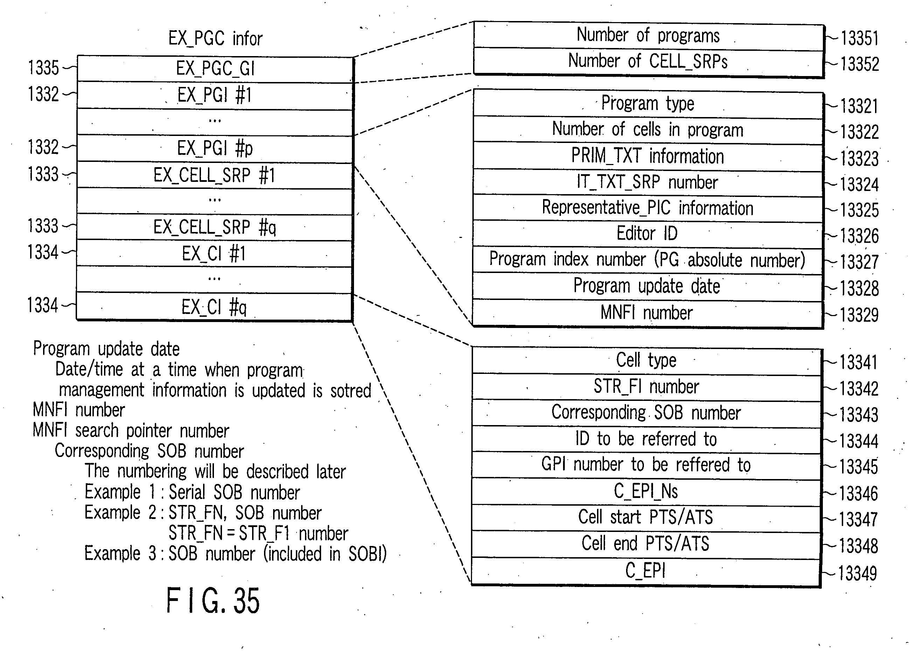

PGC information which is playback information has a format which is same as a normal VR format. The ORG_PGC information 1331 is automatically prepared by device (recorder) during recording, and is set in order of recording. The UD_PGCT information 1341 is prepared in accordance with the playback sequence which the user freely adds, and is called a play list. These two formats (original PGC information and play list) are common in PGC level, and its PGC format is shown in FIG. 35.

FIG. 35 is a view illustrating an example of how EX_PGC information of FIG. 34 is configured. The EX_PGC information'(original PGCI) 1331 comprises: its general information EX_PGC_GI 1335; one or more program information EX_PGI #1 to #p 1332; one or more cell search pointers EX_CELL_SRP #1 to #q 1333; and one or more cell information EX_CI #1 to #q 1334.

Here, the PG information (EX_PGI) 1332 stores date and time information 13328 obtained when this PG has been updated. In this manner, it is found as to what time this PG has been edited. In addition, as text information, PRIM_TXT 13323 is used for a program name. In order to store another text information, another item of information (director name, main cast name and the like') is stored in an IT_TXT area. A SRP number 13324 of the stored IT_TXT is set at this PGI, and is linked. Further, a PG number is set at IT_TXT data. The PG number used here denotes an absolute number after recorded in this disk, and is defined as an index number which is not changed even if another PG is deleted.

In addition, a SRP number of MNFI is set at PGI in order to utilize MNFI number 13329 provided to achieve a manufacturer specific function. Further, with respect to MNFI information as well, a link with data contained in the MNFI information is achieved by setting the PG number.

Further, PG update date and time information is set at both of MNFI and IT_TXT and coincidence between the times is checked during menu display, thereby making'it possible to verify whether or not editing by another manufacturer is made. Furthermore, in the CELL information (EX_CI) 1334, type of SOB is added to CELL type 13341, and SOB number, start time, end time, and GP number to be played back (or SUB-GP number to be played back) are specified. The start time and end time can be expressed in PTS units (playback time) or in ATS units (transfer time).

Now, when a time is specified as a playback time (real time during playback), an access method identical to that of conventional VR can be used, and the user can specify a desired access point during the playback time. Thus, the user's preference is fully reflected. However, this method can be specified in the case where the contents of streams can be sufficiently analyzed. When the contents are not sufficiently known, the transfer time unit must be unavoidably specified (When the playback time is specified, playback cannot be always started at the start of I-picture). When a playback start frame is not I-picture, decoding is started from I-picture which immediately precedes that frame. When decoding of up to a target frame has completed, displaying is started, and playback is started as if it were started from a specified frame from the standpoint of the user, thereby carrying out troubleshooting.

In addition, reference ID 13344 is determined by a method for setting PID (or value of component tag) of a typical stream of the streams to be played back and a method for setting ID of component group in a case of multi-view TV and the like. Further, a method for entering a reference GPI number (or SUB-GP number) 13345 and switching it (during playback) may be used. Also, a specific ID number is assigned to PG or CELL, and PG or CELL can be specified by numbers which are not changed even if they are deleted.

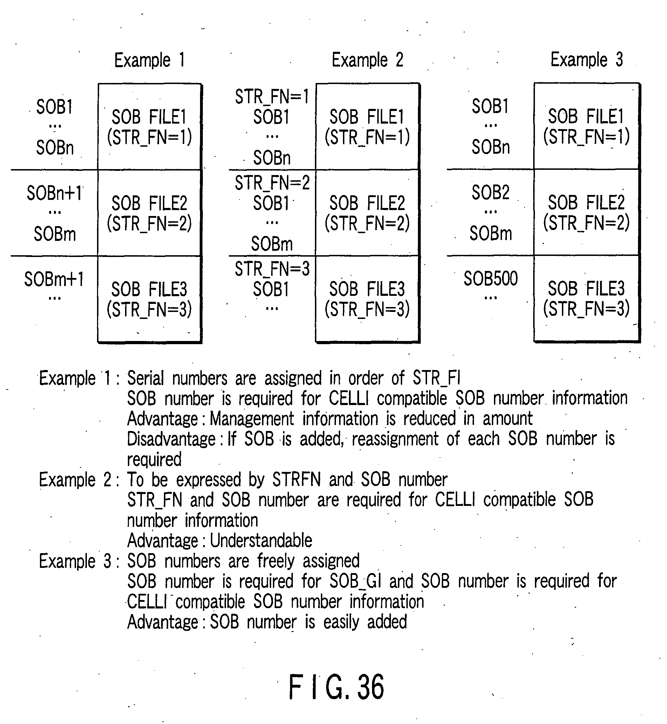

A SOB number to be played back (referenced) is set at CELL, and a plurality of methods for numbering SOB may be considered as shown in FIG. 36. First is a method for assigning and controlling serial numbers in order described in STR_FI; second is a method for expression with STR_F number and SOB number; and third is a method for freely assigning SOB number and setting the SOB number in SOB_GI.

In the first method, since a number is determined by default, the corresponding information contained in the SOBI is not required. However, SOB number must be reassigned every time SOB is added. In the case of the second method, since a number is determined by default, the corresponding information contained in the SOBI is not required. However, STR_F number as well as SOB number is required in CELLI. In the third method, SOB number is required for both of the SOBI and the CELLI, but the degree of freedom is the highest.

FIG. 37 is a view illustrating an example of how a data unit (SOBU) for a stream object shown in FIG. 1F or FIG. 2 is configured. One SOBU 134 comprises one or more packet groups 140. Each packet group 140 comprises, for example, 16 packs (1 pack = 1 sector: 2048 bytes).

Each packet group 140 comprises: a packet group header (128 bytes) 161; one or more packet arrival times PAT (4 bytes) 163 (170 PATS in this case); and MPEG-TS packets (188 bytes) 162 equal to PAT in number (170 packets in this case). A pair of PAT 163 and MPEG-TS packet 162 has the PAT 163 at the beginning thereof. The PAT 163 indicates a time when each MPEG-TS packet 162 has arrived at device.

The packet group header 161 comprises: a synchronizing pattern 151; display control information (DCI: Display Control Information) and copy generation control information (or copy control information CCI: Copy Control Information) 152; and manufacturer's information (MNI: Manufacturer's information) (or manufacturer information MNFI) 153.

In addition, each MPEG-TS packet 162 comprises: a 4-byte header 170 and an adaptation field and/or a payload 180. The header 170 comprises: a synchronizing byte 171; a transport error indicator 172; a payload unit start indicator 173; a transport priority 174; a packet identifier (PID) 175; a transport scramble control 176; an adaptation field control 177; and a continuity index 178.

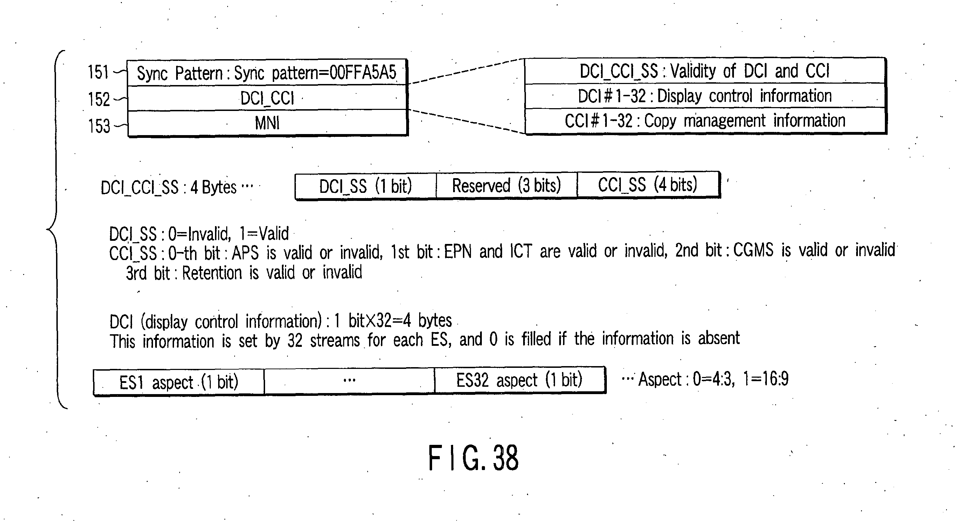

FIG. 38 is a view illustrating an example of how the DCI_CCI 152 included in the packet group header 161 shown in FIG. 37 is configured. Validity information (DCI_CCI_SS) comprises 1 byte. Among them, 1-bit DCI_SS indicates that the setting is invalid when it is 0, and indicates that the setting is valid when it is 1. 4-bit CCI_SS indicates whether APS is valid or invalid at bit 0; indicates whether EPN and ICT are valid or invalid at bit 1; indicates whether CGMS is valid or invalid at bit 2; and indicates whether Retention is valid or invalid at bit 3.

4 bytes are assigned to display control information (DCI), and DCI for 32 streams is set for each ES. When no stream available, the field of this DCI is padded with 0. In the breakdown of this DCI, an aspect flag of ES1 to ES32 is allocated (0 indicates an aspect ratio of 4:3, 1 indicates an aspect ratio of 16:9).

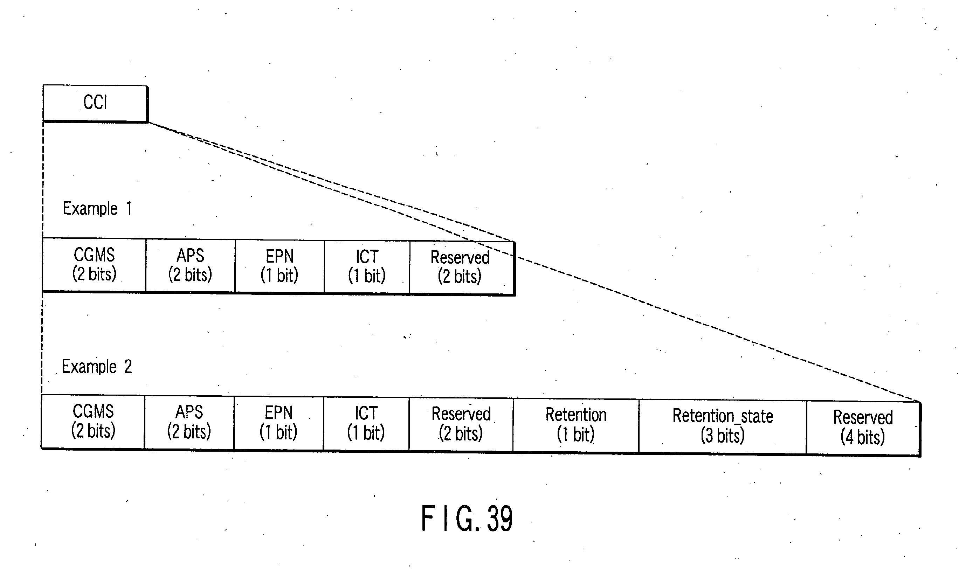

FIG. 39 is a view illustrating examples (Example 1 and Example 2) of how each copy control information CCI included in the DCI_CCI 152 of FIG. 38 is configured. The copy control information (CCI) has the same contents as ESI, and may include: digital copy control (00 = copying is inhibited, 01 = copy is permitted once, 11 = copying is permitted); analog copy control (00 = No APS, 01 = APS type 1, 10 = APS type 2, 11 = APS type 3); EPN (0 = contents are protected, 1 = contents are not protected); ICT (Image_Constraint_Token: 0 indicates an analog video output resolution constraint, 1 indicates no constraint); and retention information (temporary storage is enabled by a time interval indicated by "state" when Retention = 0 while copying is inhibited). Here, APS denotes an Analog Protection System, and assumes a macro-vision system (registered trademark) in such the embodiment.

When CCI or DCI is likely to change in the same ES and in the same packet group, the packet group is temporarily delimited, and the remaining packet groups are padded with dummy data (PAT = 0x01, TS packet = ALL 0x00) to set a next packet group. Namely, an alignment process is performed so that CCI or DCI does not change in a packet group (refer to FIG. 35).

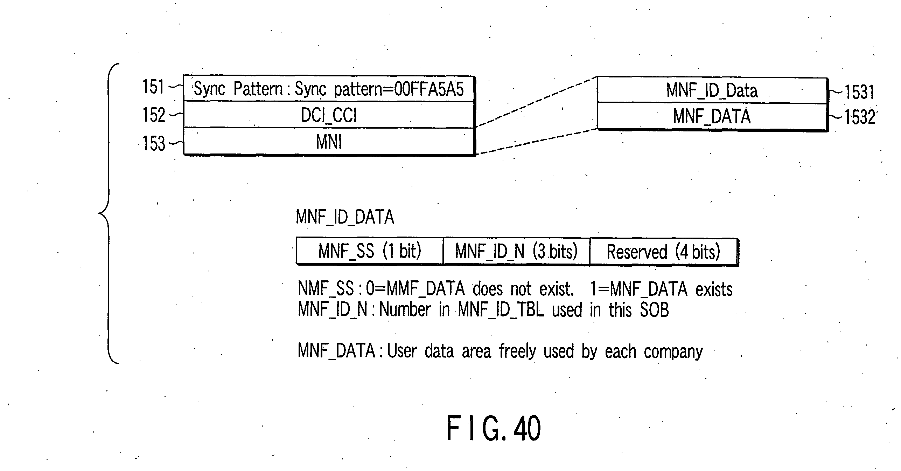

FIG. 40 is a view illustrating an example of how the MNI 153 included in the packet group header shown in FIG. 37 is configured. The MNI 153 comprises: MNF_ID_DATA 1531 and MNF_DATA 1532. The MNF_ID_DATA 1531 comprises: MNF_SS (1: MNF_DATA exists, 0: MNF_DATA does not exist); and MNF_ID_N. The MNF_ID_N is expressed with number (3 bits) indicating MNF_ID in MNF_ID_TBL (refer to FIG. 8) in STR_FI. The MNF_DATA is provided as a data region which can be freely set on a manufacturer by manufacturer basis.

In the meantime, recording device is believed to have a unique function which is not described in DVD specifications, depending on manufacturer or device type, and to make discrimination with other competitors. In that case, manufacturer unique information may be required to be padded in object data. Therefore, in the present embodiment, in order to cope with the above-described case, the region MNFI (Manufacturer's Information) 153 is provided in the Packet Group Header 161. However, a storage capacity is limited in the Packet Group Header 161 in object information. Thus, in order to enhance efficiency, an MNF_ID_TBL 13224 group is provided in the STR_FI 1322 (refer to FIG. 6). The SOBI 13223 specifies TBL which one uses from the group, and the Packet Group Header 161 specifies a number (MNF_IDN) indicating the target MNF_ID (ID of company having undergone recording operation) 132241 in that TBL, thereby enabling processing in the header having its limited capacity.

FIG. 41 is a block diagram showing an example of an apparatus which records/plays back audio/video (AV) information (digital TV broadcasting program, etc.) in an information recording medium (optical disk, hard disk, etc.) utilizing a data structure according to one embodiment of the present invention.

As shown in FIG. 41, the apparatus (digital video recorder/streamer) comprises a main MPU unit 80, key input unit 103, remote controller receiver 103b which receives user operation information from a remote controller 103a, display unit,104, decoder unit 59, encoder unit 79, system time counter (STC) unit 102, data processor (D-PRO) unit 52, temporary storage unit 53, disk drive unit 51 which records/plays back information with respect to a recordable optical disk 100 such as a DVD-RAM, hard disk drive (HDD) 100a, video mixing (V-mixing) unit 66, frame memory unit 73, D/A converter 67 for analog TV, analog TV tuner unit 82, terrestrial digital tuner unit 89, set top box (STB) unit 83 connected to a satellite antenna 83a, terrestrial tuner unit 82, and emergency broadcasting detection unit 108 which is connected to the STB unit 83 and which sets power activation instruction and emergency broadcasting information with respect to the MPU unit 80, on detecting the emergency broadcasting. Furthermore, since the apparatus corresponds to a digital input/output as a streamer, the apparatus comprises a digital I/F 74 such as IEEE 1394.

It is to be noted that the STC unit 102 is configured to count clocks on a 27 MHz base in accordance with the PAT_base of FIG. 37.

The STB unit 83 decodes received digital broadcasting data to generate an AV signal (digital), and sends the AV signal to a TV monitor 68 via the encoder unit 79, decoder unit 59, and D/A converter 67 in the streamer, so that the contents of the received digital broadcasting can be displayed. Alternatively, the STB unit 83 is also configured to be capable of sending the decoded AV signal (digital) directly to the V-mixing unit 66 and sending an analog AV signal to the TV monitor 68 from the V-mixing unit 66 via the D/A converter 67.

Additionally, since the apparatus of FIG. 23 constitutes a recorder comprising functions of both video recording and stream recording, the apparatus comprises components (IEEE 1394 I/F, etc.) unnecessary in the video recording or components (an A/D converter 84 for AV input, audio encoder unit 86, video encoder unit 87, etc.) unnecessary in the stream recording.

The encoder unit 79 comprises the A/D converter 84, video encoder unit 87, input switch selector 85 to the video encoder unit 87, audio encoder unit 86, sub-picture encoder unit (not shown, but if necessary), formatter unit 90, and buffer memory unit 91.

Moreover, the decoder unit 59 comprises a demultiplexer 60 which contains a memory 60a, video decode unit 61 which contains a memory 61a and a generator 62 of a reduced-scale picture (thumbnail, etc.), sub-picture (SP) decode unit 63, audio decode unit 64 which contains a memory 64a, TS packet transfer unit 101, video processor (V-PRO) unit 65, and D/A converter 70 for audio. An analog output (monaural, stereo, or AAC 5.1CH surround) from the D/A converter 70 is input into an AV amplifier and the like (not shown), and the necessary number of speakers 72 are driven.

Additionally, to display the contents being recorded in the TV monitor 68, stream data to be recorded is sent to the D-PRO unit 52, simultaneously sent also to the decoder unit 59, and can be reproduced. In this case, the MPU unit 80 sets the decoder unit 59 at the time of playback, and thereafter the decoder unit 59 automatically performs a playback process.

The D-PRO unit 52 forms an ECC group, for example, every 16 packs, attaches an ECC, and sends the data to the drive unit 51. Additionally, when the drive unit 51 is not prepared for the recording into the disk 100, the data is transferred to the temporary storage unit 53. The apparatus waits until preparations for recording the data are made. In a stage in which the preparations are made, the recording is started. Here, since the temporary storage unit 53 holds the recording data for several minutes or more at a high-speed access, a large-capacity memory is assumed. This temporary storage unit 53 can be configured utilizing a part of the HDD 100a.

It is to be noted that the MPU unit 80 is configured in such a manner as to be capable of reading/writing the data with respect to the D-PRO unit 52 through a microcomputer bus for exclusive use in order to read/write the data with respect to a management area of a file or the like.

In the apparatus of FIG. 23, first the optical disk 100, for example, DVD-RAM/-RW/-R/Blue media (recordable media using blue laser) or the like, is assumed as a recording medium, and the hard disk drive (HDD) 100a (and/or a large-capacity memory card (not shown), etc.) is assumed as an auxiliary storage device.

A way of using these plurality of mediums is, for example, as follows. That is, the stream recording is performed with respect to the HDD 100a utilizing the data structure (format) of FIGS. 1A to 40. Moreover, in the stream recording contents recorded in the HDD 100a, the program desired to be stored by the user is subjected to the stream recording (direct copying or digital dubbing) as such in the disk 100 (in a case where the copying is not prohibited by the copy control information CCI). In this case, only desired program having a quality equivalent to an original quality of the digital broadcasting can be recorded in the disk 100. Furthermore, since the data structure of the present embodiment is utilized in the stream recording contents copied to the disk 100, special playback such as time search is facilitated even in the stream recording.

A concrete example of a digital recorder (streamer/video recorder comprising a combination of DVD-RAM/ -RW/-R/Blue media with the HDD) having the above-described characteristics is the apparatus of FIG. 41. The digital recorder of FIG. 41 roughly comprises the tuner unit 82, 83, 89, disk unit 100, 100a, encoder unit 79, decoder unit 59, and control unit 80.

Satellite digital TV broadcasting is broadcasted from a broadcasting station through a communication satellite. The broadcasted digital data is received and reproduced by the STB unit 83. The STB unit 83 is a unit which extends and reproduces the scrambled data based on a key code supplied from the broadcasting station. At this time, the scrambling from the broadcasting station is descrambled. Here, the data is scrambled in order to prevent the broadcasted program from being illegally heard/seen by a user who does not contract reception with respect to the broadcasting station.

In the STB unit 83, the broadcasted digital data is received by a tuner system (not shown). When the received data is reproduced as such, the scrambling is descrambled by a digital extension unit, the received data is decoded by an MPEG decoder unit, the data is converted into a TV signal by a video encoder unit, and the TV signal is sent to the outside via the D/A converter 67. Accordingly, the digital broadcasting program received by the STB unit 83 can be displayed by the analog TV monitor 68.

Terrestrial digital broadcasting is received and processed in the same manner as in the satellite broadcasting except that the broadcasting does not go through the communication satellite (and the data is not be scrambled in free broadcasting). That is, the terrestrial digital broadcasting is received by the terrestrial digital tuner unit 89. When the broadcasting is reproduced as such, the decoded TV signal is sent to the outside via the D/A converter 67. Accordingly, the digital broadcasting program received by the terrestrial digital tuner unit 89 can be displayed by the analog TV monitor 68.

The terrestrial digital tuner unit 89 and the STB unit 83 supplies command/error information to a system bus (MPU unit 80).

The terrestrial analog broadcasting is received by the terrestrial tuner unit 82. When the broadcasting is reproduced as such, the received analog TV signal is sent to the outside. Accordingly, the analog broadcasting program received by the terrestrial tuner unit 82 can be displayed by the TV monitor 68.

An analog video signal analog-input from an external AV input 81 can be sent straight to the TV monitor 68, but may be once A/D converted by the A/D converter 84, thereafter returned to the analog video signal by the D/A converter 67, and sent to an external TV monitor 68. In this constitution, even when an analog VCR playback signal having many jitters is input from the external AV input 81, the (digital time base collected) analog video signal that does not have any jitter can be output on the side of the TV monitor 68.

The digital video signal digital-input from the digital I/F (IEEE 1394 interface) 74 is sent to the external TV monitor 68. Accordingly, the digital video signal input in the digital I/F 74 can be displayed in the TV monitor 68.

The satellite digital broadcasting, terrestrial digital broadcasting, or a bit stream (MPEG-TS) input from the digital I/F 74 can be stream-recorded as the stream object 132 of FIG. 1E in the stream object group recording area 131 (FIG. 1D) of the disk 100 (and/or the HDD 100a).

Moreover, the terrestrial analog broadcasting or the analog video signal from the AV input 81 can be video-recorded in the VR object group recording area 122 (FIG. 1D) of the disk 100 (and/or the HDD 100a).

It is to be noted that the apparatus can be configured in such a manner that the terrestrial analog broadcasting or the analog video signal from the AV input 81 is once A/D converted, and the stream recording is performed, not the video recording. Conversely, the apparatus may be configured in such a manner that the satellite digital broadcasting, the terrestrial digital broadcasting, or the bit stream (MPEG-TS) input from the digital I/F 74 is subjected to the video recording, not the stream recording (after necessary format conversion is performed).

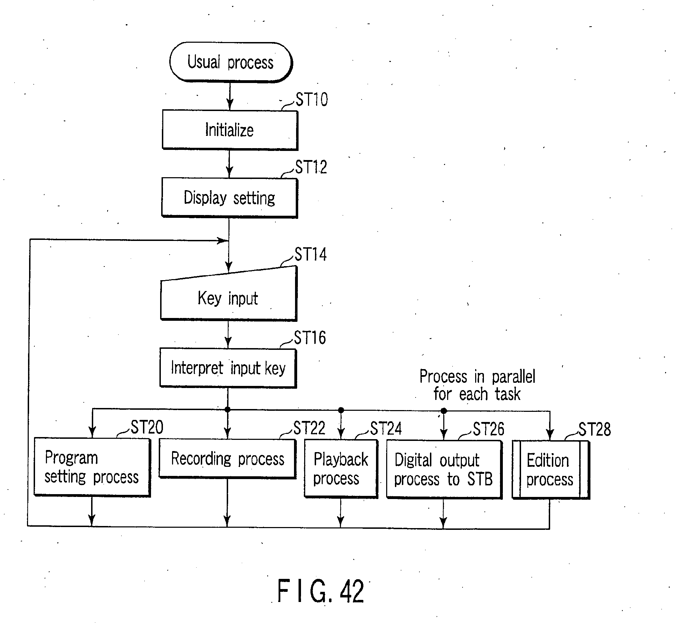

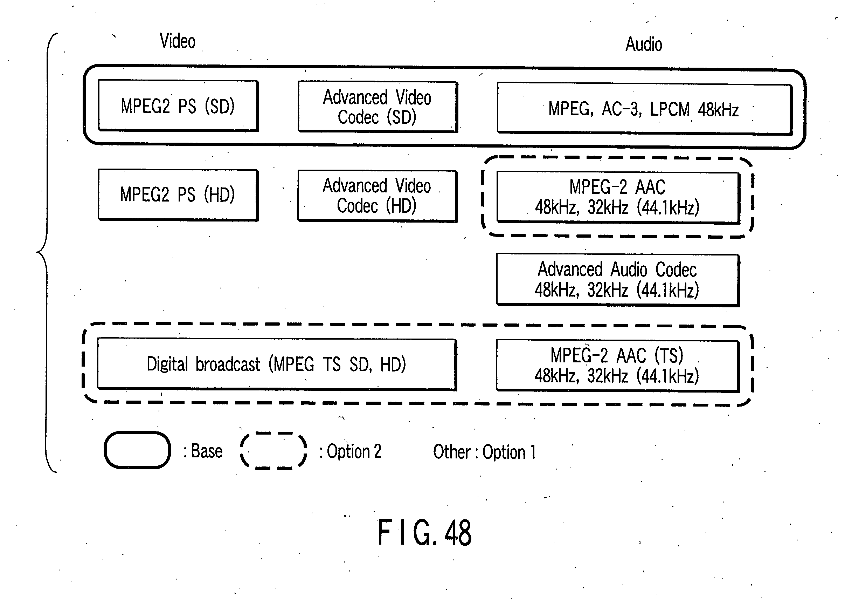

Recording/reproducing control of the stream recording or the video recording is performed by firmware (control program, etc. corresponding to an operation of FIGS. 42 to 69 described later) written in a ROM 80C of the MPU unit 80. The MPU unit 80 has a management data generator 80B of the stream recording and video recording, prepares various management information using a work RAM 80A as a work area, and appropriately records the prepared management information in the AV data management information recording area 130 of FIG. 1D. The MPU unit 80 reproduces the management information recorded in the AV data management information recording area 130, and performs various controls (FIGS. 41 to 69) based on the reproduced management information. It is to be noted that manufacture ID information or the like of the apparatus of FIG. 41 can be written beforehand in the ROM 80C of the MPU unit 80.

Characteristics of the medium 100 (100a) for use in the apparatus of FIG. 41 will be simply summarized as follows. That is, the medium comprises a management area 130 and a data area 131. The data is separated into a plurality of object data (stream objects (SOBs)) and recorded in the data area, and each object data comprises a set of data units (SOBUs). Moreover, one data unit (SOBU) comprises pack groups obtained by pack-grouping digital broadcasting signals for each TS packet, conforming to the MPEG-TS, by a plurality of packs (see FIGS. 1F and 37). On the other hand, the management area 130 comprises EX_PGC information (EX_PGCI) as information which manages the playback procedure, and the EX_PGC information (EX_PGCI) comprises cell information (CI). Furthermore, the management area 130 has information which manages the object data (stream object (SOB)).

The apparatus of FIG. 41 can perform the stream recording in addition to the video recording with respect to the medium 100 (100a) having the above-described data structure. In this case, to take the program map table PMT or the service information SI from the stream of the TS packet, the MPU unit 80 is configured in such a manner as to have a service information fetching section (not shown, firmware constituting a part of the management data generator 80B). The MPU unit 80 is also configured in such a manner as to have an attribute information generator (not shown, firmware constituting a part of the management data generator 80B) which prepares attribute information (PCR_LB number, etc.) based on the information taken by the service information fetching section.

In the apparatus of FIG. 41, the signals at the time of the recording flow, for example, as follows. The TS packet data received by the STB unit 83 (or the terrestrial digital tuner unit 89) is packet-grouped by the formatter unit 90, and stored into a work area (buffer memory unit 91). When a certain amount of data is stored (in a stage in which the data for one or integer times CDA is stored), the data is recorded in the disk 100. In the operation at this time, on received the TS packet, the packets are grouped every 170 packets, and a packet group header is prepared.

Moreover, the analog signal input via the terrestrial tuner unit 82 or a line input is digital-converted by the A/D converter 84. The digital signal is input into the respective encoder units 86 and 87. The video signal is input into the video encoder unit 87, the audio signal is input into the audio encoder unit 86, character data such as character broadcasting is input into an SP encoder unit (not shown), the video signal is MPEG-compressed, the audio signal is subjected to AC3 compression or MPEG audio compression, and the character data is subjected to run-length compression.

When the compressed data is formed into a block from each encoder unit, the block is formed in such a manner as to indicate 2,048 bytes, and the data is input into the formatter unit 90. In the formatter unit 90, each packet is formed into the block, further multiplexed, and sent to the D-PRO unit 52. The D-PRO unit 52 prepares an ECC block every 16 (or 32) blocks, attaches error correction data, and records the data in the disk'100 by the disk drive unit 51.

Here, in a case where the drive unit 51 is seeking the data or jumps to another track, the data is input into the HDD buffer unit 53 in a busy state, and the apparatus waits until the preparations of the RAM drive unit 51 are made. Furthermore, the drive unit 51 prepares each delimitation information during the recording, and periodically sends the information to the MPU unit 80 (GOP head interruption, etc.). As the delimitation information, there are the LB number of the VOBU (SOBU), end address of the I picture from the VOBU (SOBU) head, playback time of VOBU (SOBU) and the like.

Moreover, in the flow of the signal at the time of play back, the data is read from the disk 100 by the drive unit 51, errors are corrected by the D-PRO unit 52, and the data is input into the decode units 61, 63, and 64. The MPU unit 80 determines whether the type of the input data is VR data or SR data (determined by Cell TYPE), and sets the type to the decoder unit before playback. In the case of the SR data, the MPU unit 80 determines PMT_ID to be played back from cell information CI to be played back, determines the PID of each item (video, audio, etc.) to be played back from the corresponding PMT, and sets the PID to the decoder unit 59. The decoder unit 59 sends each TS packet by the demultiplexer 60 to each decoder unit 61, 63, and 64 based on the PID. Furthermore, the data is sent to the TS packet transfer unit 101, and transmitted to the STB unit 83 (1394 I/F 74) in the form of the TS packet in accordance with the arrival time. Each decoder unit 61, 63, and 64 performs the decoding, and the data is converted into the analog signal by the D/A converters 67 and 70, displayed in the TV monitor 68, and output from the speaker 72.

At the time of play back, pack data read from the disk 100 is'analyzed by the demultiplexer 60. When the pack includes TS packet, the pack is transmitted to the TS packet transfer unit 101 and further supplied to each decoder unit 61, 63, and 64 to be played back. When the pack is transmitted to the STB unit 83 (or transmitted to an external device such as a digital TV), the packet transfer unit 101 transmits only TS packets at the same time interval as reception time interval. The STB unit 83 decodes the packet to produce AV data which is displayed on the TV 68 through a video encoder unit in a streamer.

In the meantime, in a scheme for broadcasting (distributing) a compressed moving picture such as a broadcast using a wire such as a digital TV broadcast or Internet, an MPEG-TS scheme which is a common basic format is divided into a packet control data portion and a payload. In this payload, data targeted to be played back is included in a state in which the data is scrambled. According to ARIB, PAT (Program Association Table), PMT (Program Map Table), and SI (Service Information) are not scrambled. In addition, a variety of control information can be prepared by utilizing PMT or SI (SDT: Service Description Table, EIT: Event Information Table, BAT: Bouquet Association Table).

Playback objects include: MPEG video data; Dolby (registered trademark); AC3 audio data and MPEG audio data; data broadcast data and the like. Further, the playback objects further include information such as PAT, PMT, or SI (such as program information) required for playback. PID (Packet Identification) of PMT for each program is included in the PAT, and PID of video data or audio data is recorded in the PMT.

Normal playback procedures for the STB (Set Top Box) unit 83 are as follows. When the user determines a program based on EPG information, PAT is read at a start time of a target program. PID of PMT belonging to a desired program is determined based on that data, and a target PMT is read out in accordance with that PID. Then, PID of a video or audio packet to be played back, the packet included in the target PMT, is determined; a video or audio attribute is read out by PMT or SI, and the read-out attribute is set to each decoder.

The video or audio data is isolated and played back in accordance with PID. Here, PAT, PMT, SI and the like are transmitted every several hundreds milliseconds for use in intermediate playback.

FIG. 42 is a flow chart (whole operating process flow) illustrating an example of a whole operation in the apparatus of FIG. 41. The data processes used here, as shown in FIG. 42, includes 5 processes, i.e., a recording process (ST22), a playback process (ST24), a data transfer process (such as a digital output process to STB) (ST26), a program setting process (ST20), and an edition process (ST28).

For example, when the apparatus of FIG. 41 is powered ON, the MPU unit 80 performs initial setting (at the time of factory shipment or after the user setting) (ST10) and display setting (ST12); and waits for the user operation (key input). When the user makes a key input from the key input portion 103 (ST14), the MPU unit 80 interprets the contents of that key input (ST16). According to a result of this input key interpretation, the following four data processes are properly performed.

That is, when the key input is, for example, a key operation of a timer reservation recording setting, a program setting process is performed (ST20). When the key input is a key operation for starting recording, a recording process is performed (ST22). When the key input is a key operation for starting playback, a playback process is performed (ST24). When the key input is a key operation for digital STB output, a digital output process is performed (ST26). When the key input is a key operation for an edition process, an edition process is performed (ST28).

The processes at steps ST20, ST22, ST24, ST26 and ST28 are properly performed in parallel for such each task. For example, a process for digital output to STB (ST26) is performed in parallel during the playback process (ST24). Alternatively, the new program setting process (ST20) can be configured to be performed in parallel during the recording process (ST22) which is not timer reservation recording. Alternatively, by utilizing a feature of high speed accessible disk recording, a playback process (ST24) and a digital output process (ST26) can be configured so as to be performed in parallel during the recording process (ST22). A disk edition process (ST28) can be configured so as to be performed during HDD recording.

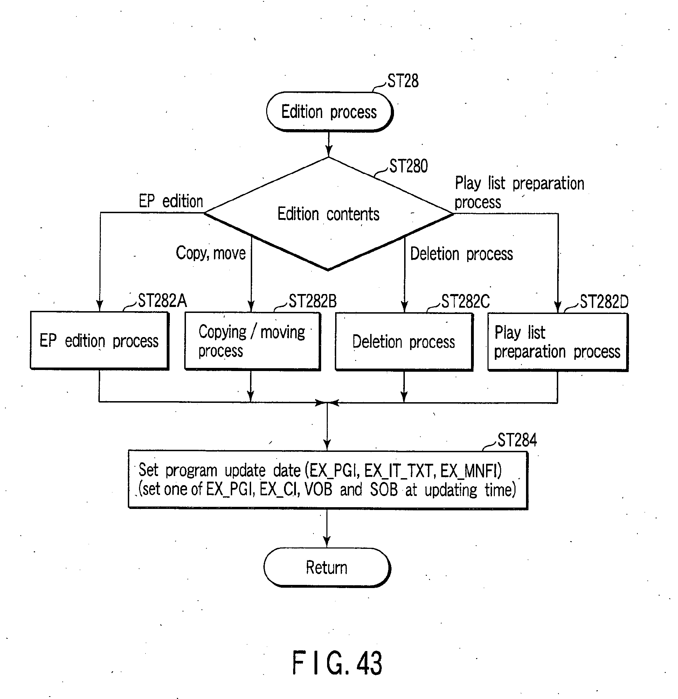

FIG. 43 is a flow chart (editing operation process flow) illustrating an example of the edition process (ST28) shown in FIG. 42. When the edition process is ready, any one of four processes (ST228A to ST228D) can be performed (ST280). When an entry point edition process (ST282A), a copying/moving process (ST282B), a deletion process (ST282C), or a play list preparation process (ST282D) is completed, the date and time of program update using this edition is set in each item of control information (EX_PGI, EX_IT_TXT, ET_MNFI) (ST284) .

When program information PCI, cell information CI, or either of VOB and SOB has been changed, this program update date and time (refer to FIG. 35) may be set.

Here, when VOBI and/or SOBI has(have) been changed, the VOBI and/or SOBI edit time (EDIT_TIME) can be set at SOB_EDIT_TIME or the like (not shown).

Moreover, in a process of ST284, manufacturer ID of device having made any of operations in ST282A to ST282D may be set at editor ID (LAST_MNF_ID) 13326 of FIG. 35. When any of PGI, CI, and SOB (or VOB) is changed, this editor ID can be set (or updated) based on ID information on device used at that time.

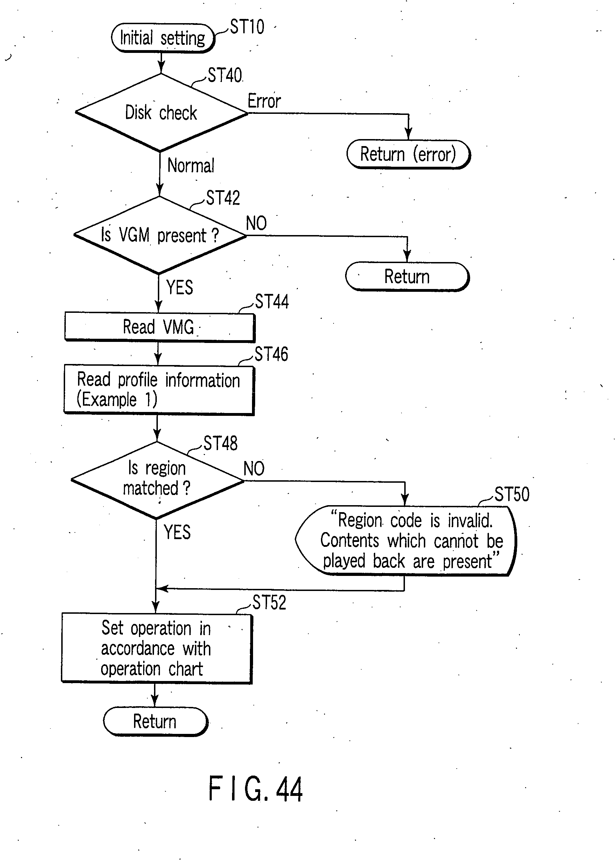

FIG. 44 is a flow chart (initial setting process flow example 1) illustrating an example of initial setting (ST10) shown in FIG. 42. This initial setting process is as follows.

A disk check is made (ST40), and when an error occurs, this check process is terminated.

When a disk is present (YES in ST42), and VMG has been recorded, VMG is read (ST44).

Profile information is read (ST46), and it is determined whether or not a region is matched (ST48). When the region is not matched (NO in ST48), setting is changed so as not to play back SOB and to display a warning message such as "The region code is not matched so that some contents may not be played back" (ST50).

When the region is matched (YES in ST48), option information is read, and setting is changed so as to operate in accordance with an operation chart shown in FIG. 45 (ST52).

FIG. 45 is a view showing an operation chart for specifying a combination between a basic format (Base) and one or more options (Option 1, Option 2) based on a relationship between a disk used and a playback operation (a relationship chart between an option and a playback operation).

For example, assuming that Base is SD_MPEG-2 PS + SD_new AVC PS, a long time signal can be recorded in a DVD-RAM disk in accordance with conventional DVD-VR (DVD video recording) specifications. When Option 1 is HD_new AVC PS + HD_MPEG-2 PS, data can be recorded in an AOD (Advanced optical Disc) disk with high quality which has not been achieved by conventional DVD-VR specifications. Further, assuming that Option 2 is compatible with digital broadcasting and is compatible with MPEG-2 TS, digital broadcast data can be recorded as a stream. In addition, what is to be introduced can be selected according to their respective equipment purposes.

In FIG. 45, Δ indicates that processing can be partially performed, and "?" indicates that a function of the corresponding option can be utilized. Because it is free as to what is specifically associated with "?" shown in plurality (unless otherwise specified in accordance with a standard or the like), although no limitation applies in particular, such an example is as described above.

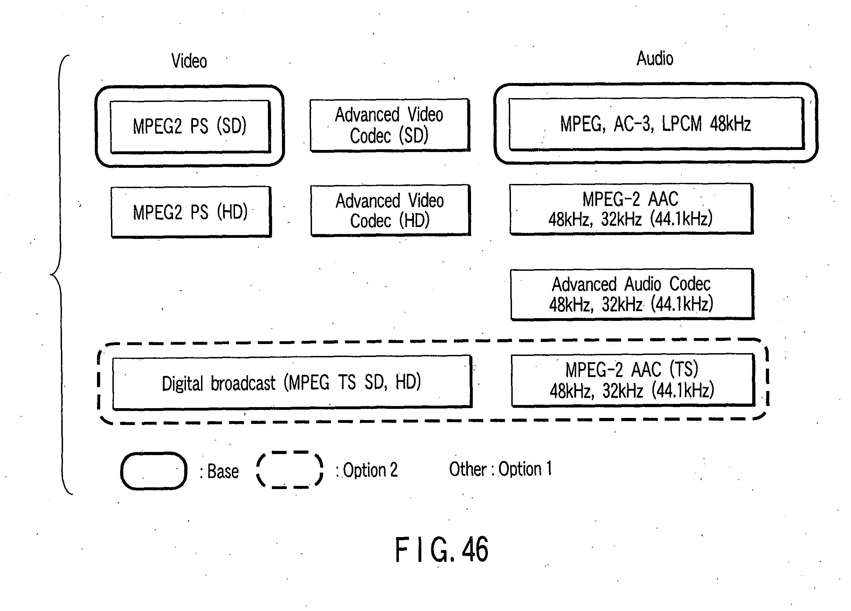

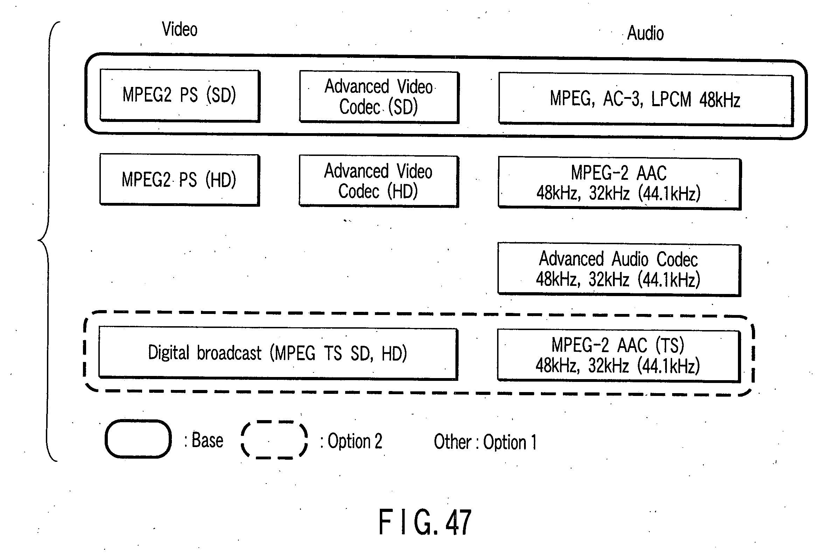

Here, methods for delimiting Base and Option include: a method for defining a portion supported in conventional VR specifications as Base, defining digital broadcast compatibility as Option 2, and defining the remaining HD self-recording and self-playback compatibility + new recording (sound) format as Option 1, as shown in FIG. 46; and a method for defining SD compatibility as Base, defining digital broadcast compatibility as Option 2, and defining the remaining HD auto self-recording and self-playback compatibility + new audio CODEC (such as AAC) as Option 1, as shown in FIG. 47; and a method for defining SD compatibility as Base, defining digital broadcast compatibility + new audio CODEC (such as AAC) as Option 2, and defining the remaining HD self-recording and self-playback compatibility as Option 1. Here, AAC or new CODEC compatible with MEPG-2 PS is believed to be required for converting digital broadcasting into PS. In the third case, these AAC or new CODEC is added only when digital broadcasting is supported.

Further, as shown in FIG. 7, the contents to be supported by Options are believed to be different from each other in accordance with a digital broadcasting scheme to be recorded, the scheme indicated by a value of AP_FORMAT_1. For example, in Japanese ARIB, an AAC scheme is used as a standard audio format. In the U.S. ATSC, an MPEG-audio format is provided as standard. Therefore, the formats to be supported are different from each other, and the formats to be supported by each option are changed in association with this digital broadcasting to be recorded.

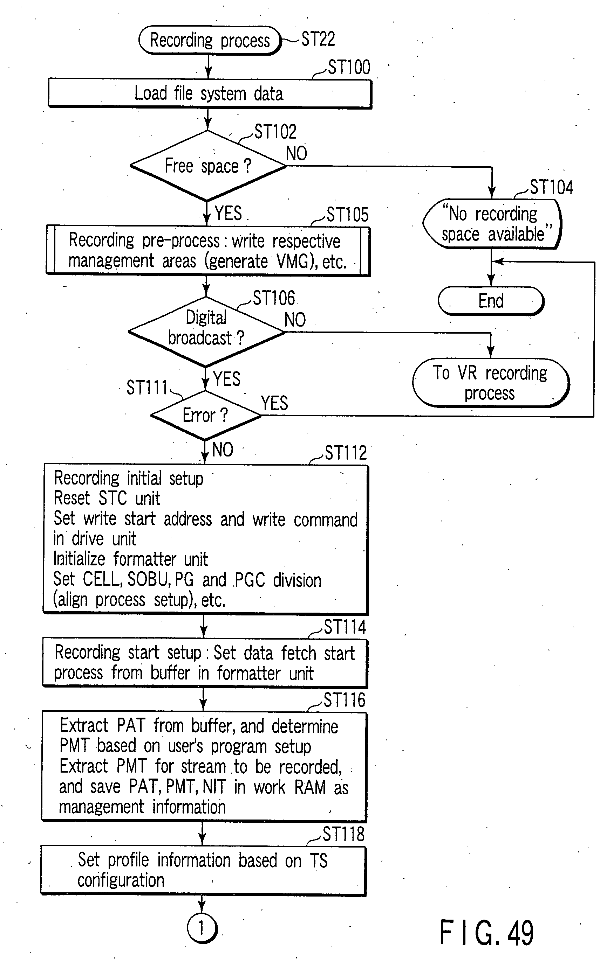

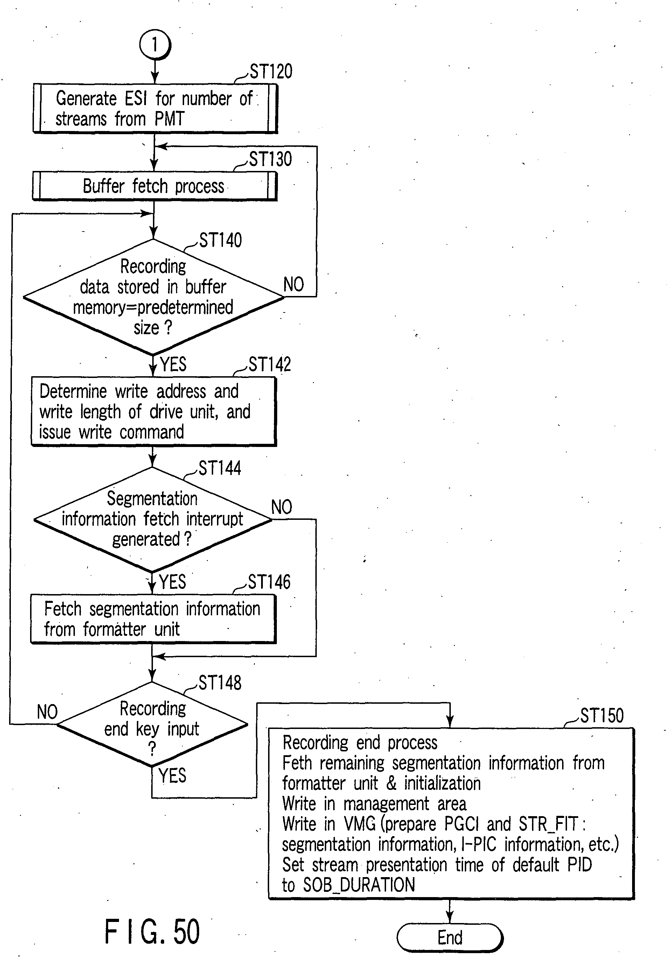

FIGS. 49 and 50 are flow charts illustrating an example of a recording operation in the apparatus of FIG. 41. Data processing during stream recording is as follows (refer to FIGS. 49 to 63).

First, a program to be recorded is determined by using an EPG (Electronic Program Guide) in a program setting process, receiving is started, and recording of the determined program is performed.

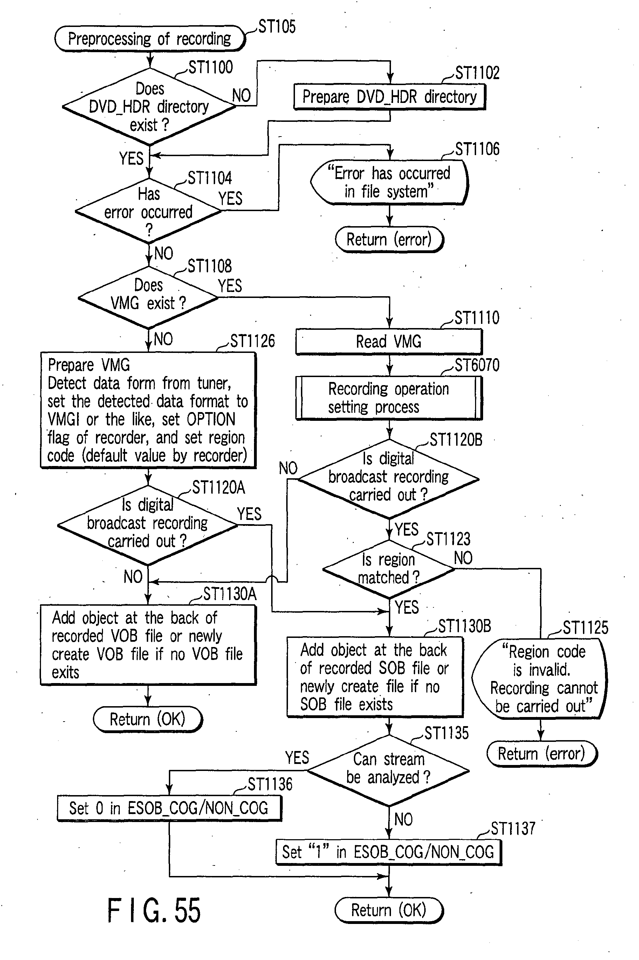

When the MPU unit 80 receives a recording command from the key input unit 103, the MPU unit 80 determines a region for reading and writing control data from the drive unit 51. At this time, the file system is checked (ST100), and it is determined whether or not a remaining capacity is enough to record an object (ST102). When recording is enabled, a preprocessing of recording such as preparation of VMG or setting of management regions is performed (ST105: Refer to FIG. 55). When recording is not enabled, the fact is indicated to the user, e.g., by displaying a warning message "no recording space remains" (ST104), and processing is canceled.

When a recording target is a digital broadcast content (ST106) and when no error occurs (No in ST111), the determined region is set in a management region so that the region is written. Then, preparation for data recording is performed. Particularly, the STC unit 102 is reset, a video data write start address and a write instruction are set to the drive unit 51, the formatter is initialized, and a delimitation of CELL, SOBU, PG, Packet Group is initially set (ST112).

In ST112, time resetting is performed at the STC unit 102. The STC unit 102 carries out recording and playback using the system timer based on the reset value as a reference.

Then, PAT of a program to be recorded is read; PID for capturing PMT of the target program is determined; the target PMT is read; and PID of each item of data (video, audio) to be decoded (recorded) is determined. At this time, PAT and PMT are stored in the work RAM unit 80A of the MPU unit 80, and the stored PAT and PMT are written into control information. Into the file system, VMG file data is written, and information required for VMGI is written.

Recording setting is performed for each section (ST114). At this time, the setting of delimitation of each item of data at the formatter unit 90 or the setting of receiving a TS packet is provided. At this time, PID of data to be recorded is set so as to record only the target video stream

In addition, setting is provided to the buffer 53 so as to start retention of the TS packet (ST116).

In ST118, profile information is set in accordance with a configuration of TS.

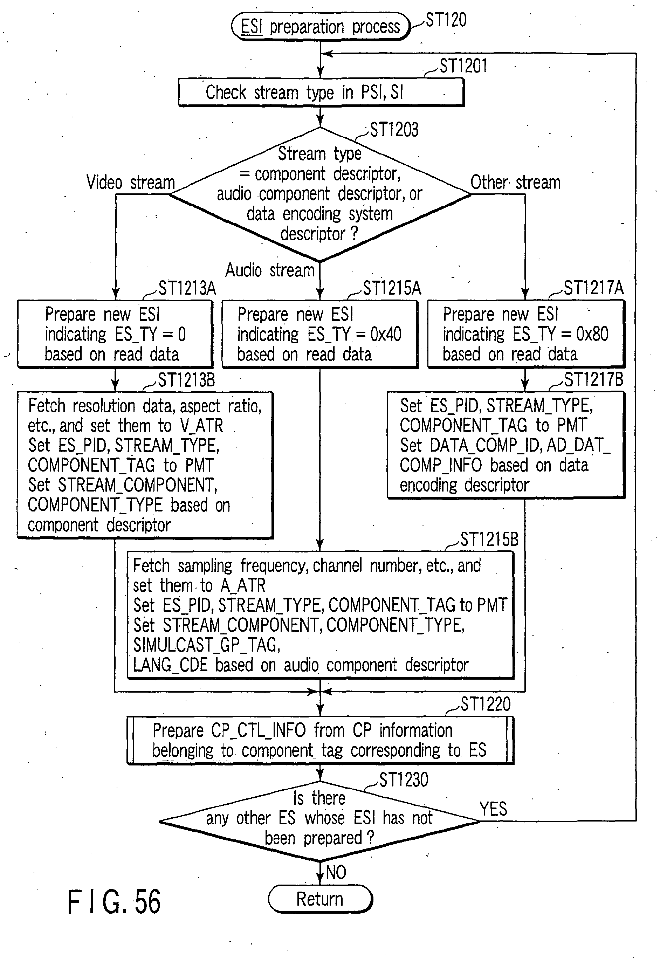

SOB_ESIs are prepared from PMT by the number of streams (ST120: Refer to FIG. 56).

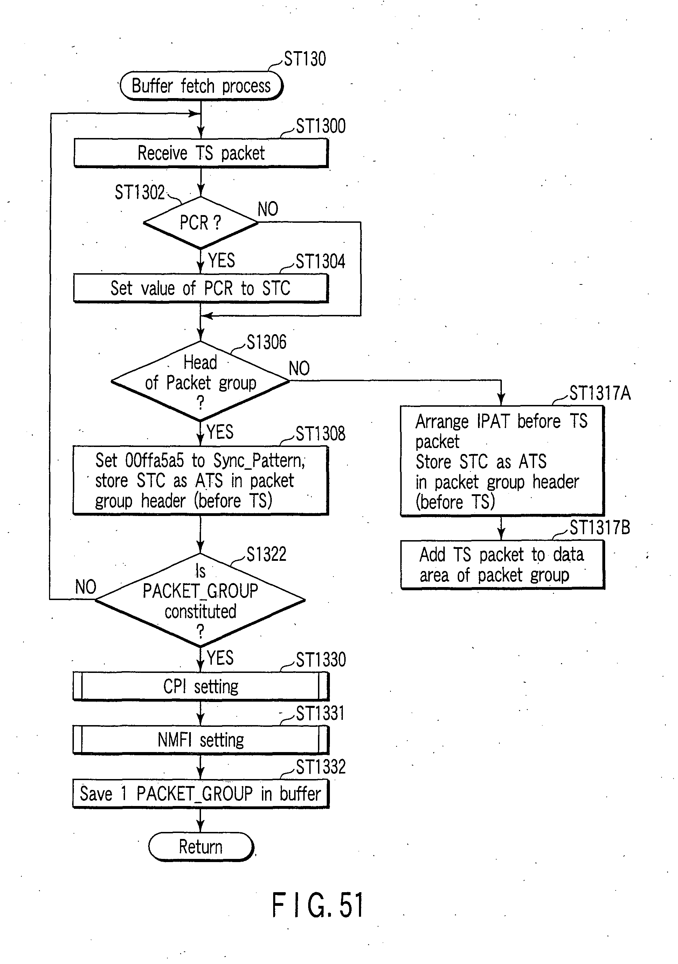

Then, the formatter unit 90 starts a buffer fetch operation (ST130), as shown in FIG. 51.

When a predetermined amount of data is accumulated in the buffer 53, an ECC process is performed through the D-PRO unit 52, and the processed data is recorded in the disk 100 (ST130).

During recording, delimitation information is periodically stored in the work RAM 80A of the MPU unit 80 (before the buffer RAM 91 of the formatter unit 90 becomes full) (ST144 and ST146). The delimitation information used here denotes SOBU delimitation information, and includes an SOBU start address, an SOBU pack length, an I-picture end address, and an SOBU arrival time (ATS).

In ST148, it is determined whether or not recording is terminated (whether or not the recording end key has input or whether or not the remaining free space becomes full). At the time of termination, in ST150, the remaining delimitation information is read from the formatter unit 90, and the read information is added to the work RAM 80A. Then, these items of data are recorded in control data (VMGI), and further, the remaining information is recorded in the file system.

When recording is not terminated, processing returns to ST140 in which data fetch and playback are continuously performed.

FIG. 51 is a flow chart (buffer fetch process flow) illustrating an example of the buffer fetch process (ST130) shown in FIG. 50. In the flow of a signal during recording, TS packet data received at the STB unit 83 (or terrestrial digital tuner 89) is stored in the work RAM 80A after packet-grouped at the formatter unit 90. At a time point when a predetermined amount of the packet data is accumulated (at a stage when the data is accumulated by one CDA or integer multiples thereof), the data is recorded in the disk 100. At this time, operation is performed as follows. When a TS packet is received (ST1300), grouping is performed on a 170 packets by packets basis, and a packet group header is prepared.

When PCR is present in the received TS packet, modification of STC is performed by setting the value of PCR to STC (ST1302 and ST1304).

Sync_Pattern: 00ffa5a5 is set at the start of a packet group (ST1308).

When a TS packet arrival time is defined as PAT, and the PAT is allocated in the TS packet group header (ST1308). The received TS packet is set in the TS packet data area.

It is determined whether or not packet grouping is terminated (it is determined whether or not 170 TS packets are grouped) (ST1322). When packet grouping is not terminated, processing returns to step ST1300. When packet grouping is terminated, a CPI setting process is performed (ST1330). Then, an MNFI setting process is further performed (ST1331), and group data is temporarily stored in the buffer RAM (ST1332).

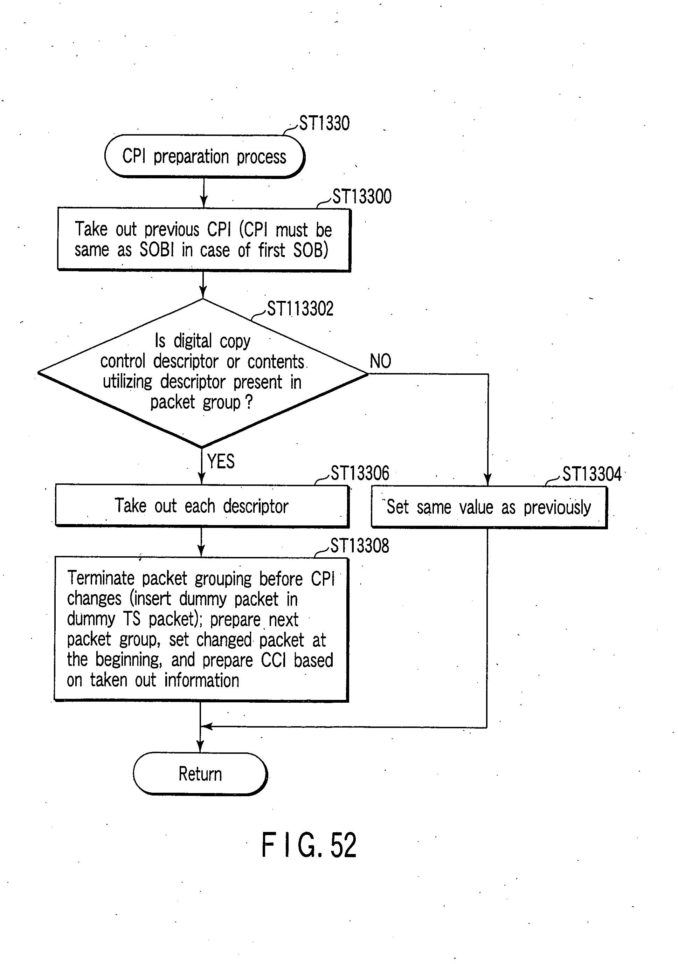

FIG. 52 is a flow chart (CPI preparation process flow) illustrating an example of the CPI setting process (ST1330) shown in FIG. 51. The CPI setting process will be described below.

Previous CPI is read out (ST13300). The previous CPI is the same as SOBI if the CPI is the header of SOB.

It is determined whether or not copy information (digital copy control descriptor and contents utilization descriptor) is present in the latest packet group (PMT and EIT) (ST13302). When the copy information is present, the copy information is 'read out and new copy information is generated and set based on that information (ST13306). Then, processing goes to step ST13308.

When no copy information is present in the received TS packet, information identical to the previous pack is generated as copy information (ST13304).

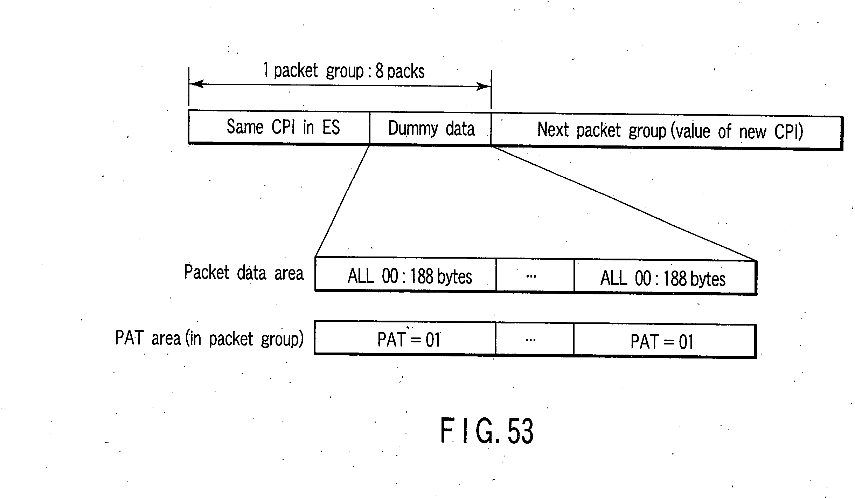

When a change occurs in the middle of a packet group, the packet group is terminated and dummy data (referring to FIG. 53) is inserted into the previous packet group so as to be handled as a new packet group from the changed place. CCI is set based on the read out copy information (ST13308).

When no copy information is present in the received TS packet, CCI is set to be copy free.

FIG. 53 is a view showing a data structure of a packet group in a process (packet group aligning process) at step ST13308 of FIG. 52. In the same ES, when CPI (CCI and/or DCI) is likely to change in the same packet group, the packet group is temporarily delimited. Then, as shown in FIG. 53, the remaining pack is padded with dummy data, and is set so as to be a next packet group. Namely, an aligning process is performed so that CCI or DCI does not change in the packet group.

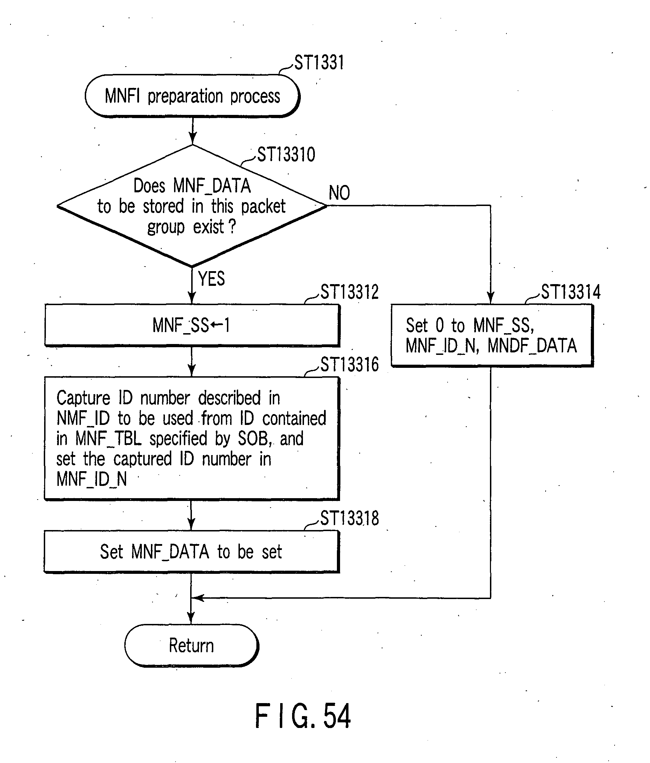

FIG. 54 is a flow chart (MMFI preparation process flow) illustrating an example of the MNFI setting process (ST1331) shown in FIG. 51. The MNFI preparation process will be described with reference to FIG. 54.

At.step ST13310, it is determined whether or not MNF_DATA to be stored exists in this packet group. When it does not exist, processing goes to step ST13314 in which 0 is set in MNF_SS, MNF_ID_N, and MNF_DATA, and this process is terminated (refer to FIG. 40 as well).

When it does exist, processing goes to step ST13312 in which 1 is set in MNF_SS, and then, processing goes to step ST13316 in which an ID number to be used is selected from the table MNF_ID_TBL, and the number is set in MNF_ID_N.

Then, processing goes to step ST13318 in which data to be recorded in MNF_DATA is set, and this process is terminated.

FIG. 55 is a flow chart (recording preprocessing) illustrating an example of preprocessing before starting recording in the disk-shaped information storage medium (for example, optical disk using blue laser) shown in FIG. 1. Recording preprocessing is performed as follows.

A search is made for a DVD_HDR directory (a directory recording new VR) (ST1100). When no DVD_HDR directory exists, a new directory DVD_HDR is prepared (ST1102). When the directory exists, processing goes to the next step.

It is determined whether or not an error is occurred (ST1104). When the error is occurred, a warning message "Error has been occurred in the file system" is displayed (ST1106) and the process is terminated.

When the error is not occurred, it is determined whether or not VMG exists (ST1108). When the VMG exists, VMGI which is its control information is read in the work memory (ST1110), and profile information (FIGS. 8 and 10 and the like) is read. Then, a recording operation setting process is performed (ST6070). Then, processing goes to step ST1120B in which it is determined whether or not contents to be recorded from now on are digital broadcasting. When they are not digital contents, processing goes to step ST1130A.

In the case of digital broadcast recording (YES at step ST1120B), a region is checked (ST1123). When the checked region does not match a region value set as a default in this apparatus (the apparatus of FIG. 41), a warning message "Region code is invalid. Recording cannot be carried out." is displayed (ST1125) and the operation is terminated. When the checked region matches the above region value, processing goes to step ST1130B.

When no VMG is recorded (NO at step ST1108), processing goes to step ST1126. VMG is prepared, and the default value of this apparatus is recorded in profile information (FIGS. 8 and 10 and the like) (ST1126).

It is determined whether or not contents to be recorded from now on are digital broadcasting contents (ST1120A). When they are not digital contents, processing goes to step ST1130A.

When an SOB file is present, the object file is set so as to be recorded following the SOB. When an SOB file is not present, a new SOB is created and the object file is set so as to be recorded following the new SOB (ST1130B).

It is determined whether or not a stream can be analyzed (ST1135). When the stream can be analyzed, "cognizant" is set (0 is set in ESOB_COG/NON_COG) (ST1136). When the stream cannot be analyzed, "non-cognizant" is set (1 is set in ESOB_COG/NON_COG) (ST1137), and this process is terminated.

FIG. 56 is a flowchart (ESI preparation process flow) showing one example of contents of the stream information (ESI) preparation process (ST120) shown in FIG. 50.

First, a stream type in PSI or SI is checked (ST1201). In the stream recording to record the MPEG-TS, the PMT is included in the stream to be recorded, and the stream type is described in the PMT.

Then, the process for each of three stream types is performed from the thus checked stream type (ST1203).

That is, when the stream type is a video stream, an ESI indicating ES_TY = 0 is prepared based on the read data at step ST1213A, and attributes (resolution, aspect ratio, etc.) of the video corresponding to the PID are fetched from a component descriptor, and set to V_ATR at step ST1213B. The ES_PID, STREAM_TYPE, and COMPONENT_TAG are set to the PMT, and STREAM_COMPONENT, COMPONENT_TYPE are set from the component descriptor. Thereafter, the process advances to step ST1220.

When the stream type is an audio stream, an ESI indicating ES_TY = 0x40 is prepared based on the read data at step ST1215A. At step ST1215B, attributes (sampling frequency, quantization bit number, channel number, etc.) of the audio corresponding to the PID are fetched from an audio component descriptor, the ES_PID, STREAM_TYPE, and COMPONENT_TAG are set to the PMT, and STREAM_COMPONENT, COMPONENT_TYPE, SIMULCAST_GP_TAG, LANG_CDE are set to the audio component descriptor. Thereafter, the process advances to step ST1220.

When the stream type is another stream, an ESI indicating ES_TY = 0x80 is prepared based on the read data at step ST1217A. At step ST1217B, the ES_PID, STREAM_TYPE, and COMPONENT_TAG are set to the PMT, and DATA_COMP_ID, AD_DAT_COMP_INFO are set to a data encoding descriptor. Thereafter, the process advances to step ST1220.

At step ST1220, CP_CTRLR_INFO (copy control information in SOB_ESI) is prepared from the CP information belonging to a component tag corresponding to the ES.

The above-described process is repeated, when there is another elementary stream that has not prepared the ESI (YES at step ST1230).

When there is not any more stream that has not prepared the ESI (NO in ST1230), the process of FIG. 56 ends, and the process returns to ST130 of FIG. 50.

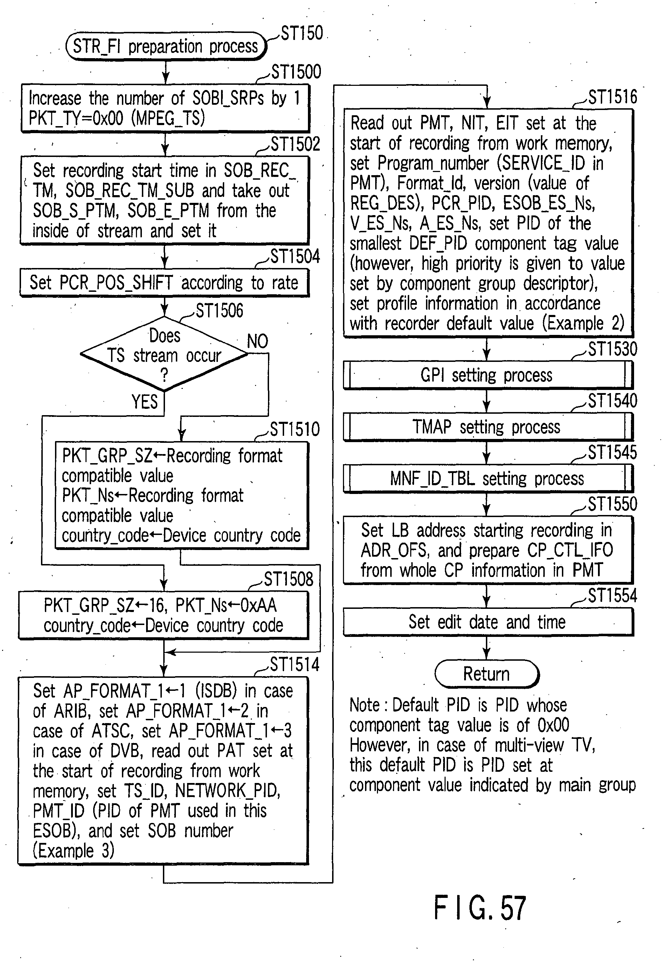

FIG. 57 is a flowchart (stream file information preparation process flow) showing one example of contents of the stream file information (STR_FI) preparation process in the recording end process (ST150) shown in FIG. 50.

At step ST1500, the number of search pointers SOBI_SRPs is increased by one to increase SOBI by one and PKT_TY = 0x00 (MPEG_TM) is set.

A recording start time is set to SOB_REC_TM, SOB_REC_TM_SUB, and start PTM (SOB_S_PTM) and end PTM (SOB_E_PTM) are fetched from the stream and set (ST1502). Here, a clock (the STC unit 102 of FIG. 41) inside the apparatus is set/corrected by a time data table (TDT), and a correct time is constantly obtained.

PCR_POS_SHIFT is set in accordance with a recording rate (ST1504).

It is determined whether or not the type of the stream is a transport stream (ST1506). When the type of the stream is a transport stream (TS stream, ARIB or DVB in a broadcasting system) (YES at ST1506), "16" is set to PKT_GRP_SZ, "0xAA" is set to PKT_Ns, and a country code of the apparatus is set to the country code (ST1508). When the type is not the transport stream (NO in ST1510), a value corresponding to the format to be recorded in the PKT_GRP_SZ is set, a value corresponding to the format to be recorded in the PKT_Ns is set, and the country code of the apparatus is set to the country code (ST1510).

At ST1514, 1 (ISDB-S) is set to AP_FORMAT in the case of ARIB, 2 is set to AP_FORMAT in the case of ATSC, and 3 is set to AP_FORMAT in the case of DVB, the PAT at the recording start is read from a work memory, and TS_ID, NETWORK_PID, PMT_ID (PID of the PMT for use in the present SOB) are set (ST1514). The SOB number is set (Example 3).

At step ST1516, the PMT, NIT, EIT at the recording start are read from the work memory, and Program_number (SERVICE_ID in the PMT), Format_Id, version (value of REG_DES), PCR_PID, ESOB_Es_Ns, V_ES_Ns, and A_Es_Ns are set. The PID having a smallest component tag value (additionally, the value set by the component group descriptor is preferential) is set to DEF_PID. Profile information is set based on default value of a recorder (Example 2).

A GPI setting process (described later) (ST1530), TMAPI setting process based on each item of delimitation information (described later) (ST1540), and an MNF_ID_TBL setting process (described later) (ST1545) are performed.

An LB address having started recording is set in ADR_OFS (FIG. 19) and CP_CTL_IFO is prepared based on a whole CP information of the PMT (ST1550). An edit date and time is set (ST1554). A default PID is set. The default PID corresponds to a PID of a component tag value 00 or PID of a stream which corresponds to a component tag described in a main component group in the case of a multi-view TV.

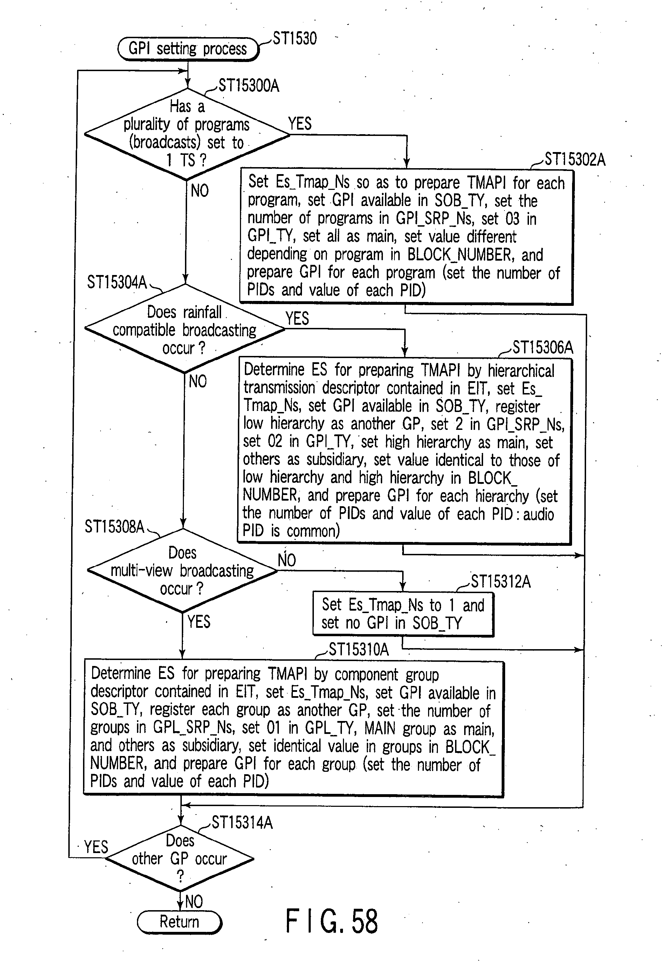

FIG. 58 is a flow chart illustrating an example (Example 1) of the GPI setting process ST1530 of FIG. 57. The GPI setting process contains two types of data schemes. A first method relating to the first data scheme will be described below with reference to FIG. 58.

A stream type is checked in various aspects (steps ST15300A, ST15304A, and ST15308A).

When a plurality of programs are prepared in one TS stream (YES at step ST15300A), Es_Tmap_Ns is set such that TMAPI is prepared for each program. SOB_TY is set such that GPI is present. The number of programs is set to GPI_SRP_Ns. 03 is set to GPI_TY and all is set as "main". Different values are set to BLOCK_NUMBER for each program. The number of PIDs and value of each PID are set such that GPI is prepared for each program (ST15302A).

In the case of rainfall compatible broadcasting (YES at step ST15304A), ES for preparing TMAPI is set based on a hierarchical descriptor in EIT. Es_Tmap_Ns is set. SOB_TY is set such that GPI is present. Data in a low hierarchy is registered as another GP and 2 is set to GPI_SRP_Ns. 02 is set to GPI_TY and data in a high hierarchy is set as "main" and remaining is set as "sub". The same value is set to BLOCK_NUMBER for a low hierarchy and a high hierarchy. The number of PIDs and value of each PID are set such that GPI is prepared for each hierarchy. PID for audio is common for all hierarchies (ST15306A).

In the case of multi-view broadcasting (YES at step ST15308A), ES for preparing TMAPI is set based on a hierarchical descriptor in EIT. Es_Tmap_Ns is set. SOB_TY is set such that GPI is present. Data in each group are registered as other GPs and the number of groups is set to GPI_SRP_Ns. 01 is set to GPI_TY and data of a main group is set as "main" and remaining is set as "sub". The same value is set to BLOCK_NUMBER for each group. The number of PIDs and value of each PID are set such that GPI is prepared for each group (ST15310A).

When NO is determined at step ST15308A, 1 is set to Es_Tmap_Ns and SOB_TY is set such that GPI is absent (ST15312A).

It is determined whether or not there is a remaining GP (ST15314A). When there is a remaining GP, the processing is returns to step ST15300A and when no remaining GP exists, this process is terminated.

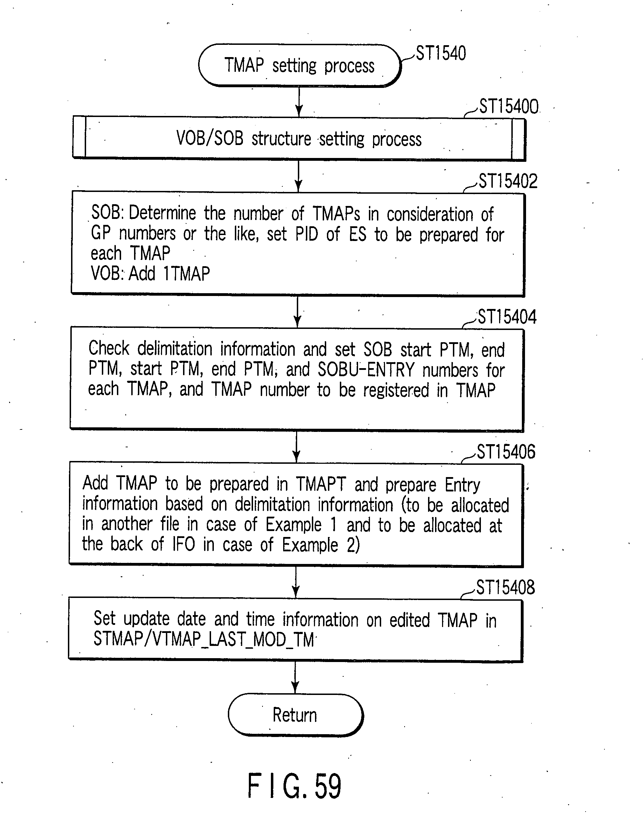

FIG. 59 is a flow chart illustrating the TMAP setting process ST1540 of FIG. 57.

The TMAP setting process is as follows.

A structure of VOB/SOB is determined (ST15400).

In the case of SOB, the number of TMAPs is determined in consideration of the number of GPs, etc.. ES_PID which is prepared for each TMAP is determined. In the case of VOB, one TMAP is added (ST15402). However, there is no need for always assigning one TMAP to one GP. When no setting is provided, playback, search, special playback, and the like are performed by utilizing TMAP of GP to which the same MAIN_GP or the same BLOCK_NUMBER is assigned.

At step ST15404, a SOB/VOB start time and end time; a start time and end time for each TMAP; the number of SOB_ENTRYs; a TMAP number to be registered to TMAPT are set based on delimitation information.

At step ST15406, a TMAP to be prepared for TMAPT is added, and ENTRY information is prepared based on delimitation information. Here, TMAPT information may be stored in another file (FIG. 3 or the like) and may be added at the end of an IFO file (FIG. 4 or the like). TMAPT information may be stored in another file (Example 1; FIG. 3) and may be added at the end of an IFO file (Example 1; FIG. 4).

At step ST15408, an updated date and time information for an edited TMAP is set to STAMP/VTMAP_LAST_MOD_TM.

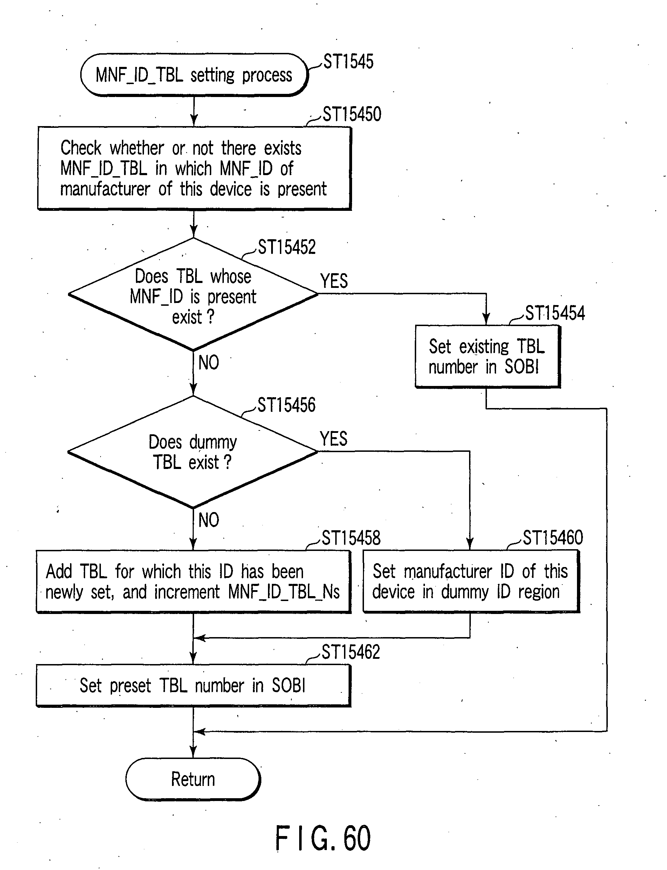

FIG. 60 is a flow chart illustrating an example (Example 1) of an MNF_ID_TBL setting process (ST1545) of FIG. 57. The MNF_ID_TBL setting process is performed as follows.

It is determined whether or not there is MNF_ID_TBL including MNF_ID of a manufacture of this device (ST15450, ST15452). When TBL exists, that TBL number is set in SOBI (ST15454), and this process is terminated.

When no TBL exists, it is determined whether or not a free space is present in TBL (ST15456). When such a free space exists, manufacturer ID of this device is set in the free space (ST15460). When no free space is present in TBL, TBL in which the present ID is set is added and MNF_ID_TBL_Ns is incremented (ST15458). At step ST15462, the set TBL number is set in SOBI, and this process is terminated.

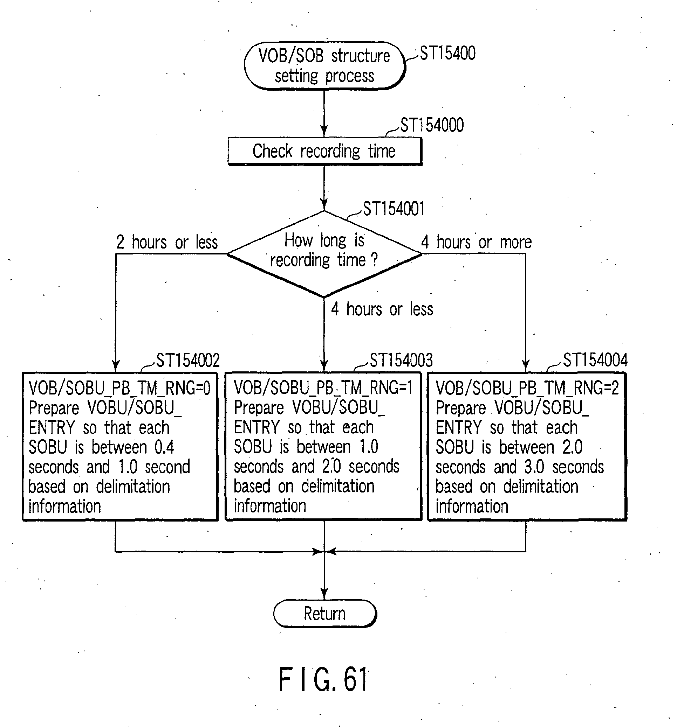

FIG. 61 is a flow chart illustrating the VOB/SOB structure setting process ST15400 of FIG. 59. The VOB/SOB structure setting process will be described below.

A recording time after recording is checked (ST154000). When the recording time is equal to or smaller than 2 hours, processing goes to step ST154002. When the recording time ranges 2 hours to 4 hours, processing goes to step ST154003. When the recording time is equal to or longer than 4 hours, processing goes to step ST154004.

At step ST154002, 0 is set in VOB/SOBU_PB_TM_RNG, VOBU/SOBU_ENTRY is prepared so that SOBU ranges from 0.4 seconds to 1 second based on delimitation information (information on 0.4 seconds to 1 second), and this process is terminated.

At step ST154003, 1 is set in VOB/SOBU_PB_TM_RNG, VOBU/SOBU_ENTRY is prepared so that SOBU ranges from 1 second to 2 seconds based on delimitation information (information on 0.4 seconds to 1 second); and this process is terminated.

At step ST154004, 2 is set in VOB/SOBU_PB_TM_RNG, VOBU/SOBU_ENTRY is prepared so that SOBU ranges from 2 seconds to 3 seconds based on delimitation information (information on 0.4 seconds to 1 second); and this process is terminated.

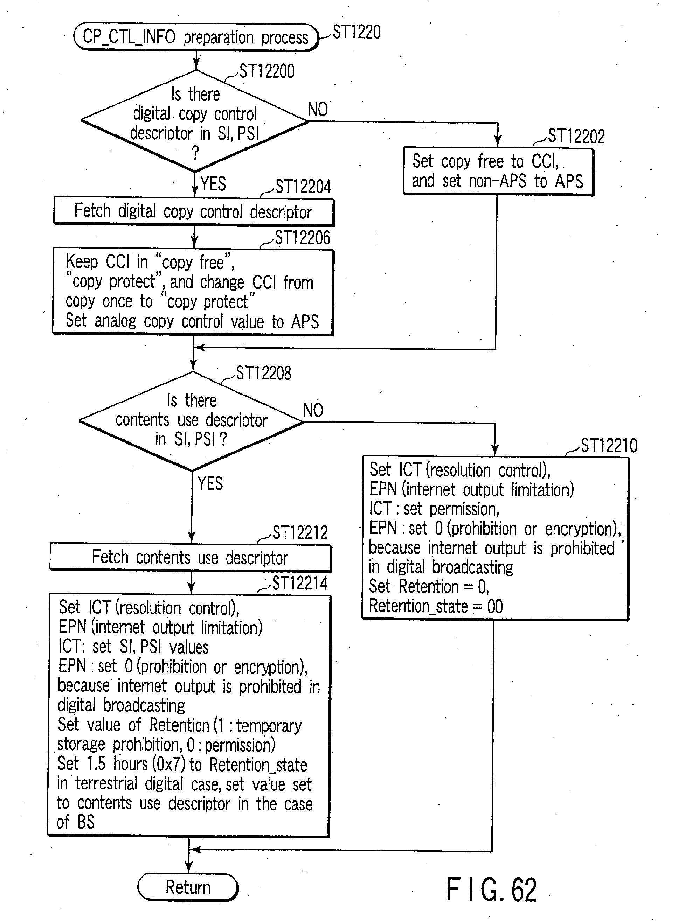

FIG. 62 is a flow chart illustrating the CP_CTL_INFO preparation process ST1220 of FIG. 56. The CP_CTL_INFO setting process will be described below.

It is determined at step ST12200 whether or not there is a digital copy control descriptor in latest program map tables PMT, EIT (SI, PSI). When there is the descriptor, the digital copy control descriptor is fetched at step ST12204. At step ST12206, copy free, copy protect in CCI are set as such, the copy protect is set in a case where one copying operation is permitted, and an analog copy control value is set to APS. When there is not any copy descriptor, at step ST12202, the copy free is set to CCI, and non-APS is set to the APS.

It is determined at step ST12208 whether or not there is a contents use descriptor in the latest program map tables PMT, EIT (SI, PSI). When the contents use descriptor is present, the descriptor is fetched at step ST12212, and ICT (resolution limitation) and EPN (internet output limitation) are set at step S12214. The value in the SI, PSI is set to the ICT, 0 (prohibition or encryption) is set to the EPN because an internet output is prohibited in the digital broadcasting, and a value of Retention (1: temporary storage prohibited, 0: permitted) is set. With respect to Retention_State, 1.5 hours (0x7) is set in the case of the terrestrial digital broadcasting, and the value set to the contents use descriptor is set in the case of the BS. The process returns to an original routine.

When there is not any contents use descriptor, at step ST12210, the ICT (resolution limitation) and EPN (internet output limitation) are set. Permission is set to the ICT, 0 (prohibition or encryption) is set to the EPN because the internet output is prohibited in the digital broadcasting, 0 is set to the value of Retention, 00 is set to Retention_State, and the process returns to the original routine.

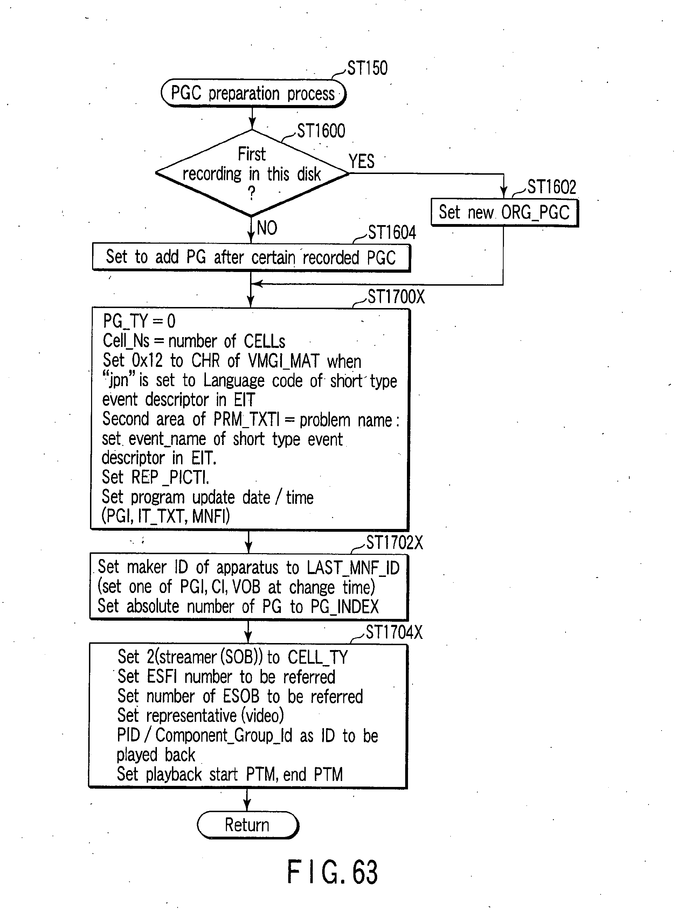

FIG. 63 is a flowchart of an EX_PGC preparation process which is a part of the step ST150 during the recording process of FIG. 50.

It is determined at step ST1600 whether or not the recording is first recording with respect to the disk. In the first recording, ORG_EX_PGC is prepared anew at step ST1604. When the recording is not the first recording, EX_PGC is added after a certain recorded ORG_EX_PGC at step S1602.

At step ST1700X after steps ST1604 and ST1602, erase permission: 0 is set to PG_TY, and the number of the CELLs is set to Cell_Ns. In the ARIB system, when "jpn" is set to language_code of a short type event descriptor in the EIT, 0x12 is set to CHR of VMG_MAT, EVENT_NAME is set in a second area of PRM_TXTI, and information of a representative picture is set to REP_PICTI.

At step ST1702X, manufacture ID of the present apparatus is set to LAST_MNF_ID. When there is a change in PGC, CI, VOB, the manufacture ID of the changed apparatus is set to the value, the value is set to indicate a manufacture which has performed editing/recording at last, and accordingly the change by the different manufacture is easily handled. Furthermore, an absolute number of PG is set to PG_INDEX, and reference by a PG unit is enabled in a case where reference is made from another application software or the like. Furthermore, the present PG update date/time information is recorded. In this case, when there is the corresponding (manufacture's code is matched) MNFI or IT_TXT in the present apparatus, the update date/time information of the corresponding data is also set. Information peculiar to each manufacture is set to the MNFI.

At step ST1704X, information for identifying the streamer (SOB) is set to CELL_TY. An STI_FI number to be referred to, and the number of the SOB to be referred to are set. As the ID to be played back, a representative (video) packet identifier PID, or Component_Group_Id is set, and the number of EPIs, playback start PTM, end PTM, and EP are set, respectively.

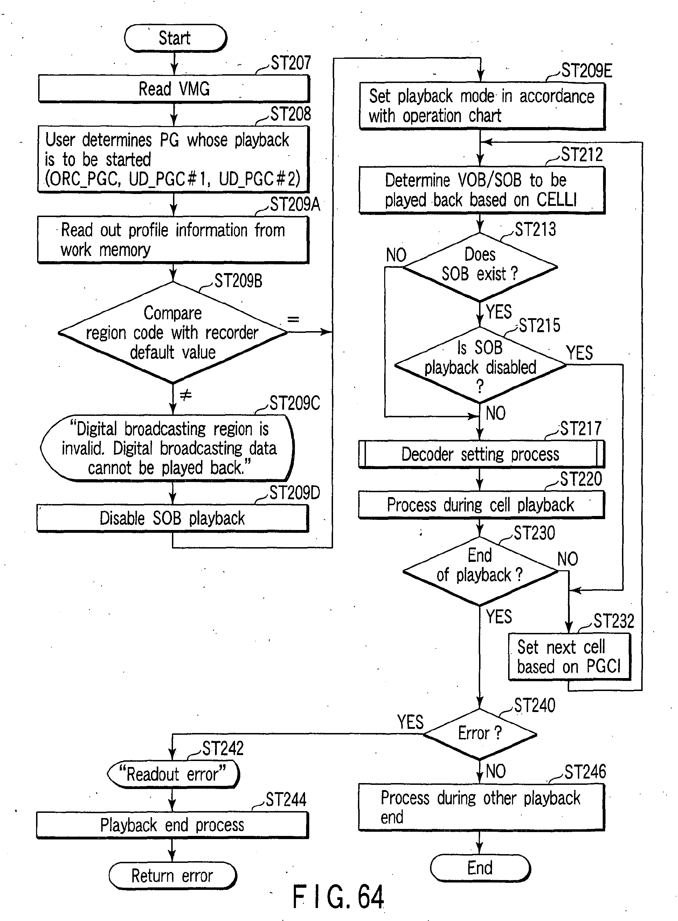

FIG. 64 is a flow chart (whole playback operation flow) illustrating an example of a playback operation in the apparatus of FIG. 41. Data processes during playback are performed as follows (refer to FIGS. 64 to 67).

Before entering a playback process, a disk check is made, and it is determined whether or not the disk is a write once disk or a rewritable disk (R, RW, RAM); hereinafter, temporarily referred to as a "rewritable disk". When the disk is not a rewritable disk, the fact is returned, and processing is terminated.

A disk file system is read out, and it is determined whether or not recorded data is present. When the data is not present, a message "no data is recorded" is displayed, and processing is terminated.

At the playback process, a VMG file is read (ST207), and a program to be played back is determined by a user (ST208). VMG information is stored in the work RAM 80A of FIG. 41. From this work RAM 80A, profile information (FIGS. 8 and 10 and the like) is read out (ST209A). Then, a region code contained in the read-out profile information is compared with a region value which a recorder (the apparatus of FIG. 41) has as a default (ST209B).

If the region is not matched, the fact is displayed, for example "Region of digital broadcast is not matched so that digital broadcast data cannot be played back" is displayed (ST209C). Playback of SOB for which a region is not matched is disabled (ST209D). On the other hand, when the region is matched, playback is enabled and a playback mode is set in accordance with an operation chart (FIG. 45) (ST209E). Then, a cell to be played back is determined (selected by the user). When playback in order of recording has been selected, playback is performed in accordance with ORG_PGCI. When playback for each program is performed, playback is performed in accordance with UD_PGC of a number which corresponds to a program desired to be played back.

A value of PKT_TY is read out, and it is determined whether or not the read-out value is. a compatible broadcast scheme. When it is not compatible, the fact is displayed, and processing is terminated (or processing goes to the next CELL).

SOB/VOB to be played back is determined from CELLI to be played back (ST212), and a file pointer (logical address) FP for starting playback is determined from playback start PTM. Further, the setting of each decoder unit is provided based on the value of STI (ST217), and preparation for playback is performed (ST220). In addition, from CCI contained in the first packet group header, APS setting is provided to a video decoder. Then, APS ON/OFF setting, ATS type setting and the like are provided, and CGMSA setting is provided to the video decoder by means of digital copy control. Furthermore, when a digital output (such as IEEE 1394, Internet) occurs, output IC is set as 0: Scramble ON or output disable or 1: Output based on a value of EPN. When ICT is 0, a certain limitation is applied to image resolution. Then, HD (high resolution) is converted into SD (standard resolution). When ICT is 1, output IC is set so as to be outputted as is. At this time, when a frame starting playback is not an I-picture, another I-picture immediately preceding that non-I picture is read out, and decoding is started from the I-picture. Then, displaying is started when a target frame is reached, and normal playback is started.

A process at the time of starting playback is performed (ST212).

The setting of each decoder (described later) is provided (ST217).

A cell playback process (described later) is performed (ST220), and it is determined whether or not playback is terminated (ST230). When playback is terminated, an error check is made (ST240). When an error occurs, the fact is displayed (ST242). When no error occurs, a playback end process is performed (ST240), and this operation is terminated.

A next cell is determined from PGCI (ST232), and it is determined whether or not decoder setting has been changed. When the setting has been changed, a change attribute is set at the decoder so that decoder setting is changed to the next sequence end code.

It is determined whether or not playback has been terminated at step ST230. When playback has not been terminated, processing goes to step ST232.

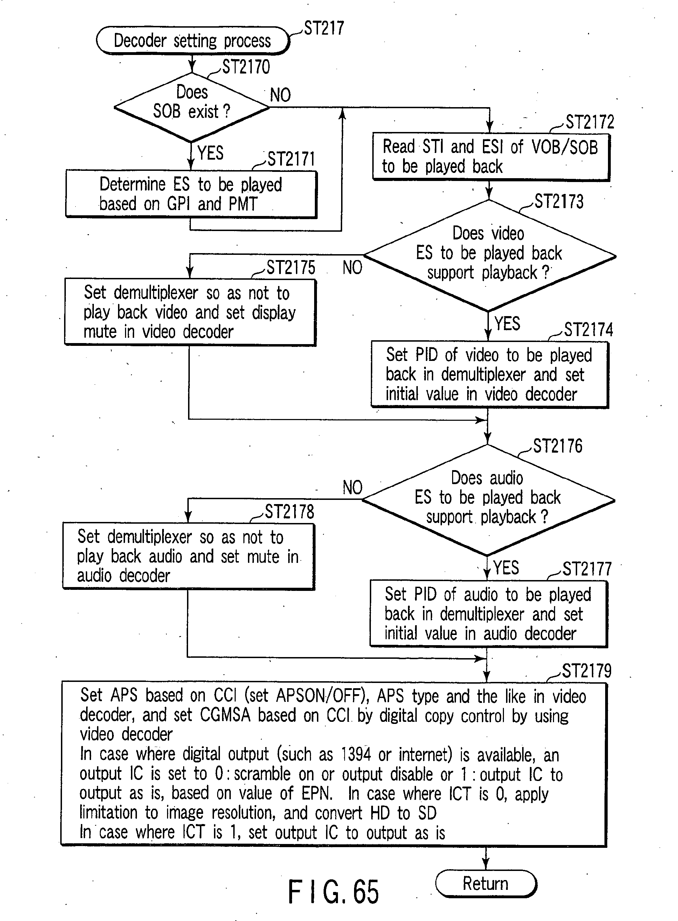

FIG. 65 is a flow chart illustrating the decoder setting process of FIG. 64.

First, it is determined whether the SOB is input (ST2170). When the SOB is input, ES to be played back is determined in accordance with GPI and PMT (ST2171). Attribute information STI and ESI of VOB/SOB to be played back are read (ST2172).

It is determined whether or not the video ES to be played back supports playback (ST2173). When the video ES supports playback, PID of a video to be played back is set to the demultiplexer and the video decoder,is initialized (ST2174). When the video ES does not support playback, the demultiplexer is set such that the video is not played back and "display mute" is set to the video decoder (ST2175).

It is determined whether or not the audio ES to be played back supports playback (ST2176). When the audio ES supports playback, PID of an audio to be played back is set to the demultiplexer and the audio decoder is initialized (ST2177). When the audio ES does not support playback, the demultiplexer is set such that the audio is not played back and "audio mute" is set to the audio decoder (ST2178).

At step ST2179, setting of APS (APSON/OFF, APS type and the like) is set to a video decoder based on CCI. Setting of CGMSA is set to a video decoder by a digital copy control based on CCI. When there is a digital output (IEEE 1394, Internet and the like), 0 (scramble on or output inhibit) or 1 (output as it is) is set to an output IC based on a value of EPN. When ICT is 0, the resolution of image is limited and HD is changed to SD. When ICT is 1, 1 (output as it is) is set to an output IC. In this case, in a case where a frame to start the playback is not the I picture, the previous I picture is read, the decoding is started from the read picture, the display is started at a target frame, and usual reproduction is started.

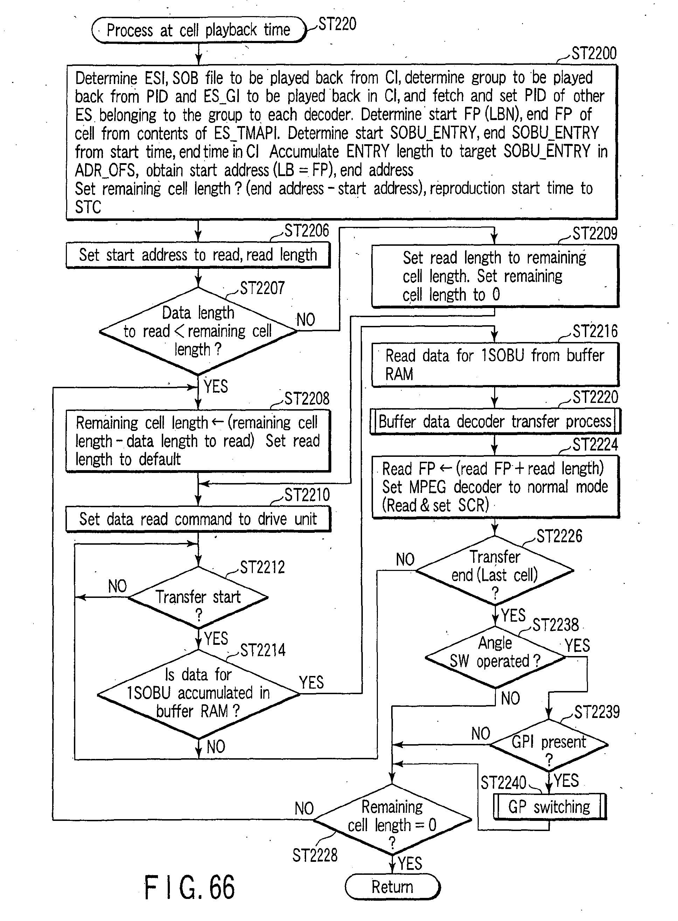

FIG. 66 is a flow chart illustrating an example of a process (ST220) during cell playback of FIG. 64. The cell playback process is performed as follows.

At step ST2200, elementary stream information ESI to be played back, and stream object (SOB) file are determined from cell information CELLI (CI), a group to be played back is determined from the program ID (PID) to be played back in the cell information CI and elementary stream general information ES_GI, and the PID of another ES belonging to the group is fetched and set to each decoder. Furthermore, a start file pointer FP (LBN) of the cell, and end FP are determined from the contents of the ES_TMAPI. Start SOBU_ENTRY, and end SOBU_ENTRY are determined from start time and end time in the CI. A data length of ENTRY to the target SOBU_ENTRY is accumulated in ADR_OFS to obtain a start address (LB = FP), and an end address. A remaining CELL length is a value obtained by subtracting the start address from the end address, and a playback start time is set to the STC. That is, the PID to be played back is fetched from the CI, and collated with SOB_GPI, the GPI to be played back is determined, each PID of the GP is set to the demultiplexer, and each ES is input into the decoder (STB, digital tuner).

Setting is performed in such a manner as to perform a decoding process inside the decoder unit.

At step ST2206, a read process during the playback is executed, and a start address, and read length (read size) read from the start file pointer are determined.

At step ST2207, a read unit size to be read is compared with a remaining cell length. When the remaining cell length is larger, at step ST2208, a value obtained by subtracting the read unit size to be read from the remaining cell length is set to the remaining cell length, and a read length is set to the default. When the remaining cell length is smaller, at step ST2209, the read length is set to the remaining cell length, and the remaining cell length is set to 0.

At step ST2210, the read length is set to the length of the read unit, and read address, read length, and read command are set to the drive unit.

After the transfer starts (YES at step ST2212), at step ST2214, the process waits until data for one SOBU is accumulated in the buffer RAM. When the data for one SOBU is accumulated, the data is read from the buffer RAM at step ST2216, a buffer decoder transfer process is performed at step ST2220, and the process shifts to the next step.

At step ST2224, read FP is increased by the read length and updated, and the MPEG decoder is set to a normal mode (reading & setting of a system clock reference SCR).

It is determined at step ST2226 whether or not the transfer has ended. When the transfer ends, it is determined at step ST2238 whether or not an angle switch is operated. When the angle switch is operated, it is determined at step ST2239 whether or not GPI is present. When the GPI is present, GP switching is performed (ST2240).

It is determined at step ST2228 whether the remaining cell length is 0. When the remaining cell length is 0, the present process ends. When the remaining cell length is not 0, the process returns to step ST2206.

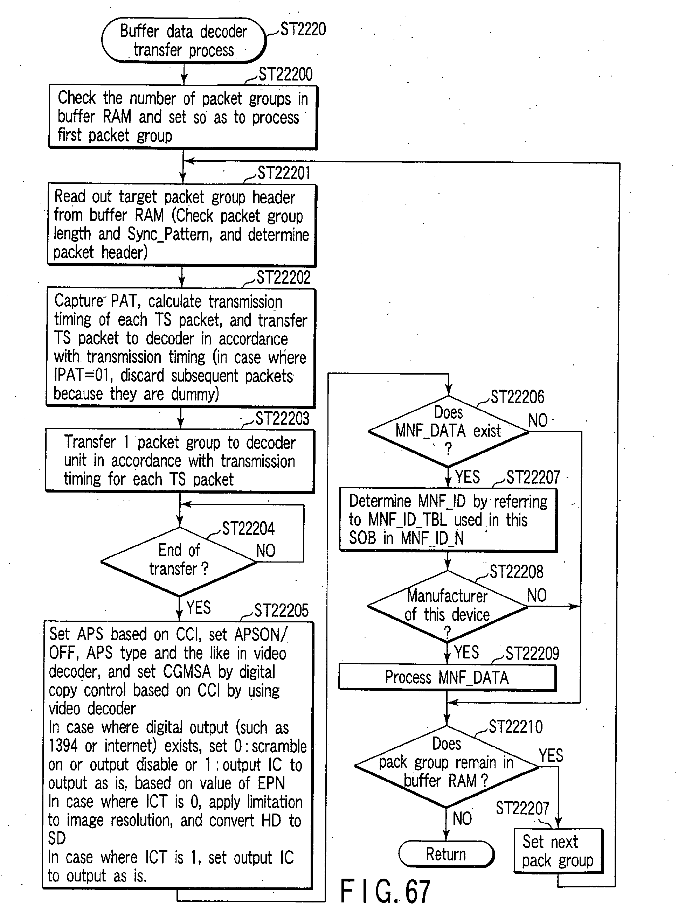

Details of the buffer decoder transfer process (ST2220) of FIG. 66 are shown in FIG. 67.

At step ST22200, the number of the packet groups in the buffer RAM is checked. When there is not one packet group, the present process ends. When there are one or more groups, the setting is performed in such a manner as to process the first packet group.

At step ST22201, the target packet group header is read from the buffer RAM. The head of the packet group (packet group header) is detected from a pattern group length and Sync_Pattern.

At step ST22202, a packet arrival time PAT is read and a transfer timing of each TS packet is calculated. TS packet is transmitted to the decoder according to the transfer timing. When IPAT = 01, remaining data is discarded since it is dummy data.

At step ST22203, each packet is sent to the decoder unit (STB unit) at a transfer time of each TS packet.

At step ST22204, the process waits till the end of the transfer.

At step ST22205, setting of APS (APSON/OFF, APS type and the like) is set to a video decoder based on CCI. Setting of CGMSA is set to a video decoder by a digital copy control based on CCI. When there is a digital output (IEEE 1394, Internet and the like), 0 (scramble on or output inhibit) or 1 (output as it is) is set to an output IC based on EPN. When ICT is 0, the resolution of image is limited and HD is changed to SD. When ICT is 1, 1 (output as it is) is set to an output IC.

After this output IC setting, it is determined whether or not MNF_DATA is present by checking MNF_SS (ST22206). When the MNF_DATA is present, MNF_ID indicated by MNF_ID_N is determined from MNF_ID_TBL indicated by MNF_TBLN in SOBI (ST22207). Then, it is determined whether or not this ID matches manufacturer ID of this device (ST22208). When the check result is affirmative, a process specific to this device is performed in accordance with the value of MNF_DATA (ST22209).

In the case where the result is NO at step ST22206, in the case where the result is NO at step ST22208, and in the case where the process at step ST22209 terminates, it is then determined whether or not a next packet group remains in the buffer RAM (ST22210). When such a packet group does not remain (NO at step ST22210), this process is terminated. When such a packet group remains (YES at step ST22210), setting is provided so as to process a next packet group (ST22207), and processing returns to step ST22202.

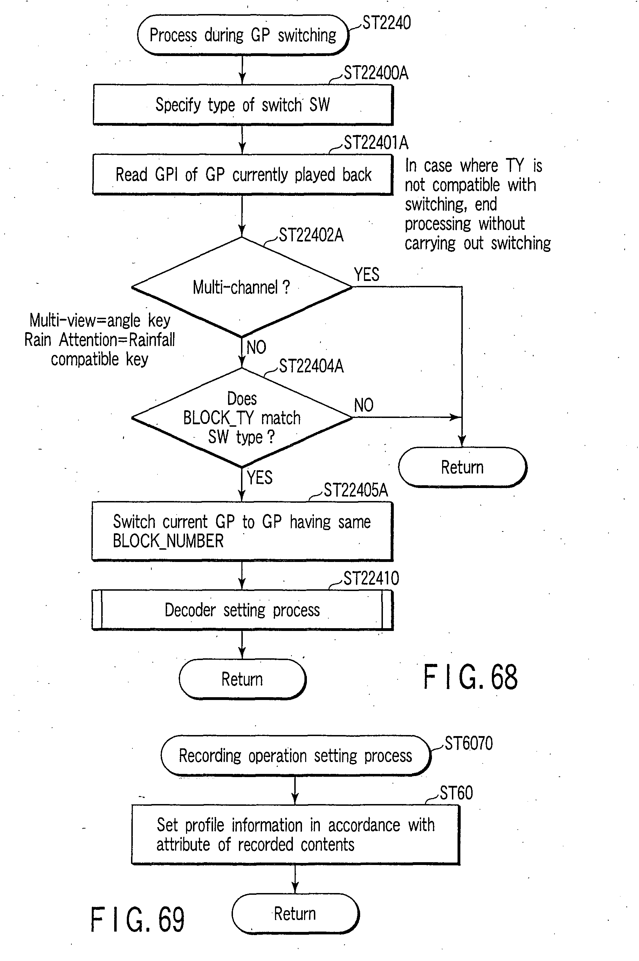

FIG. 68 is a flow chart illustrating an example (Example 1) of the GP switch setting process ST2240 of FIG. 66. The GP switching process is believed to have two types of GPI schemes, as described above. In addition to these two types of methods, a further simpler method may be assumed. First is as shown in FIG. 68.

Type of the switch SW is checked (ST22400A).

GPI of currently played-back GP is read (ST22401A).

When SW type is a multi-channel type (YES at step ST22402A), there is a possibility that a video image attribute changes. Thus, processing is terminated without carrying out switching (in the case of type which is not compatible with switching, processing is terminated without doing anything).

When SW type is not a multi-channel type, (NO at step ST22402A), it is determined whether or not SW type is present in BLOCK_TY which exists in GP (ST22404A). When SW type is not present, processing is terminated without doing anything.

When SW type is present in BLOCK_TY (YES at step ST22404A), BLOCK_NUMBER is obtained from BLOCK_TY, and GP having the same BLOCK_NUMBER is checked. When GP having the same BLOCK_NUMBER is present, GPI information is read in order to switch the current setting to the GP (ST22405A), and then, a decoder setting process is performed (ST22410).

A method as shown in FIG. 69 may be used for a recording operation setting process. FIG. 69 is a flow chart illustrating an example of the recording operation setting process ST6070 of FIG. 55. That is, disk profile information is set in accordance with an attribute of the recorded contents (ST60).

In this manner, a fine control operation compatible with digital broadcasting can be achieved.

<Summary of embodiments>

(1) It is determined whether or not a stream in a disk can be played back in accordance with a recorder option compatible level (FIGS. 5, 44, and 45 and the like).

(2) A stream which can be decoded is determined in accordance with a region code, and it is determined whether or not playback of that disk is enabled (FIGS. 5 and 44 and the like)..

(3) Time map (TMAP) information is recorded in a separate region, and an amount of processing during TMAP change is reduced (see TMAP file of FIG. 3, TMAPT at the end of HDVR_VMG of FIG. 4, and the like).

(4) A group type (number) is added to group information, and it is determined whether or not such a group type is a switchable group (FIG. 31 and the like) .

(5) MNF_ID_TBL is provided, and ID in the above table (ID of company having undergone recording operation) is specified in a packet group header contained in contents data.

(6) Types of playback of corresponding streams are limited to combinations as shown in FIG. 45, (the number of which is limited to some extent). Then, the combinations are written in profile information (FIGS. 5 and 10 and the like) contained in control 'information. As a recording and playback apparatus (such as a DVD recorder), only a decoder according to its intended purpose may be mounted, and device configuration can be relatively lightened. For example, in device targeted for a user having an intended use of only AAC audio instead of AC3 audio, the mounting of only an AAC decoder will suffice.

(7) A usable region can be specified in accordance with a region code, thus making it unnecessary for device to have hardware components compatible with all regions. For example, even if ATSC (US Digital Broadcasting Standard) is supported by a recorder used in Japan, such hardware components are hardly used. Thus, it is advisable that the recorder be mounted so as to be compatible with ARIB (Japanese Digital Broadcasting Standard) only.

In this way, device cost can be reduced by selecting a decoder mounted in accordance with an intended use and/or use region. In other words, it becomes possible to construct an optimal recorder according to an object of a product.

That is, a recorder compatible with a region and having a different grade can be easily prepared at a product price suitable to the grade, based on profile information.

(8) In a disk capable of recording and playback (in particular, in a disk having a large capacity), a data amount of the control information is likely to increase. In this case, time map (TMAP) information is stored in a separate file, whereby the target process can be performed quickly during frequent rewriting of TMAP. Alternatively, TMAP information is allocated at the end of control information (VMG), only the TMAP information can be changed independent of another item of control information allocated in front of the TMAP information during frequent rewriting of time map information. Thus, the target process can be performed quickly.

That is, TMAP is recorded in a separate region of a recording medium, thereby making it possible to reduce a burden on processing at the device side using this recording medium.

(9) A disk management region (HDVR_VMG of FIG. 7) includes a stream file information table (STR_FIT). Group type information (such as GP_TY and BLOCK_NUMBER) can be provided in a data hierarchy of this STR_FIT (SOBI of FIG. 8; SOB_ES_GPI of FIG. 9; SOB_ES_GPI of FIG. 30; GPI of FIG. 30; and GPI_GI of FIG. 31). By using this information (such as GP_TY and BLOCK_NUMBER of FIG. 31), a variety of broadcast formats (such as a multi-view format, a rainfall compatible format, or a multi-channel format) can be identified. In addition, a switchable group (for example, multi-channel broadcast) can be switched by that number (GP_NUM).

That is, plural types of groups can coexist due to a change of group information (GPI).

(10) Information unique to manufacturers is recorded in advance in a recording medium, thereby making it possible for recording device to have a unique function which is not described in DVD specifications according to manufacturers or device type and to provide discrimination from other competitors. In addition, MNF_ID_TBL is provided, and the ID in that table (the ID of company having undergone recording operation) is specified in a packet group header contained in contents data, thereby making it possible to provide the above unique function and discrimination in a header having its limited capacity.

According to the present invention, there can be provided an information recording medium (or data structure); an information recording/playback method; and an information recording/playback apparatus capable of efficiently storing manufacturer information relating to recording device in a recording medium and capable of easily reading out the stored information from the recording medium.

While the description above refers to particular embodiments of the present invention, it will be understood that many modifications may be made without departing from the spirit thereof. The accompanying claims are intended to cover such modifications as would fall within the true scope and spirit of the present invention. The presently disclosed embodiments .are therefore to be considered in all respects as illustrative and not restrictive, the scope of the invention being indicated by the appended claims, rather than the foregoing description, and all changes that come within the meaning and range of equivalency of the claims are therefore intended to be embraced therein.

## Claims

**1.** An information recording medium for recording a digital stream signal, **characterized by** comprising:

a management region (20, 23) for storing control information and a table (MNF_ID_TBL) of identifiers representing manufactures of recording devices; and

a data region (30) for storing objects (SOBU) storing portions of the digital stream signal and number information for specifying an identifier in the table representing a manufacturer of a recording device which records the objects in the data region.

2. A method for recording a digital stream signal in an information recording medium according to claim 1, the method **characterized by** comprising:

storing, in the management region, control information and a table (MNF_ID_TBL) of identifiers representing manufactures of recording devices in the management region;

storing, in the data region, objects (SOBU) storing portions of the digital stream signal and number information for specifying an identifier in the table representing a manufacturer of a recording device which records the objects;

selecting number information specifying a specified identifier in the table; and

recording the selected number information in the data region.

3. A method for reproducing information from an information recording medium according to claim 1, the method **characterized by** comprising:

specifying a device manufacturer having undergone recording operation to the information recording medium based on the number information read from the data region; and

executing a function unique to a device manufacture in accordance with the specified device manufacturer.

4. An apparatus for recording a digital stream signal in an information recording medium according to claim 1, **characterized by** comprising:

means for storing, in the management region, control information and a table (MNF_ID_TBL) of identifiers representing manufactures of recording devices in the management region;

means for storing, in the data region, objects (SOBU) storing portions of the digital stream signal and number information for specifying an identifier in the table representing a manufacturer of a recording device which records the objects;

means for selecting number information specifying a specified identifier in the table; and

means for recording the selected number information in the data region.

5. An apparatus for reproducing information from an information recording medium according to claim 1, **characterized by** comprising:

means for specifying a device manufacturer having undergone recording operation to the information recording medium based on the number information read from the data region; and

means for executing a function unique to a device manufacture in accordance with the specified device manufacturer.

FIG. 1A

Disk-shaped information storage medium 100

FIG. 1B

| 110 | 111 | 112 | 113 |
|---|---|---|---|
| Lead-in area | Volume/file atructure information area | Data area | Lead-out area |

FIG. 1C

| 120 | 121 | 120 |
|---|---|---|
| General computer information recording area | AV data recording area | General computer information recording area |

FIG. 1D

| 130 | 122 | 131 |
|---|---|---|
| AV data management information recording area | VR object group recording area | Stream object group recording area |

FIG. 1E

| 132 | 132 | | 132 |
|---|---|---|---|
| Stream object | Stream object | · · · | Stream object |

FIG. 1F

| 134 | 134 | | 134 |
|---|---|---|---|
| SOBU | SOBU | · · · · · · · · · · · · · | SOBU |

FIG. 1G

| 140 | 140 | | 140 |
|---|---|---|---|
| Packet_Group | Packet_Group | · · · · · · · · · · · · · | Packet_Group |

Packet_Group length = 16 Packs

FIG. 1H

| 160 |
|---|
| DVD-TS packet recording area |

FIG. 1I

| 161 | 163 | 162 | 163 | 162 | | 162 |
|---|---|---|---|---|---|---|
| Packet_Group Header | PAT | MPEG-TS packet | PAT | MPEG-TS packet | · · · | MPEG-TS packet |

Presentation control information layer 10

| Program chain (PGC) | | | | | | | | 11 |
|---|---|---|---|---|---|---|---|---|

| Program | | | | Program | | ······ | Program | | 12 |
|---|---|---|---|---|---|---|---|---|---|

13 | 13 | 13 12 | 13 12 | 13

| Cell | Cell | Cell | Cell | ······ | Cell | Cell | 13 |
|---|---|---|---|---|---|---|---|

Playback time : designate playback place of cell by PTS

Stream object control information layer 20

VOB management information layer 23

21

| SOBI | | SOBI | | ···· |
|---|---|---|---|---|
| SOBUI | ··· | SOBUI | SOBUI | ······ 21 |

| VOBI | | ··· | 24 |
|---|---|---|---|
| VOBUI | ··· | VOBUI | 25 |

25

Global information 22    Stream object layer 30

31

| SOB | | SOB | ··· | 31 |
|---|---|---|---|---|

VOB 38

| SOBU | | SOBU | SOBU | ··· | SOBU | ··· |
|---|---|---|---|---|---|---|

32    33    32768 Bytes    33

VOBU ··· 39

40    40

| Packet_Group | Packet_Group | ···· For a predetermined period of playback time | Packet_Group |
|---|---|---|---|

| Pack | Pack | ··· |
|---|---|---|

33

VOB layer 37

152 Bytes 34    4 Bytes 35    188 Bytes 36    36

| Packet_Group Header | PAT | TS Packet_1 | ··· | TS Packet_170 |
|---|---|---|---|---|

FIG. 2

Root

DVD_HDVR

| | |
|---|---|
| HR_MANGER.IFO | HDVMG File |
| HR_VTMAP.IFO | VOB TMAP File |
| HR_STMPx.IFO | SOB TMAP File |
| HR_MOVIEO.VRO | VR Object File |
| HR_STRxx.SRO | SR Object File |
| HR_STILL.VRO | Still Video Object file |
| HR_AUDIO.VRO | Audio object file |
| HR_VTMAP.BUP | VOB TMAP Backup File |
| HR_STMPx.BUP | SOB TMAP Backup File |
| HR_MANGER.BUP | HDVMG Backup File |

F I G. 3

EP 1 553 768 A2

130 ~ HDVR_VMG

| | |
|---|---|
| 1310 ~ | HDVR_VMGI |
| | ... |
| 1380 ~ | M_AVFIT |
| | ... |
| 1320 ~ | STR_FIT |
| 1330 ~ | Program chain information (EX_PGCI) |
| 1340 ~ | Play list information (EX_PL) |
| 1350 ~ | EX_TXTD_MG |
| 1360 ~ | EX_MNFIT |
| 1370 ~ | TMAPT (Ex. 2) |

| | |
|---|---|
| Disk management identification information (VMG_ID) | ~ 1311 |
| ... | |
| Version number of DVD video specifications (VERN) | ~ 1312 |
| VTMAP_LAST_MOD_TM | ~ 1317 |
| STMAP_LAST_MOD_TM | ~ 1318 |
| Start address of stream object control information (SFIT_SA) | ~ 1314 |
| ... | |
| Start address of program chain information (EX_PGCI_SA) | ~ 1315 |
| Start address of play list information (EX_PL_SA) | ~ 1316 |

VTMAP_LAST_MOD_TM : Date and time during updating of VTMAPT is entered, thereby preventing mismatch of VTMAPT

STMAP_LAST_MOD_TM : Date and time during updating of STMAPT is entered, thereby preventing mismatch of STMAPT

FIG. 4

FIG.5

EP 1 553 768 A2

| | |
|---|---|
| STR_FI_Ns : Number of STR_FI | ~13211 |
| STR_FIT_EA : End Address of STR_FIT | ~13212 |

| | |
|---|---|
| 1321 ~ | STR_FITI |
| 1323 ~ | STR_FI_SRP #1 |
| | ... |
| 1323 ~ | STR_FI_SRP #n |
| 1322 ~ | STR_FI #1 |
| | ... |
| 1322 ~ | STR_FI #n |

| | |
|---|---|
| STR_FI_GI | ~13221 |
| SOBI_SRP #1 | ~13222 |
| ... | |
| SOBI_SRP #K | ~13222 |
| MNF_ID_TBL #1 | ~13224 |
| ... | |
| MNF_ID_TBL #m | ~13224 |
| SOBI #1 | ~13223 |
| ... | |
| SOBI #K | ~13223 |

F I G. 6

| | |
|---|---|
| STR_FI_GI — 13221 | FILE_NAME — 132211 |
| SOBI_SRP #1 — 13222 | AP_FORMAT-1 — 132212 |
| ... | COUNTRY_CODE — 132213 |
| SOBI_SRP #K — 13222 | PKT_TY — 132214 |
| MNF_ID_TBL #1 — 13224 | PKT_SZ — 132215 |
| ... | PKT_GRP_SZ — 132216 |
| MNF_ID_TBL #m — 13224 | PKT_Ns — 132217 |
| SOBI #1 — 13223 | SOBI_SRP_Ns : Number of SOBI_SRPs — 132218 |
| ... | MNF_ID_TBL_Ns : Number of NMF_ID_TBLs — 132219 |
| SOBI #K — 13223 | |

| |
|---|
| MNF_ID #0 — 132241 |
| ... |
| MNF_ID #7 — 132241 |

| |
|---|
| SOBI_GI — 132231 |
| SOB_ESI #1 — 132232 |
| ... |
| SOB_ESI #m — 132232 |
| SOB_SMLI — 132233 |
| SOB_AGAPI — 132234 |
| SOB_TMAPI — 132235 |
| SOB_ES_GPI — 132236 |

PKT_TY : 01=MPEG-TS, 0xff=Non cognizant
PKT_SZ : 00BCh=188
PKT_GP_SZ : Packet Group size=8LB
PKT_Ns : Packet number of 1PKT_GPP=0xAA
Country_code : Recorded country code (Japan : JP)
AP_FORMAT-1 : Type of digital broadcasting such as
ISDB, DVB, or ATSC
MNF_ID_TBL : Reference table of MNF_ID
(MNF_IDs for 8 companies are set)
This table is specified for each SOB
When MNF_ID=ALL 0, region of that ID is unused

FIG. 7

| | |
|---|---|
| SOBI_GI | 132231 |
| SOB_V_ESI#1 | 1322321 |
| ... | |
| SOB_V_ESI#m | 1322321 |
| SOB_A_ESI#1 | 1322322 |
| ... | |
| SOB_A_ESI#m | 1322322 |
| SOB_SMLI | 132233 |
| SOB_AGAPI | 132234 |
| SOB_TMAP | 132235 |
| SOB_ES_GPI | 132236 |

| | |
|---|---|
| SOB_TY | 13223101 |
| AP_FORMAT_2 | 13223102 |
| SOB_PROFILE | 13223103 |
| PMT_PID | 13223104 |
| PCR_PID | 13223105 |
| NETWORK_ID | 13223106 |
| TS_ID | 13223107 |
| PROGRAM_NUMBER | 13223108 |
| Format_ID | 13223109 |
| SERVICE_TYPE | 13223110 |
| CP_CTRL_INFO | 13223111 |
| Reserved | |
| SOB_REC_TM | 13223112 |
| SOB_REC_TM_SUB | 13223113 |
| LOCAL_TM_ZONE | 13223114 |
| SOB_DEF_PID | 13223115 |
| SOB_S_PTM | 13223116 |
| SOB_E_PTM | 13223117 |
| SOB_DURATION | 13223118 |
| PCR_POS_COUNT | 13223119 |
| PCR_POS_SHIFT | 13223120 |
| SOB_ES_Ns | 13223121 |
| SOB_V_ES_Ns | 13223122 |
| SOB_A_ES_Ns | 13223123 |
| MNF_ID_TBLNs | 13223124 |
| SOB_NUMBER (Example 3) | 13223125 |

# FIG. 8

| | |
|---|---|
| 13223101 | SOB_TY |
| 13223102 | AP_FORMAT_2 |
| 13223103 | SOB_PROFILE |
| 13223104 | PMT_PID |
| 13223105 | PCR_PID |
| 13223106 | NETWORK_ID |
| 13223107 | TS_ID |
| 13223108 | PROGRAM_NUMBER |
| 13223109 | Format_ID |
| 13223110 | SERVICE_TYPE |
| 13223111 | CP_CTRL_INFO |
| | Reserved |
| 13223112 | SOB_REC_TM |
| 13223113 | SOB_REC_TM_SUB |
| 13223114 | LOCAL_TM_ZONE |
| 13223115 | SOB_DEF_PID |
| 13223116 | SOB_S_PTM |
| 13223117 | SOB_E_PTM |
| 13223118 | SOB_DURATION |
| 13223119 | PCR_POS_COUNT |
| 13223120 | PCR_POS_SHIFT |
| 13223121 | SOB_ES_Ns |
| 13223122 | SOB_V_ES_Ns |
| 13223123 | SOB_A_ES_Ns |
| 13223124 | MNF_ID_TBLN |
| 13223125 | SOB_NUMBER (Example 3) |

SOB_TY : b13=0 : Normal SOB, b13=1 : Temporary erase SOB
    b12=0 : No GPI, b12=1 : GPI is available
AP_FORMAT_2 : ISDB-S, ISDB-T, ATSC_1···
Information of recorded PSI or SI
    PROGRAM_NUMBER (SERVICE_ID), SERVICE_TYPE, PMT_ID, NETWORK_ID,
    TS_ID, FORMAT_ID, SOB_DEF_PID or the like
Default PID : In case of ARIB, small value of component tag
High priority is given to component group descriptor
SOB_DURATION
    SOB playback time
    (number of fields and total number of SOBU_ENTs belonging to default PID)
SOB_ES_Ns : The number of ESs
SOB_V_ES_Ns (number of SOB_V_ESIs) : The number of Video ESs
SOB_A_ES_Ns : The number of Audio ESs (number of SOB_A_ESIs)
    SOB_ES_Ns≧SOB_V_ES_Ns+SOB_A_ES_Ns
    SOB_V_ES_Ns+SOB_A_ES_Ns≧ES_TMAP_Ns
PCR_POS_COUNT : This indicates what PCR preceding the start of Packet_Group is to
    be referenced
PCR_POS_SHIFT : Index portion of 2 of LB indicating PCR packet position
CP_CTL_INFO : described later
MNF_ID_TBLN : PLN (Palette Number) contained in number TBL of NMF_ID_TBL used
    in this SOB is specified by packet group header
SOB_NUMBER : SOB number (Example 3 : refer to FIG. 36)

# FIG.9

EP 1 553 768 A2

Profile information

| Option support flag (8 bits) |
| Region number (16 bits) |

Option support flag : 00=only Base, 01=Base+Option1,
02=Base+Option2, 03=Base+Option1+Option2
Region number : 00=JPN (ARIB), 01=US (ATSC),
02=Europe (DVB) ..., 0xffff=universal

# F I G. 10

132231 — SOBI_GI

132232 — SOB_ESI#1

...

132232 — SOB_ESI#m

132233 — SOB_SMLI

132234 — SOB_AGAPI

132235 — SOB_TMAP

132236 — SOB_ES_GPI

SOB_V_ESI ~1322321

SOB_A_ESI ~1322322

SOB_OTHER_ESI ~1322323

SOB_ESI includes V_ESI, A_ESI, and OTHER_ESI

# F I G. 12

| SOB_PROFILE / AP_FORMAT1&2 | ARIB (ISDB-S&T) | DVB | ATSC-1 | ... |
|---|---|---|---|---|
| Mandatory SD | V : MPEG2 50/60Hz<br>A : MPEG2, AC3, LPCM : 48KHz | V : MPEG2 50/60Hz<br>A : MPEG2, AC3, LPCM : 48KHz | V : MPEG2 50/60Hz<br>A : MPEG2, AC3, LPCM : 48KHz | ... |
| Option 1 : HD compatible | V : MPEG2<br>1080i, 720p, 480i, 480p<br>: 50/60Hz<br>NEW Codec<br>A : MPEG2, AC3, LPCM : 96KHz<br>AAC : 48/96KHz | V : MPEG2<br>1152*1440i, 1080*1920 (I, p),<br>1035*1920, 720*1280,<br>(576, 480)*(720*, 544, 480,<br>352), (288, 240)*352 : 30Hz,<br>25Hz<br>NEW Codec<br>A : MPEG2, AC3, LPCM : 96KHz<br>DTS : 48, 49Hz<br>NEW codec | V : MPEG2<br>1080*1920 (i, p), 720*1280p,<br>480*704 (i, p), 480*640<br>(i, p) : 23.976Hz, 24Hz,<br>29.97Hz, 30Hz, 59.94Hz<br>NEW Codec<br>A : MPEG2, AC3 : 48KHz,<br>44.1kHz, 32kHz<br>MPEG : 48, 96KHz,<br>NEW codec | ... |
| Option 2 : TS compatible (digital broadcasting) | Conformance to broadcast scheme | Conformance to broadcast scheme | Conformance to broadcast scheme1 | ... |

F I G. 11

EP 1 553 768 A2

EP 1 553 768 A2

1322321 — | SOB_V_ESI |

| ES_TY | ~13223211 |
| ES_PID | ~13223212 |
| STREAM_TYPE | ~13223213 |
| COMPONENT_TAG | ~13223214 |
| STREAM_CONTENT | ~13223215 |
| COMPONENT_TYPE | ~13223216 |
| V_ATTR | ~13223217 |
| CP_CTL_INFO | ~13223218 |

ES_TY : Type of ES, 00=Video ES
ES_PID : PID of ES
STREAM_TYPE : Stream type indicated in PMT
COMPONENT_TAG : Value of COMPONENT_TAG indicated by
        component descriptor
STREAM_CONTENT : Value of STREAM_CONTENT indicated by
        component descriptor
COMPONENT_TYPE : Value of COMPONENT_TYPE indicated by
        component descriptor
V_ATTR : Attributes of Video

| b15    b10 | b9          b8 | b7 | b6 | b5          b2 | b1    b0 |
|---|---|---|---|---|---|
| Reserved (6bits) | Application Flag (2bits) | Line21 SW1 (1bit) | Line21 SW2 (1bit) | Horizontal resolution (4bits) | reserved (2bits) |

Application flag : 0=Aspect ratio is specified by this V_ATR
                01=Aspect ratio may be specified by this V_ATR
                Actual aspect ratio is recorded in stream
Horizontal resolution : 00=1920, 01=1440, 02=1280, 03=720, 04=544, 05=480

F I G. 13

1322322 ~ | SOB_A_ESI |

| ES_TY | ~132232201 |
| ES_PID | ~132232202 |
| STREAM_TYPE | ~132232203 |
| COMPONENT_TAG | ~132232204 |
| STREAM_CONTENT | ~132232205 |
| COMPONENT_TYPE | ~132232206 |
| SIMULCAST_GP_TAG | ~132232207 |
| AUDIO_ATTR | ~132232208 |
| LANG_CODE | ~132232209 |
| LANG_CODE2 | ~132232210 |
| CP_CTL_INFO | ~132232211 |

ES_TY : 0x40=AUDIO ES
SIMULCAST_GP_TAG : Deviation value of audio frame at the time of
  start during multi-broadcasting
LANG_CODE : First audio language code
LANG_CODE 2 : Second audio language code
AUDIO_ATTR : Attribute value of AUDIO

| Multi_lng (1 bit) | Main_Comp (1 bit) | Quality_Indicator (2 bits) | Sampling_Rate (3 bits) | Reserved (1 bit) |

Multi_lng : 1=DUAL mono, 0=others
Main_Comp : 1=main audio, 0=others
Quality_Indicator : This indicates audio quality display
Sampling_Rate : 011=24KHz, 101=32KHz, 111=48KHz

FIG. 14

EP 1 553 768 A2

1322323 ~ | SOB_OTHER_ESI |

| ES_TY | ~13223231 |
| ES_PID | ~13223232 |
| STREAM_TYPE | ~13223233 |
| COMPONENT_TAG | ~13223234 |
| DAT_COMP_ID | ~13223235 |
| AD_DAT_COMP_IFO | ~13223236 |
| CP_CTL_INFO | ~13223237 |

ES_TY : 0x80=Other ES
DAT_COMP_ID : Data encoding identification
AD_DAT_COMP_IFO : Additional information on identification

# FIG. 15

13223237

| CP_CTL_INFO |
| --- |

| CGMS (2 bits) | APS (2 bits) | EPN (1 bit) | ICT (1 bit) | Reserved (2 bits) | Retention (1 bit) | Retention_state (3 bits) | Reserved (4 bits) |
| --- | --- | --- | --- | --- | --- | --- | --- |

CGMS : 0=Inhibited, 1=Permitted unlimitedly
APS : 0=No APS, 1=APS type 1 is added, 2=APS type 2 is added, 3=APS type 3 is added
EPN : 0=Contents are protected (IE output is protected), 1=No contents are protected
ICT : 0=Resolution is limited, 1=No limit
Retention : 1=None, 0=Temporary storage time is effective
Retention_State : 0=No limit, 1=1 week, 2=2 days, 3=1 day, 4=12 hours, 5=6 hours, 6=3 hours, 7=1.5 hours

# F I G. 16

EP 1 553 768 A2

EP 1 553 768 A2

SOB_ADR_OFS
  LB address from the start of file to the start of SOB
SOB_SZ : The number of SOB Packet Groups
SOBU_PB_TM_RNG
  SOBU playback time range
  1=0.4s~1.2s, 2=1s~2s, 3=2s~3s
SOB_S_PKT_POS
  Start in Packet group at the start of SOB
  1≦SOB_S_PKT_POS≦85
SOB_E_PKT_POS
  End in Packet group at the start of SOB
  1≦SOB_E_PKT_POS≦85

ES_S_ADR_OFS
  The number of Packet Groups from the start of SOB to the start of this ES
ES_E_ADR_OFS
  The number of Packet Groups from the end of this ES to the end of SOB
ES_S_PTM : PTM at the start of this ES
ES_E_PTM : PTM at the end of this ES
LAST_SOBU_E_PKT_POS
  Last TS packet group number of the last ESOBU in packet group
STMAP_SRPN : STMAP search number.  Number from the start of the inside of STMAP file
  This may not be present in case where STMAP file is divided for each STR_FI and in case
  where the STMAP files are recorded in order of recording of STMAP.IFO

| | |
|---|---|
| 132235~ | SOB_TMAPI |

| | |
|---|---|
| 1322351~ | SOB_TMAPI_GI |
| 1322352~ | ES_TMAPI#1 |
| | ... |
| 1322352~ | ES_TMAPI#1 |

| | |
|---|---|
| SOB_ADR_OFS | ~13223511 |
| SOB_SZ | ~13223512 |
| SOB_PB_TM_RNG | ~13223513 |
| SOB_S_PKT_POS | ~13223514 |
| SOB_E_PKT_POS | ~13223515 |
| ES_TMAP_Ns | ~13223516 |

| | |
|---|---|
| ES_PID | ~132235211 |
| ES_S_PTM | ~132235212 |
| ES_E_PTM | ~132235213 |
| ES_E_ADR_OFS | ~132235214 |
| LAST_SOBU_E_PKT_POS | ~132235215 |
| ES_SOBU_ENT_Ns | ~132235216 |
| ES_E_ADR_OFS | ~132235217 |
| STMAP_SRPN | ~132235218 |

F I G. 17

If SOBU equal to or shorter than 0.4 seconds is disabled, there is a
possibility that SOBU start and GOP start are not matched for a long time
(Processing moves while a shift for "A" occurs)

A

| SOBU=0.5 | SOBU=0.5 | SOBU=1 | SOBU=1 | SOBU |

GOP
0.5s

GOP
0.5s

← GOP=1s →

← GOP →
1.2s

SOBU equal to or shorter than 0.4 is enabled
Limitation: One frame or more
To be cut in units of frames, fields, or pictures
In case where no I-picture exists: 1ST_REF_SZ=ALL 0x00

# FIG. 18

EP 1 553 768 A2

| | |
|---|---|
| 13801~ MVOB_TMAP | MVOBU_ENT Ns ~1380111 |
| | TM_OFS ~1380112 |
| | ADR_OFS ~1380113 |
| 138011~ MVOB_TMAPI_GI | VOBU_PB_TM_RNG ~1380114 |
| | VTMAP_N ~1380115 |

ADR_OFS
  Packet Group number (LB address) from the start of file to the start of SOB
VOBU_PB_TM_RNG
  SOBU playback time range
  1=0.4s~1.2s, 2=1s~2s, 3=2s~3s

VMAP_N : VTMAP number
VTMAP number from the start of the inside of VTMAP file (VTMAP)
This number may not be assigned in case where determination is made in
order of recording of VTMAP.IFO

F I G. 19

Note: TMAP_SRP does not always specify TMAP in ascending order
Dummy data may exist between TMAPs

F I G. 20

| VTMAPTI |
| --- |
| VTMAP_SRP #1 |
| ... |
| VTMAP_SRP #q |
| VTMAPI |
| ... |
| VTMAPI |

| VMG_ID |
| --- |
| VTMAPT_EA |
| VERN |
| VTMAP_LAST_MOD_TM |
| VTMAP_SRP_Ns |

| VMAP_SA |
| --- |
| VOBU_ENT_Ns |

| VOBU_ENT #1 |
| --- |
| ... |
| VOBU_ENT #q |

VMG_ID : VMG identification information
VTMAPT_EA : End address of this file
VERN : TMAP version information
VTMAP_LAST_MOD_TM : Update Date and time of VTMAPT are described
(The same value as HR_MANGR.IFO)
VMAP_SA : Start address of each VMAP
VOBU_ENT_Ns : The number of VOBU_ENTs

# F I G. 21

In case of VOBU_ENT

| VOBU_ENT #1 |
| --- |
| ... |
| VOBU_ENT #q |

| 1STREF_SZ | ~13703311 |
| --- | --- |
| VOBU_PB_TM (number of fields) | ~13703312 |
| VOBU_SZ | ~13703313 |

# F I G. 22

| STMAPTI |
| --- |
| STMAP_SRP #1 |
| ... |
| STMAP_SRP #q |
| STMAPI |
| ... |
| STMAPI |

| VMG_ID |
| --- |
| STMAPT_EA |
| VERN |
| STMAP_LAST_MOD_TM |
| STMAP_SRP_Ns |

| STMAP_SRP_GI |
| --- |
| ES_TMAPI_GI #1 |
| ... |
| ES_TMAPI_GI #q |

| STMAP_SA |
| --- |
| ES_TMAPI_Ns |

| ES_TMAP #1 |
| --- |
| ... |
| ES_TMAP #q |

VMG_ID : VMG identification information
STMAPT_EA : End address of this file
VERN : TMAP version information
STMAP_LAST_MOD_TM : Update Date and time of STMAPT are described
(The same value as HR_MANGR.IFO)
SMAP_SA : Start address of each SMAP
ES_TMAPI_Ns : The number of ES_TMAPIs

# F I G. 23

| STMAP_SRP_GI | |
| --- | --- |
| ES_TMAPI_GI #1 | SOBU_ENT_Ns |
| ... | |
| ES_TMAPI_GI #q | |

| ES_TMAP #1 | SOBU_ENT #1 |
| --- | --- |
| ... | ... |
| ES_TMAP #q | SOBU_ENT #q |

ES_ADR_S_OFS
The number of Packet Groups from the start of SOB to the start of this ES
ES_ADR_E_OFS
The number of Packet Groups from the end of this ES to the end of SOB
ES_S_PTM : PTM when this ES is started
ES_E_PTM : PTM when this ES is ended
LAST_SOBU_E_PKT_POS
Last TS packet group number of last ESOBU in packet group
T_MAP_N : TMAP number
Number from the start of TMAP file (T_MAP_T)

# F I G. 24

In case of SOBU_ENT

| SOBU_ENT #1 |
| --- |
| ... |
| SOBU_ENT #q |

| | |
| --- | --- |
| 1st_Ref_PIC_SZ | ~13703301 |
| SOBU_PB_TM (number of fields) | ~13703302 |
| SOBU_SZ (number of Packet GPs) | ~13703303 |
| SOBU_S_PKT_POS | ~13703304 |
| PCR_POS | ~13703305 |

# F I G. 25

(1) When video data is available

SOBU : (i) To be delimited at random access enable position

(ii) All SOBUs other than last SOBU are delimited by minimum VOBU/SOBU_PB_TM_RNG=0 : 0.4 seconds, =1 : 1 second,=2 : 2 seconds or more

(iii) To be delimited by maximum VOBU/SOBU_PB_TM_RNG=0 : 1 second,=1 : 2 seconds, and=2 : 3 seconds

SOBU_SZ : The number of packet groups belonging to SOBU

(the first fraction is counted, and the subsequent fractions are not counted.)

SOBU_S_PKT_POS : First packet number (1-85) in packet group of SOBU to be started

Example

78-th packet

SOBU n

SOBU_SZ=3,
SOBU_START=78

| ...... | PKTGP | PKTGP | PKTGP | PKTGP | ...... |

1st_ref_PIC_SZ : In case of the number of LBs from the start of ESOBU to the end of Ref_PIC=0x000000f, Ref_PIC does not exist in SOBU. Further, in that case, SOBU playback time may be shorter than 0.4 seconds (to be set in units of frames or fields)

PCR_POS : The position of PCR at position indicated by PCR_POS_COUNT. It is expressed by 0x0000 in case where the number of addresses from the start of SOBU does not exist. The number of LBs is expressed by PCR_POS×2^PCR_POS_SHIFT

(2) When video data is unavailable and audio data is available

SOBU : To be delimited at intervals equal to the maximum of (i)

1st_Ref_PIC_SZ=The number of last TS packets of audio frame at the start of SOBU

PCR_POS : Same as (i)

(3) When video data and audio data are unavailable and data broadcasting is available

SOBU : To be delimited at intervals equal to the maximum of (i)

1st_Ref_PIC_SZs=fixed to 0xffffffff

PCR_POS=fixed to 0xffffffff

F I G. 26

EP 1 553 768 A2

Multi-View Program

Main Group (V1, A1)

Sub1 Group (V2, A2)

Sub2 Group (V3, A3)

F I G. 27

F I G. 28

Value entered in SOB_TMAPI_GI : ADR_OFS, SOB_S_PKT_POS, SOB_E_PKT_POS
Value entered in TMAPI : ES_S_ADR_OFS, SOBU_SZ, ES_SOBU_S_PKTES_LAST_SOBU_PKT_POS, ES_E_ADR_OFS
PG : Packet Group=16Logical Blocks
PKT : Packet
Other values
SOB_TMAPI_GI : ES_TMAP_Ns=3, ES_TMAPI #1 : GI ES_PID=V1, ES_TMAPI #2 : GI ES_PID=V2, ES_TMAPI #3 : GI ES_PID=V3

EP 1 553 768 A2

132231 — SOBI_GI

132232 — SOB_ESI#1

...

132232 — SOB_ESI#m

132233 — SOB_SMLI

132234 — SOB_AGAPI

132235 — SOB_TMAP

F I G. 29    132236 — SOB_ES_GPI

SOB_ES_GPI_GI — 1322361

GPI_SRP#1 — 1322362

...

GPI_SRP#m — 1322362

GPI#1 — 1322363

...

GPI#m — 1322363

1322361 — SOB_ES_GPI_GI

1322362 — GPI_SRP#1

...

1322362 — GPI_SRP#m

1322363 — GPI#1

...

1322363 — GPI#m

F I G. 30

GPI_SRP_Ns — 13223611

The number of GPI_SRPs

GPI_SA — 13223612

Start address of ES_GPI

GPI_GI — 13223631

ES_PID#1 — 13223632

...

ES_PID#m — 13223632

13223631 — GPI_GI

13223632 — ES_PID#1

...

13223632 — ES_PID#m

Block_TY — 132236311

GP_TY — 132236312

BLOCK_NUMBER — 132236313

ES_PID_Ns — 132236314

ES_PID_Ns : The number of ES PIDs belonging to this GP
BLOCK_TY : 1=Multi-View, 2=Multi-channel_video, 3=Rain Attenuation
GP_TY : 01=Main Group, 02=Sub Group, 03=Others
BLOCK_NUMBER : Block number
    GP having the same number can be switched by angle button or the like

F I G. 31

Multi View Broadcasting

X
angle 1 (MAIN)
Rain attenuation (MAIN)
V1, A1

Rain attenuation

U
Rain attenuation (SUB)
V4, A1

Y
angle 2 (SUB)
V2, A2

Z
angle 3 (SUB)
V3, A3

X, Y, Z is changeable by Angle Key
X, U is changeable by Rain-ATT Key

FIG. 32

Block

Multi View Broadcasting
BLOCK_NUM=1

Block

X
angle 1 (MAIN)
Rain attenuation (MAIN)
V1, A1

Rain attenuation
BLOCK_NUM=2

U
Rain attenuation (SUB)
V4, A1

Y
angle 2 (SUB)
V2, A2

Z
angle 3 (SUB)
V3, A3

GPI_SRP_Ns=5
X1 : BLOCK_TY=1, GP_TY=1, BLOCK_NUM=1 (for Multi View)
X2 : BLOCK_TY=3, GP_TY=1, BLOCK_NUM=2 (for Rain attenuation)
Y : BLOCK_TY=1, GP_TY=2, BLOCK_NUM=1
Z : BLOCK_TY=1, GP_TY=2, BLOCK_NUM=1
U : BLOCK_TY=3, GP_TY=2, BLOCK_NUM=2

F I G. 33

130 — HDVR_VMG

- 1310 — HDVR_VMGI
- 1380 — M_AVFIT
- 1320 — STR_FIT
- 1330 — Program chain information (EX_PGCI)
- 1340 — Play list information (EX_PL)
- 1350 — EX_TXTD_MG
- 1360 — EX_MNFIT
- 1370 — TMAPT (Ex. 2)

1331 — ORG_EX_PGC information

- 1341 — UD_EX_PGCT information
- 1342 — UD_EX_PGC search pointer #1
- ...
- 1342 — UD_EX_PGC search pointer #r
- 1343 — UD_EX_PGC information #1
- ...
- 1343 — UD_EX_PGC information #s

F I G. 34

EX_PGC infor

| | |
|---|---|
| 1335 → | EX_PGC_GI |
| 1332 → | EX_PGI #1 |
| | ... |
| 1332 → | EX_PGI #p |
| 1333 → | EX_CELL_SRP #1 |
| | ... |
| 1333 → | EX_CELL_SRP #q |
| 1334 → | EX_CI #1 |
| | ... |
| 1334 → | EX_CI #q |

| | |
|---|---|
| Number of programs | ~13351 |
| Number of CELL_SRPs | ~13352 |

| | |
|---|---|
| Program type | ~13321 |
| Number of cells in program | ~13322 |
| PRIM_TXT information | ~13323 |
| IT_TXT_SRP number | ~13324 |
| Representative_PIC information | ~13325 |
| Editor ID | ~13326 |
| Program index number (PG absolute number) | ~13327 |
| Program update date | ~13328 |
| MNFI number | ~13329 |

| | |
|---|---|
| Cell type | ~13341 |
| STR_FI number | ~13342 |
| Corresponding SOB number | ~13343 |
| ID to be referred to | ~13344 |
| GPI number to be reffered to | ~13345 |
| C_EPI_Ns | ~13346 |
| Cell start PTS/ATS | ~13347 |
| Cell end PTS/ATS | ~13348 |
| C_EPI | ~13349 |

Program update date
    Date/time at a time when program
    management information is updated is sotred
MNFI number
MNFI search pointer number
    Corresponding SOB number
        The numbering will be described later
      Example 1 : Serial SOB number
      Example 2 : STR_FN, SOB number
             STR_FN = STR_F1 number
      Example 3 : SOB number (included in SOBI)

# F I G. 35

| | Example 1 | | Example 2 | | Example 3 |
|---|---|---|---|---|---|
| SOB1<br>...<br>SOBn | SOB FILE1<br>(STR_FN=1) | STR_FN=1<br>SOB1<br>...<br>SOBn | SOB FILE1<br>(STR_FN=1) | SOB1<br>...<br>SOBn | SOB FILE1<br>(STR_FN=1) |
| SOBn+1<br>...<br>SOBm | SOB FILE2<br>(STR_FN=2) | STR_FN=2<br>SOB1<br>...<br>SOBm | SOB FILE2<br>(STR_FN=2) | SOB2<br>...<br>SOBm | SOB FILE2<br>(STR_FN=2) |
| SOBm+1<br>... | SOB FILE3<br>(STR_FN=3) | STR_FN=3<br>SOB1<br>... | SOB FILE3<br>(STR_FN=3) | SOB500<br>... | SOB FILE3<br>(STR_FN=3) |

Example 1 : Serial numbers are assigned in order of STR_FI
SOB number is required for CELLI compatible SOB number information
Advantage : Management information is reduced in amount
Disadvantage : If SOB is added, reassignment of each SOB number is
required
Example 2 : To be expressed by STRFN and SOB number
STR_FN and SOB number are required for CELLI compatible SOB
number information
Advantage : Understandable
Example 3 : SOB numbers are freely assigned
SOB number is required for SOB_GI and SOB number is required for
CELLI compatible SOB number information
Advantage : SOB number is easily added

# F I G. 36

F I G. 37

151 — | Sync Pattern : Sync pattern=00FFA5A5 |
152 — | DCI_CCI |
153 — | MNI |

| DCI_CCI_SS : Validity of DCI and CCI |
| DCI # 1-32 : Display control information |
| CCI # 1-32 : Copy management information |

DCI_CCI_SS : 4 Bytes ⋯ | DCI_SS (1 bit) | Reserved (3 bits) | CCI_SS (4 bits) |

DCI_SS : 0=Invalid, 1=Valid
CCI_SS : 0-th bit : APS is valid or invalid, 1st bit : EPN and ICT are valid or invalid, 2nd bit : CGMS is valid or invalid
   3rd bit : Retention is valid or invalid

DCI (display control information) : 1 bit×32=4 bytes
This information is set by 32 streams for each ES, and 0 is filled if the information is absent

| ES1 aspect (1 bit) | ⋯ | ES32 aspect (1 bit) | ⋯ Aspect : 0=4:3, 1=16:9

F I G. 38

EP 1 553 768 A2

CCI

Example 1

| CGMS (2 bits) | APS (2 bits) | EPN (1 bit) | ICT (1 bit) | Reserved (2 bits) |
|---|---|---|---|---|

Example 2

| CGMS (2 bits) | APS (2 bits) | EPN (1 bit) | ICT (1 bit) | Reserved (2 bits) | Retention (1 bit) | Retention_state (3 bits) | Reserved (4 bits) |
|---|---|---|---|---|---|---|---|

FIG. 39

| | |
|---|---|
| 151 ~ Sync Pattern : Sync pattern=00FFA5A5 | |
| 152 ~ DCI_CCI | |
| 153 ~ MNI | |

| | |
|---|---|
| MNF_ID_Data | ~1531 |
| MNF_DATA | ~1532 |

MNF_ID_DATA

| MNF_SS (1 bit) | MNF_ID_N (3 bits) | Reserved (4 bits) |
|---|---|---|

NMF_SS : 0=MMF_DATA does not exist.   1=MNF_DATA exists
MNF_ID_N : Number in MNF_ID_TBL used in this SOB

MNF_DATA : User data area freely used by each company

# F I G. 40

EP 1 553 768 A2

EP 1 553 768 A2

FIG. 41

Usual process

ST10
Initialize

ST12
Display setting

ST14
Key input

ST16
Interpret input key

Process in parallel
for each task

ST20
Program
setting process

Recording process

ST22 ST24
Playback
process

ST26
Digital output
process to STB

ST28
Edition
process

F I G. 42

ST28

Edition process

ST280

EP edition      Edition contents      Play list preparation process

Copy, move      Deletion process

ST282A
EP edition process

ST282B
Copying / moving process

ST282C
Deletion process

ST282D
Play list preparation process

ST284
Set program update date (EX_PGI, EX_IT_TXT, EX_MNFI) (set one of EX_PGI, EX_CI, VOB and SOB at updating time)

Return

F I G. 43

Initial setting — ST10

ST40

Disk check — Error → Return (error)

Normal

ST42

Is VGM present ? — NO → Return

YES

Read VMG — ST44

Read profile information (Example 1) — ST46

ST48

Is region matched ? — NO → "Region code is invalid. Contents which cannot be played back are present" — ST50

YES

Set operation in accordance with operation chart — ST52

Return

F I G. 44

| STR_F / Play operation | Only Base | Base+OP1 | Base+OP2 | Base+OP1+OP2 |
|---|---|---|---|---|
| Only Base | ○ | △ (Only Base) | △ (Only Base) | △ (Only Base) |
| Base+OP1 | ○ | ○ | △ (Only Base) | △ (base+OP1) |
| Base+OP2 | ○ | △ (Only Base) | ○ | △ (base+OP2) |
| Base+OP1+OP2 | ○ | ○ | ○ | ○ |

F I G. 45

EP 1 553 768 A2

Video

Audio

┌─────────────────────┐
│ ┌─────────────────┐ │
│ │ MPEG2 PS (SD)   │ │
│ └─────────────────┘ │
└─────────────────────┘

┌─────────────────┐
│ Advanced Video  │
│ Codec (SD)      │
└─────────────────┘

┌──────────────────────────────────────┐
│ ┌──────────────────────────────────┐ │
│ │ MPEG, AC-3, LPCM 48kHz           │ │
│ └──────────────────────────────────┘ │
└──────────────────────────────────────┘

┌─────────────────┐
│ MPEG2 PS (HD)   │
└─────────────────┘

┌─────────────────┐
│ Advanced Video  │
│ Codec (HD)      │
└─────────────────┘

┌──────────────────────────────────────┐
│ MPEG-2 AAC                            │
│ 48kHz, 32kHz (44.1kHz)               │
└──────────────────────────────────────┘

┌──────────────────────────────────────┐
│ Advanced Audio Codec                  │
│ 48kHz, 32kHz (44.1kHz)               │
└──────────────────────────────────────┘

┌─────────────────────────────────┐   ┌──────────────────────────────┐
│ Digital broadcast (MPEG TS SD, HD) │   │ MPEG-2 AAC (TS)              │
└─────────────────────────────────┘   │ 48kHz, 32kHz (44.1kHz)       │
                                       └──────────────────────────────┘

⬭ : Base     ⌐ ¬ : Option 2     Other : Option 1

FIG. 46

Video                                                Audio

| MPEG2 PS (SD) | Advanced Video Codec (SD) | MPEG, AC-3, LPCM 48kHz |

| MPEG2 PS (HD) | Advanced Video Codec (HD) | MPEG-2 AAC 48kHz, 32kHz (44.1kHz) |

Advanced Audio Codec
48kHz, 32kHz (44.1kHz)

| Digital broadcast (MPEG TS SD, HD) | MPEG-2 AAC (TS) 48kHz, 32kHz (44.1kHz) |

⬭ : Base     ( ⬚ ) : Option 2     Other : Option 1

FIG. 47

EP 1 553 768 A2

Video                                                    Audio

| MPEG2 PS (SD) | Advanced Video Codec (SD) | MPEG, AC-3, LPCM 48kHz |

| MPEG2 PS (HD) | Advanced Video Codec (HD) | MPEG-2 AAC 48kHz, 32kHz (44.1kHz) |

Advanced Audio Codec
48kHz, 32kHz (44.1kHz)

| Digital broadcast (MPEG TS SD, HD) | MPEG-2 AAC (TS) 48kHz, 32kHz (44.1kHz) |

( ) : Base   ( ) : Option 2   Other : Option 1

FIG. 48

EP 1 553 768 A2

Recording process — ST22

ST100
Load file system data

ST102
Free space ? — NO — ST104
"No recording space available"

YES

ST105
Recording pre-process : write respective management areas (generate VMG), etc.

ST106
Digital broadcast ? — NO

End

To VR recording process

YES

ST111
Error ? — YES

NO

ST112
Recording initial setup
Reset STC unit
Set write start address and write command in drive unit
Initialize formatter unit
Set CELL, SOBU, PG and PGC division (align process setup), etc.

ST114
Recording start setup : Set data fetch start process from buffer in formatter unit

ST116
Extract PAT from buffer, and determine PMT based on user's program setup
Extract PMT for stream to be recorded, and save PAT, PMT, NIT in work RAM as management information

ST118
Set profile information based on TS configuration

①

F I G. 49

①

ST120
Generate ESI for number of streams from PMT

ST130
Buffer fetch process

ST140
Recording data stored in buffer memory=predetermined size ? — NO

YES ST142
Determine write address and write length of drive unit, and issue write command

ST144
Segmentation information fetch interrupt generated ? — NO

YES ST146
Fetch segmentation information from formatter unit

ST148
NO — Recording end key input ?

YES

ST150
Recording end process
Feth remaining segmentation information from formatter unit & initialization
Write in management area
Write in VMG (prepare PGCI and STR_FIT : segmentation information, I-PIC information, etc.)
Set stream presentation time of default PID to SOB_DURATION

FIG. 50

End

FIG. 51

ST1330

CPI preparation process

ST13300

Take out previous CPI (CPI must be same as SOBI in case of first SOB)

ST113302

Is digital copy control descriptor or contents utilizing descriptor present in packet group ?

NO

YES

ST13306

Take out each descriptor

ST13304

Set same value as previously

ST13308

Terminate packet grouping before CPI changes (insert dummy packet in dummy TS packet); prepare next packet group, set changed packet at the beginning, and prepare CCI based on taken out information

Return

F I G. 52

EP 1 553 768 A2

1 packet group : 8 packs

| Same CPI in ES | Dummy data | Next packet group (value of new CPI) |
|---|---|---|

Packet data area

| ALL 00 : 188 bytes | ··· | ALL 00 : 188 bytes |
|---|---|---|

PAT area (in packet group)

| PAT = 01 | ··· | PAT = 01 |
|---|---|---|

F I G. 53

ST1331

MNFI preparation process

ST13310

Does MNF_DATA
to be stored in this packet
group exist ?

NO

YES

ST13312

MNF_SS←1

ST13314

Set 0 to MNF_SS,
MNF_ID_N, MNDF_DATA

ST13316

Capture ID number described in
NMF_ID to be used from ID contained
in MNF_TBL specified by SOB, and
set the captured ID number in
MNF_ID_N

ST13318

Set MNF_DATA to be set

Return

F I G. 54

Preprocessing of recording ─ ST105

ST1100
Does DVD_HDR directory exist ?
— NO → Prepare DVD_HDR directory ─ ST1102

YES

ST1104
Has error occurred ?
— YES →

"Error has occurred in file system" ─ ST1106

Return (error)

NO

ST1108
Does VMG exist ?
— YES → Read VMG ─ ST1110

NO

Prepare VMG
Detect data form from tuner, set the detected data format to VMGI or the like, set OPTION flag of recorder, and set region code (default value by recorder) ─ ST1126

Recording operation setting process ─ ST6070

ST1120A
Is digital broadcast recording carried out ?
— YES →

ST1120B
Is digital broadcast recording carried out ?
— NO →

NO

ST1130A
Add object at the back of recorded VOB file or newly create VOB file if no VOB file exits

Return (OK)

YES

ST1123
Is region matched ?
— NO →

YES

ST1130B
Add object at the back of recorded SOB file or newly create file if no SOB file exists

ST1125
"Region code is invalid. Recording cannot be carried out"

Return (error)

ST1135
Can stream be analyzed ?

YES

Set 0 in ESOB_COG/NON_COG ─ ST1136

NO

Set "1" in ESOB_COG/NON_COG ─ ST1137

Return (OK)

F I G. 55

ESI preparation process ~ST120

ST1201

Check stream type in PSI, SI

ST1203

Stream type = component descriptor, audio component descriptor, or data encoding system descriptor ?

Video stream

Other stream

Audio stream

ST1213A

Prepare new ESI indicating ES_TY = 0 based on read data

ST1215A

Prepare new ESI indicating ES_TY = 0x40 based on read data

ST1217A

Prepare new ESI indicating ES_TY = 0x80 based on read data

ST1213B

Fetch resolution data, aspect ratio, etc., and set them to V_ATR
Set ES_PID, STREAM_TYPE, COMPONENT_TAG to PMT
Set STREAM_COMPONENT, COMPONENT_TYPE based on component descriptor

ST1217B

Set ES_PID, STREAM_TYPE, COMPONENT_TAG to PMT
Set DATA_COMP_ID, AD_DAT_COMP_INFO based on data encoding descriptor

ST1215B

Fetch sampling frequency, channel number, etc., and set them to A_ATR
Set ES_PID, STREAM_TYPE, COMPONENT_TAG to PMT
Set STREAM_COMPONENT, COMPONENT_TYPE, SIMULCAST_GP_TAG,
LANG_CDE based on audio component descriptor

ST1220

Prepare CP_CTL_INFO from CP information belonging to component tag corresponding to ES

ST1230

Is there any other ES whose ESI has not been prepared ?

YES

NO

Return

F I G. 56

STR_FI preparation process —ST150

—ST1500
Increase the number of SOBI_SRPs by 1
PKT_TY=0x00 (MPEG_TS)

—ST1502
Set recording start time in SOB_REC_
TM, SOB_REC_TM_SUB and take out
SOB_S_PTM, SOB_E_PTM from the
inside of stream and set it

—ST1504
Set PCR_POS_SHIFT according to rate

—ST1506
Does
TS stream occur
?
YES / NO

—ST1510
PKT_GRP_SZ←Recording format
compatible value
PKT_Ns←Recording format
compatible value
country_code←Device country code

—ST1508
PKT_GRP_SZ←16, PKT_Ns←0xAA
country_code←Device country code

—ST1514
Set AP_FORMAT_1←1 (ISDB) in case
of ARIB, set AP_FORMAT_1←2 in
case of ATSC, set AP_FORMAT_1←3
in case of DVB, read out PAT set at
the start of recording from work
memory, set TS_ID, NETWORK_PID,
PMT_ID (PID of PMT used in this
ESOB), and set SOB number
(Example 3)

—ST1516
Read out PMT, NIT, EIT set at the
start of recording from work memory,
set Program_number (SERVICE_ID in
PMT), Format_Id, version (value of
REG_DES), PCR_PID, ESOB_ES_Ns,
V_ES_Ns, A_ES_Ns, set PID of the
smallest DEF_PID component tag value
(however, high priority is given to value
set by component group descriptor),
set profile information in accordance
with recorder default value (Example 2)

—ST1530
GPI setting process

—ST1540
TMAP setting process

—ST1545
MNF_ID_TBL setting process

—ST1550
Set LB address starting recording in
ADR_OFS, and prepare CP_CTL_IFO
from whole CP information in PMT

—ST1554
Set edit date and time

Return

Note : Default PID is PID whose
component tag value is of 0x00
However, in case of multi-view TV,
this default PID is PID set at
component value indicated by main group

F I G. 57

GPI setting process ST1530

ST15300A

Has a plurality of programs (broadcasts) set to 1 TS ?

YES

NO

ST15302A

Set Es_Tmap_Ns so as to prepare TMAPI for each program, set GPI available in SOB_TY, set the number of programs in GPI_SRP_Ns, set 03 in GPI_TY, set all as main, set value different depending on program in BLOCK_NUMBER, and prepare GPI for each program (set the number of PIDs and value of each PID)

ST15304A

Does rainfall compatible broadcasting occur ?

YES

NO

ST15306A

Determine ES for preparing TMAPI by hierarchical transmission descriptor contained in EIT, set Es_Tmap_Ns, set GPI available in SOB_TY, register low hierarchy as another GP, set 2 in GPI_SRP_Ns, set 02 in GPI_TY, set high hierarchy as main, set others as subsidiary, set value identical to those of low hierarchy and high hierarchy in BLOCK_NUMBER, and prepare GPI for each hierarchy (set the number of PIDs and value of each PID : audio PID is common)

ST15308A

Does multi-view broadcasting occur ?

NO

YES

ST15312A

Set Es_Tmap_Ns to 1 and set no GPI in SOB_TY

ST15310A

Determine ES for preparing TMAPI by component group descriptor contained in EIT, set Es_Tmap_Ns, set GPI available in SOB_TY, register each group as another GP, set the number of groups in GPL_SRP_Ns, set 01 in GPL_TY, MAIN group as main, and others as subsidiary, set identical value in groups in BLOCK_NUMBER, and prepare GPI for each group (set the number of PIDs and value of each PID)

ST15314A

YES

Does other GP occur ?

NO

Return

F I G. 58

TMAP setting process — ST1540

ST15400
VOB/SOB structure setting process

ST15402
SOB: Determine the number of TMAPs in consideration of GP numbers or the like, set PID of ES to be prepared for each TMAP
VOB: Add 1TMAP

ST15404
Check delimitation information and set SOB start PTM, end PTM, start PTM, end PTM, and SOBU-ENTRY numbers for each TMAP, and TMAP number to be registered in TMAP

ST15406
Add TMAP to be prepared in TMAPT and prepare Entry information based on delimitation information (to be allocated in another file in case of Example 1 and to be allocated at the back of IFO in case of Example 2)

ST15408
Set update date and time information on edited TMAP in STMAP/VTMAP_LAST_MOD_TM

Return

F I G. 59

MNF_ID_TBL setting process ST1545

Check whether or not there exists MNF_ID_TBL in which MNF_ID of manufacturer of this device is present ST15450

Does TBL whose MNF_ID is present exist ? ST15452

YES → Set existing TBL number in SOBI ST15454

NO

Does dummy TBL exist ? ST15456

YES → Set manufacturer ID of this device in dummy ID region ST15460

NO

Add TBL for which this ID has been newly set, and increment MNF_ID_TBL_Ns ST15458

Set preset TBL number in SOBI ST15462

Return

F I G. 60

VOB/SOB structure
setting process ⌐ST15400

Check recording time ⌐ST154000

ST154001

2 hours or less ◄— How long is
recording time ? —► 4 hours or more

4 hours or less

ST154002

VOB/SOBU_PB_TM_RNG=0
Prepare VOBU/SOBU_
ENTRY so that each
SOBU is between 0.4
seconds and 1.0 second
based on delimitation
information

ST154003

VOB/SOBU_PB_TM_RNG=1
Prepare VOBU/SOBU_
ENTRY so that each
SOBU is between 1.0
seconds and 2.0 seconds
based on delimitation
information

ST154004

VOB/SOBU_PB_TM_RNG=2
Prepare VOBU/SOBU_
ENTRY so that each
SOBU is between 2.0
seconds and 3.0 seconds
based on delimitation
information

Return

F I G. 61

CP_CTL_INFO preparation process — ST1220

ST12200
Is there digital copy control descriptor in SI, PSI ?

NO → ST12202
Set copy free to CCI, and set non-APS to APS

YES — ST12204
Fetch digital copy control descriptor

ST12206
Keep CCI in "copy free", "copy protect", and change CCI from copy once to "copy protect"
Set analog copy control value to APS

ST12208
Is there contents use descriptor in SI, PSI ?

NO → ST12210
Set ICT (resolution control),
EPN (internet output limitation)
ICT : set permission,
EPN : set 0 (prohibition or encryption), because internet output is prohibited in digital broadcasting
Set Retention = 0,
Retention_state = 00

YES

ST12212
Fetch contents use descriptor

ST12214
Set ICT (resolution control),
EPN (internet output limitation)
ICT: set SI, PSI values
EPN : set 0 (prohibition or encryption), because internet output is prohibited in digital broadcasting
Set value of Retention (1 : temporary storage prohibition, 0 : permission)
Set 1.5 hours (0x7) to Retention_state in terrestrial digital case, set value set to contents use descriptor in the case of BS

Return

F I G. 62

ST150

( PGC preparation process )

ST1600

First recording in this disk ?

YES

ST1602

Set new ORG_PGC

NO

ST1604

Set to add PG after certain recorded PGC

ST1700X

PG_TY = 0
Cell_Ns = number of CELLs
Set 0x12 to CHR of VMGI_MAT when "jpn" is set to Language code of short type event descriptor in EIT
Second area of PRM_TXTI = problem name : set event_name of short type event descriptor in EIT.
Set REP _PICTI.
Set program update date / time
(PGI, IT_TXT, MNFI)

ST1702X

Set maker ID of apparatus to LAST_MNF_ID (set one of PGI, CI, VOB at change time)
Set absolute number of PG to PG_INDEX

ST1704X

Set 2(streamer (SOB)) to CELL_TY
Set ESFI number to be referred
Set number of ESOB to be referred
Set representative (video)
PID / Component_Group_Id as ID to be played back
Set playback start PTM, end PTM

( Return )

F I G. 63

```
                    ┌──────────┐
                    │  Start   │
                    └────┬─────┘
                         │            ST207
            ┌────────────▼────────────┐
            │       Read VMG          │
            └────────────┬────────────┘
                         │            ST208
```

Start

ST207 — Read VMG

ST208 — User determines PG whose playback is to be started (ORC_PGC, UD_PGC#1, UD_PGC#2)

ST209A — Read out profile information from work memory

ST209B — Compare region code with recorder default value

( = )

( ≠ )

ST209C — "Digital broadcasting region is invalid. Digital broadcasting data cannot be played back."

ST209D — Disable SOB playback

ST209E — Set playback mode in accordance with operation chart

ST212 — Determine VOB/SOB to be played back based on CELLI

ST213 — Does SOB exist ?  NO / YES

ST215 — Is SOB playback disabled ?  YES / NO

ST217 — Decoder setting process

ST220 — Process during cell playback

ST230 — End of playback ?  NO / YES

ST232 — Set next cell based on PGCI

ST240 — Error ?  YES / NO

ST242 — "Readout error"

ST244 — Playback end process

Return error

ST246 — Process during other playback end

End

FIG. 64

## FIG. 65

**Decoder setting process** — ST217

ST2170
**Does SOB exist?**

— NO → **Read STI and ESI of VOB/SOB to be played back** — ST2172

— YES → **Determine ES to be played based on GPI and PMT** — ST2171

ST2173
**Does video ES to be played back support playback?**

— NO → **Set demultiplexer so as not to play back video and set display mute in video decoder** — ST2175

— YES → **Set PID of video to be played back in demultiplexer and set initial value in video decoder** — ST2174

ST2176
**Does audio ES to be played back support playback?**

— NO → **Set demultiplexer so as not to play back audio and set mute in audio decoder** — ST2178

— YES → **Set PID of audio to be played back in demultiplexer and set initial value in audio decoder** — ST2177

ST2179
Set APS based on CCI (set APSON/OFF), APS type and the like in video decoder, and set CGMSA based on CCI by digital copy control by using video decoder
In case where digital output (such as 1394 or internet) is available, an output IC is set to 0 : scramble on or output disable or 1 : output IC to output as is, based on value of EPN. In case where ICT is 0, apply limitation to image resolution, and convert HD to SD
In case where ICT is 1, set output IC to output as is

**Return**

Process at cell playback time — ST220

ST2200

Determine ESI, SOB file to be played back from CI, determine group to be played back from PID and ES_GI to be played back in CI, and fetch and set PID of other ES belonging to the group to each decoder. Determine start FP (LBN), end FP of cell from contents of ES_TMAPI. Determine start SOBU_ENTRY, end SOBU_ENTRY from start time, end time in CI Accumulate ENTRY length to target SOBU_ENTRY in ADR_OFS, obtain start address (LB = FP), end address
Set remaining cell length ? (end address – start address), reproduction start time to STC

ST2206
Set start address to read, read length

ST2207
Data length to read < remaining cell length ?
NO
YES

ST2209
Set read length to remaining cell length. Set remaining cell length to 0

ST2208
Remaining cell length ← (remaining cell length – data length to read) Set read length to default

ST2216
Read data for 1SOBU from buffer RAM

ST2220
Buffer data decoder transfer process

ST2224
Read FP ← (read FP + read length) Set MPEG decoder to normal mode (Read & set SCR)

ST2210
Set data read command to drive unit

ST2212
Transfer start ?
NO
YES

ST2214
Is data for 1SOBU accumulated in buffer RAM ?
YES
NO

ST2226
Transfer end (Last cell) ?
NO
YES

ST2238
Angle SW operated ?
YES
NO

ST2239
GPI present ?
NO
YES
ST2240

GP switching

ST2228
Remaining cell length = 0 ?
NO
YES

Return

F I G. 66

```
        ┌─────────────────────┐
        │  Buffer data decoder │──ST2220
        │   transfer process   │
        └─────────────────────┘
                   │           ST22200
                   ▼
  ┌──────────────────────────────────┐
  │ Check the number of packet groups │
  │ in buffer RAM and set so as to    │
  │ process first packet group        │
  └──────────────────────────────────┘
                   │           ST22201
                   ▼
  ┌──────────────────────────────────┐
  │ Read out target packet group      │
  │ header from buffer RAM (Check      │
  │ packet group length and            │
  │ Sync_Pattern, and determine        │
  │ packet header)                     │
  └──────────────────────────────────┘
                   │           ST22202
                   ▼
  ┌──────────────────────────────────┐
  │ Capture PAT, calculate transmission│
  │ timing of each TS packet, and      │
  │ transfer TS packet to decoder in   │
  │ accordance with transmission       │
  │ timing (in case where IPAT=01,     │
  │ discard subsequent packets because │
  │ they are dummy)                    │
  └──────────────────────────────────┘
                   │           ST22203
                   ▼
  ┌──────────────────────────────────┐
  │ Transfer 1 packet group to decoder │
  │ unit in accordance with            │
  │ transmission timing for each TS    │
  │ packet                             │
  └──────────────────────────────────┘
```

ST22204 — End of transfer ? — NO

ST22205

Set APS based on CCI, set APSON/OFF, APS type and the like in video decoder, and set CGMSA by digital copy control based on CCI by using video decoder
In case where digital output (such as 1394 or internet) exists, set 0 : scramble on or output disable or 1 : output IC to output as is, based on value of EPN
In case where ICT is 0, apply limitation to image resolution, and convert HD to SD
In case where ICT is 1, set output IC to output as is.

ST22206 — Does MNF_DATA exist ? — NO
YES — ST22207
Determine MNF_ID by referring to MNF_ID_TBL used in this SOB in MNF_ID_N

ST22208 — Manufacturer of this device ? — NO
YES — ST22209
Process MNF_DATA

ST22210 — Does pack group remain in buffer RAM ? — YES
NO
Return

ST22207 — Set next pack group

# F I G. 67

Process during GP switching —ST2240

—ST22400A

Specify type of switch SW

—ST22401A

Read GPI of GP currently played back

In case where TY is not compatible with switching, end processing without carrying out switching

—ST22402A

Multi-channel ? — YES

Multi-view=angle key
Rain Attention=Rainfall compatible key

NO

—ST22404A

Does BLOCK_TY match SW type ? — NO

YES

—ST22405A

Switch current GP to GP having same BLOCK_NUMBER

—ST22410

Decoder setting process

Return

Return

F I G. 68

Recording operation setting process —ST6070

—ST60

Set profile information in accordance with attribute of recorded contents

F I G. 69

Return